# EUROPEAN PATENT SPECIFICATION

(11) **EP 4 304 691 B1**
(45) Date of publication and mention of the grant of the patent: **13.05.2026**
(21) Application number: 22766492.7
(22) Date of filing: 10.03.2022
(51) Int. Cl.: A61M 16/08, A61M 16/04, A61M 16/06, A61M 16/10, A61M 16/16, A61M 16/00, B01D 46/00, B01D 46/10

(54) **CONNECTORS FOR RESPIRATORY SYSTEM COMPONENTS AND A FILTER**
VERBINDER FÜR ATEMSYSTEMKOMPONENTEN UND FILTER
RACCORDS POUR COMPOSANTS DE SYSTÈME RESPIRATOIRE ET FILTRE

(30) Priority: 10.03.2021 US 202163159315 P; 17.05.2021 US 202163201882 P; 24.08.2021 AU 2021221449
(43) Date of publication of application: 17.01.2024
(73) Proprietor: Fisher & Paykel Healthcare Limited, Auckland, 2013 (NZ)
(72) Inventor: O'CONNOR, Mark Thomas, Auckland, 2013 (NZ); LAING, Brent Ian, Auckland, 2013 (NZ); KLENNER, Jason Allan, Auckland, 2013 (NZ); POWELL, Kevin Blake, Auckland, 2013 (NZ)
(74) Representative: HGF
(86) International application number: PCT/IB2022/052130
(87) International publication number: WO 2022/190019

(56) References cited:
- WO-A1-2017/037660
- US-A- 4 676 241
- US-A1- 2010 116 272
- US-A1- 2010 148 500
- US-A1- 2013 167 841
- US-A1- 2016 001 031
- US-A1- 2016 193 440
- US-A1- 2018 064 901
- US-A1- 2018 078 728
- US-A1- 2018 078 728
- US-A1- 2018 142 690
- US-A1- 2019 022 344

## Description

### TECHNICAL FIELD

The present disclosure relates to connectors for use in breathing circuits, more particularly, though not solely, to connectors to be used at the terminal end of an inspiratory conduit.

### BACKGROUND

Breathing therapy or respiratory therapy circuits are typically single use items. Once a therapy circuit, for example a respiratory gas conduit, has been used by a patient, the conduit together with a patient interface is typically thrown away. One of the reasons for this is to prevent contamination from one patient to another.

Respiratory support systems that are used in multiple-patient environments typically require at least an inspiratory conduit and patient interface to be discarded and replaced between each patient to ensure the components provided for use by each patient are clean and not infected from prior users. This is time consuming which may be detrimental in an emergency. It also creates significant amounts of waste and adds to the overall cost of the procedures or at least to the overall hospital operation costs since the hospital needs to keep a larger stock of inspiratory conduits. This cost can sometimes be passed on to the patient.

In this specification where reference has been made to patent specifications, other external documents, or other sources of information, this is generally for the purpose of providing a context for discussing the features of the invention. Unless specifically stated otherwise, reference to such external documents is not to be construed as an admission that such documents, or such sources of information, in any jurisdiction, are prior art, or form part of the common general knowledge in the art.
US 2018/078728 A1 discloses an apparatus or kit for a respiratory support system for delivering humidified gas to a user or patient. US 2010/148500 A1 discloses a connector for connecting the end of a chemical tube, a blood tube or a tracheal tube inserted to a human body with that of an external circuit. WO 2017/037660 A1 discloses connectors to be provided into fluid communication or engagement either directly with, or via a component to be associated with, a terminal end of a breathing conduit. US 2013/167841 A1 discloses a fluid delivery connection that includes a cuff to engage a port.

### SUMMARY

At times, an airflow source may need to be removed or replaced, or a patient interface may need to be removed from a breathing circuit, or other conduit forming a part of a breathing circuit may need to be replaced. If detaching the particular component (e.g. conduit or a patient interface) from the circuit is difficult or time consuming, such detachments may adversely impact on the maintenance or continued delivery of a gas therapy to a patient, or may become a frustration for the person making such a detachment. Further, in emergencies, a slow or difficult connection mechanism can potentially place the patient's health in danger. Thus, a conduit connector that provides a "quick-connect" or "quick-release" capability, and yet which facilitates the rapid and correct alignment and connection of a new connector, as well as facilitating interchangeability of components, can provide greater comfort and/or safety for the patient.

It may be desirable in a medical setting to utilize certain respiratory circuit components among multiple patients, particularly when each patient may require therapy for only a short time period. Examples include ambulances, Emergency Departments (ED), and transfer of patients between departments within a hospital, where patients may be kept on a respiratory support system for a short period of time (for example, maybe about 2-4 hours for the ED). A bacterial filter is therefore used between a patient interface and the inspiratory tube to prevent contamination of the components upstream of the filter so that these components can be used between patients without reprocessing. The patient interface and filter then stays with the patient as a single patient use component or alternatively may be discarded.

In order to ensure that the components upstream of the filter are not contaminated and the system components are correctly connected to provide the requisite therapy, a connection assembly that can be used between the filter and the inspiratory tube is disclosed herein, such that the inspiratory conduit only connects to the filter and the patient interface conduit cannot directly connect to the inspiratory conduit.

Reducing the risk of contaminating the inspiratory conduit (and its upstream components) can lead to a reduction in waste, and cost, as fewer circuit items are required to be replaced. Furthermore, the reduced need to reprocess the respiratory therapy apparatus minimises downtime for the apparatus. The reprocessing step would normally require replacing and/or sanitising components. Providing the connection assembly also improves safety of the respiratory support provided to the patient.
The present invention is defined by the connector of independent claim 1 and the filter of claim 14. Optional features of the invention are specified in the dependent claims. Any embodiments and/or aspects referred to and described herein which do not fall within the scope of the claims are not part of the present invention and should be considered as illustrative examples.

In an aspect of the disclosure, in accordance with certain features, aspects and advantages of at least one of the embodiments disclosed herein, a connector for use in a respiratory system is disclosed, the connector comprising: a body defining a bore for receipt of a complementary connector component, the bore having a terminal end that provides an entry into the bore for the complementary connector component, a sealing region for engaging with a seal on the complementary connector component, a sealing region dimension between opposed surfaces of the sealing region, and a taper between the opposed surfaces, wherein the sealing region dimension is between about 25 mm and between about 27 mm and wherein the taper is between about 0 degrees and about 4 degrees.

In some configurations, the taper is more than 0 degrees, optionally more than 0.5 degrees, optionally more than 1 degree, optionally more than 1.5 degrees.

In some configurations, the taper is less than 4 degrees, optionally less than 3.5 degrees, optionally less than 3 degrees, optionally less than 2.5 degrees.

In some configurations, the taper is about 2 degrees.

In some configurations, the sealing region dimension is between about 25.5 mm and about 26.5 mm, is about 25 mm, is about 25.1 mm, is about 25.2 mm, is about 25.3 mm, is about 25.4 mm, is about 25.5 mm, is about 25.6 mm, is about 25.7 mm, is about 25.8 mm, is about 25.9 mm, is about 26 mm, is about 26.1 mm, is about 26.2 mm, is about 26.3 mm, is about 26.4 mm, is about 26.5 mm, is about 26.6 mm, is about 26.7 mm, is about 26.8 mm, is about 26.9 mm, is about 27 mm, or is any value between any two of those values.

In some configurations, the sealing region comprises an effective sealing location for contact by a surface of a seal on the complementary connector component, and wherein the sealing region dimension is a distance between opposed surfaces of the effective sealing location.

In some configurations, the effective sealing location is substantially at a centre of the sealing region, for contact by the surface of the seal which is at a centre of the seal.

In some configurations, the connector comprises at least one internal retaining feature.

In some configurations, the at least one internal retaining feature comprises diametrically opposed retaining protrusions that extend into the bore from an inner wall of the body.

In some configurations, the connector comprises at least one internal alignment feature.

In some configurations, the at least one internal alignment feature comprises at least one alignment member that extends into the bore from an inner wall of the body.

In some configurations, the sealing region is closer to the terminal end relative to the internal retaining feature(s) and/or the internal alignment feature(s).

In some configurations, the sealing region comprises a first radial periphery configured to engage a first support protrusion of the complementary connector component.

In some configurations, the taper is configured to provide the first radial periphery with a dimension that is less than the sealing region dimension of the effective sealing location.

In some configurations, the effective sealing location is closer to the terminal end relative to the first radial periphery.

In some configurations, the sealing region comprises a second radial periphery configured to engage a second support protrusion of the complementary connector component.

In some configurations, the taper is configured to provide the first radial periphery with a dimension that is less than a dimension of the second radial periphery.

In some configurations, the second radial periphery is closer to the terminal end relative to the effective sealing location.

In a further aspect of the disclosure, in accordance with certain features, aspects and advantages of at least one of the embodiments disclosed herein, a connector for use in a respiratory system is disclosed, the connector comprising: a body defining a bore for receipt of a complementary connector component, the bore having a terminal end that provides an entry into the bore for the complementary connector component, a sealing region for engaging with a seal on the complementary connector component, and a sealing region dimension between opposed surfaces of the sealing region, the body comprising diametrically opposed retaining protrusions that extend into the bore from an inner wall of the body, the diametrically opposed retaining protrusions having a distance between radially inward surfaces thereof, wherein the sealing region dimension is between about 25 mm and about 27 mm and wherein the distance between the radially inward surfaces is between about 21 mm and about 23 mm.

In some configurations, the sealing region dimension is between about 25.5 mm and about 26.5 mm, is about 25 mm, is about 25.1 mm, is about 25.2 mm, is about 25.3 mm, is about 25.4 mm, is about 25.5 mm, is about 25.6 mm, is about 25.7 mm, is about 25.8 mm, is about 25.9 mm, is about 26 mm, is about 26.1 mm, is about 26.2 mm, is about 26.3 mm, is about 26.4 mm, is about 26.5 mm, is about 26.6 mm, is about 26.7 mm, is about 26.8 mm, is about 26.9 mm, is about 27 mm, or is any value between any two of those values.

In some configurations, the distance between the radially inward surfaces is between about 21.5 mm and about 22.5 mm, or is about 21 mm, is about 21.1 mm, is about 21.2 mm, is about 21.3 mm, is about 21.4 mm, is about 21.5 mm, is about 21.6 mm, is about 21.7 mm, is about 21.8 mm, is about 21.9 mm, is about 22 mm, is about 22.1 mm, is about 22.2 mm, is about 22.3 mm, is about 22.4 mm, is about 22.5 mm, is about 22.6 mm, is about 22.7 mm, is about 22.8 mm, is about 22.9 mm, is about 23 mm, or is any value between any two of those values.

In some configurations, each of the diametrically opposed retaining protrusions has a retaining face, wherein the angle of the retaining face is between about 90 degrees and about 125 degrees relative to a longitudinal axis of the bore.

In some configurations, the angle of the retaining face is between about 95 degrees and about 120 degrees, is between about 100 degrees and 115 degrees, is between about 105 degrees and about 115 degrees, is about 90 degrees, is about 91 degrees, is about 92 degrees, is about 93 degrees, is about 94 degrees, is about 95 degrees, is about 96 degrees, is about 97 degrees, is about 98 degrees, is about 99 degrees, is about 100 degrees, is about 101 degrees, is about 102 degrees, is about 103 degrees, is about 104 degrees, is about 105 degrees, is about 106 degrees, is about 107 degrees, is about 108 degrees, is about 109 degrees, is about 110 degrees, is about 111 degrees, is about 112 degrees, is about 113 degrees, is about 114 degrees, is about 115 degrees, is about 116 degrees, is about 117 degrees, is about 118 degrees, is about 119 degrees, is about 120 degrees, is about 121 degrees, is about 122 degrees, is about 123 degrees, is about 124 degrees, or is about 125 degrees relative to a longitudinal axis of the bore, or is any angle between any two of those values.

In some configurations, the diametrically opposed retaining protrusions are configured to provide a retention force in an axial direction of the connector of between about 10 N and about 100 N, optionally between about 10 N and about 75 N, optionally between about 10 N and about 50 N.

In some configurations, the diametrically opposed retaining protrusions comprise a first pair of adjacent retaining protrusions on one side of the bore and a second pair of adjacent retaining protrusions on an opposite side of the bore.

In some configurations, the sealing region comprises an effective sealing location for contact by a surface of a seal on the complementary connector component, and wherein the sealing region dimension is a dimension of the effective sealing location.

In some configurations, the effective sealing location is substantially at a centre of the sealing region, for contact by the surface of the seal which is at a centre of the seal.

In some configurations, the sealing region is closer to the terminal end relative to the diametrically opposed retaining protrusions.

In a further aspect of the disclosure, in accordance with certain features, aspects and advantages of at least one of the embodiments disclosed herein, a connector for connecting a first respiratory system component to a second respiratory system component is disclosed, the connector coupled to or formed with the first respiratory system component, the connector comprising: a body defining a bore for receipt of a complementary connector component associated with the second respiratory system component, the bore having a terminal end that provides an entry into the bore for the complementary connector component, and comprising one or more retaining features in the bore to assist with retaining the complementary connector component in engagement with the connector, wherein the first respiratory system component has a pressure drop in use through the first respiratory system component of between about 80 Pa and about 490 Pa, and wherein the one or more retaining features are configured to provide a retention force in an axial direction of the connector of between about 10 N and about 100 N.

In some configurations, the first respiratory system component is a filter and the second respiratory system component is an inspiratory conduit.

In some configurations, the filter comprises a filtration material that is hydrophobic.

In some configurations, the one or more retaining features are configured to provide a retention force of between about 10 N and about 75 N, optionally wherein the one or more retaining features are configured to provide a retention force of between about 10 N and about 50 N.

In some configurations, the one or more retaining features are configured to provide a retention force of about 10 N, 11 N, about 12 N, about 13 N, about 14 N, about 15 N, about 16 N, about 17 N, about 18 N, about 19 N, about 20 N, about 21 N, about 22 N, about 23 N, about 24 N, about 25 N, about 26 N, about 27 N, about 28 N, about 29 N, about 30 N, about 31 N, about 32 N, about 33 N, about 34 N, about 35 N, about 36 N, about 37 N, about 38 N, about 39 N, about 40 N, about 41 N, about 42 N, about 43 N, about 44 N, about 45 N, about 46 N, about 47 N, about 48 N, about 49 N, about 50 N, about 51 N, about 52 N, about 53 N, about 54 N, about 55 N, about 56 N, about 57 N, about 58 N, about 59 N, about 60 N, about 61 N, about 62 N, about 63 N, about 64 N, about 65 N, about 66 N, about 67 N, about 68 N, about 69 N, about 70 N, about 71 N, about 72 N, about 73 N, about 74 N, about 75 N, about 76 N, about 77 N, about 78 N, about 79 N, about 80 N, about 81 N, about 82 N, about 83 N, about 84 N, about 85 N, about 86 N, about 87 N, about 88 N, about 89 N, about 90 N, about 91 N, about 92 N, about 93 N, about 94 N, about 95 N, about 96 N, about 97 N, about 98 N, about 99 N, about 100 N, or is any value between any two of those values.

In some configurations, the one or more retaining features comprise diametrically opposed retaining protrusions that extend into the bore from an inner wall of the body.

In some configurations, the diametrically opposed retaining protrusions having a distance between radially inward surfaces thereof, and wherein the distance between the radially inward surfaces is between about 21 mm and about 23 mm.

In some configurations, the distance between the radially inward surfaces is between about 21.5 mm and about 22.5 mm, or is about 21 mm, is about 21.1 mm, is about 21.2 mm, is about 21.3 mm, is about 21.4 mm, is about 21.5 mm, is about 21.6 mm, is about 21.7 mm, is about 21.8 mm, is about 21.9 mm, is about 22 mm, is about 22.1 mm, is about 22.2 mm, is about 22.3 mm, is about 22.4 mm, is about 22.5 mm, is about 22.6 mm, is about 22.7 mm, is about 22.8 mm, is about 22.9 mm, is about 23 mm, or is any value between any two of those values.

In some configurations, each of the diametrically opposed retaining protrusions has a retaining face, wherein the angle of the retaining face is between about 90 degrees and about 125 degrees relative to a longitudinal axis of the bore.

In some configurations, the angle of the retaining face is between about 95 degrees and about 120 degrees, is between about 100 degrees and 115 degrees, is between about 105 degrees and about 115 degrees, is about 90 degrees, is about 91 degrees, is about 92 degrees, is about 93 degrees, is about 94 degrees, is about 95 degrees, is about 96 degrees, is about 97 degrees, is about 98 degrees, is about 99 degrees, is about 100 degrees, is about 101 degrees, is about 102 degrees, is about 103 degrees, is about 104 degrees, is about 105 degrees, is about 106 degrees, is about 107 degrees, is about 108 degrees, is about 109 degrees, is about 110 degrees, is about 111 degrees, is about 112 degrees, is about 113 degrees, is about 114 degrees, is about 115 degrees, is about 116 degrees, is about 117 degrees, is about 118 degrees, is about 119 degrees, is about 120 degrees, is about 121 degrees, is about 122 degrees, is about 123 degrees, is about 124 degrees, or is about 125 degrees relative to a longitudinal axis of the bore, or is any angle between any two of those values.

In some configurations, the diametrically opposed retaining protrusions comprise a first pair of adjacent retaining protrusions on one side of the bore and a second pair of adjacent retaining protrusions on an opposite side of the bore.

In a further aspect of the disclosure, in accordance with certain features, aspects and advantages of at least one of the embodiments disclosed herein, a connector for use in a respiratory system is disclosed, the connector comprising: a body defining a bore for receipt of a complementary connector component in an axial direction of the bore, the bore having a terminal end that provides an entry into the bore for the complementary connector component and a sealing region for engaging with a seal on the complementary connector component, and a retaining face, wherein an axial distance between the sealing region and the retaining face is up to about 17 mm.

In some configurations, the axial distance between the sealing region and the retaining face is at least about 1 mm and up to about 17 mm, optionally is more than about 3 mm and up to about 17 mm, and optionally is more than about 3 mm and up to about 14 mm.

In some configurations, the axial distance between the sealing region and the retaining face is about 1 mm, 2 mm, 3 mm, 4 mm, 5 mm, 6 mm, 7 mm, 8 mm, 9 mm, 10 mm, 11 mm, 12 mm, 13 mm, 14 mm, 15 mm, 16 mm, 17 mm, or is any distance between any two of those values.

In some configurations, the retaining face is provided on at least one retaining protrusion that extends into the bore from an inner wall of the body.

In some configurations, the connector comprises diametrically opposed retaining protrusions that extend into the bore from an inner wall of the body, each retaining protrusion comprising the retaining face.

In some configurations, the sealing region comprises an effective sealing location for contact by a surface of the seal on the complementary connector component, and wherein the distance between the sealing region and the retaining face is the distance between the effective sealing location and the retaining face.

In some configurations, the effective sealing location is substantially at a centre of the sealing region, for contact by the surface of the seal which is at a centre of the seal.

In some configurations, the sealing region is closer to the terminal end relative to the retaining face.

In some configurations, the connector comprises an axially oriented recess located in the body of the connector at the terminal end of the bore.

In some configurations, the retaining face is axially aligned with the axially oriented recess.

In some configurations, the connector comprises an alignment member that extends into the bore from an inner wall of the body, wherein the alignment member is proximal to the retaining face.

In some configurations, the alignment member is at a 90 degree offset around the bore from the retaining face.

In a further aspect of the disclosure, in accordance with certain features, aspects and advantages of at least one of the embodiments disclosed herein, a connector for use in a respiratory system is disclosed, the connector comprising: a body defining a bore for receipt of a complementary connector component, the bore having a central axis and a terminal end that provides an entry into the bore for the complementary connector component, at least one alignment member that extends into the bore from an inner wall of the body and that comprises opposed engagement surfaces, the alignment member comprising a tapered configuration in an axial direction of the bore wherein proximal ends of the opposed engagement surfaces that are more proximal to the terminal end of the bore are closer together than distal ends of the opposed engagement surfaces that are more distal from the terminal end of the bore, wherein the engagement surfaces comprise a twist along at least a substantial part of a length thereof between their proximal ends and their distal ends.

In some configurations, the twist is substantially continuous along the length of each engagement surface.

In some configurations, the alignment member comprises a tapered configuration in a radial direction of the bore, wherein inner edges of the opposed engagement surfaces more proximal to the central axis of the bore are positioned closer together than outer edges of the opposed engagement surfaces more distal from the central axis of the bore and more proximal to the inner wall.

In some configurations, the inner edge of each engagement surface is positioned inwardly of an imaginary radial line extending from the outer edge of the engagement surface to the central axis of the bore.

In some configurations, the proximal end is angularly offset by an angle of greater than 0 degrees and less than 90 degrees, optionally at least about 30 degrees and less than 90 degrees, optionally between about 35 degrees and about 85 degrees, optionally between about 40 degrees and about 80 degrees, optionally between about 45 degrees and about 75 degrees, optionally between about 50 degrees and about 70 degrees, optionally between about 55 degrees and about 65 degrees, optionally at least about 60 degrees relative to the distal end, when viewed from the terminal end of the bore.

In some configurations, the proximal end is angularly offset by at least about 65 degrees, at least about 70 degrees, at least about 75 degrees, at least about 80 degrees, at least about 85 degrees, about 90 degrees, or any angle between any two of those angles, relative to the distal end when viewed from the terminal end of the bore.

In some configurations, either the proximal ends meet at a tip, or wherein the alignment member comprises a surface between the proximal ends that is rounded or filleted.

In some configurations, the alignment member has a substantial chevron shape.

In some configurations, the at least one alignment member comprises two diametrically opposed alignment members.

In some configurations, the engagement surfaces are configured to engage with complementary engagement surfaces on the complementary connector component.

In some configurations, the engagement surfaces are configured to interact with the complementary engagement surfaces to cause the complementary connector component to rotate into a correct alignment, if the complementary connector component is misaligned upon initial insertion into the bore of the connector.

In some configurations, the complementary engagement surfaces comprise an opposite tapered configuration in an axial direction of the complementary connector component wherein proximal portions of the opposed engagement surfaces that are more proximal to the terminal end of the complementary connector that is receivable in the bore of the connector are further apart than distal portions of the complementary engagement surfaces, and wherein the complementary engagement surfaces comprise complementary twists along at least a substantial part of a length thereof between their proximal portions and their distal portions.

In some configurations, each engagement surface is configured to contact a respective one of the complementary engagement surfaces substantially over the length thereof when the connector is engaged with the complementary connector component.

In a further aspect of the disclosure, in accordance with certain features, aspects and advantages of at least one of the embodiments disclosed herein, a connector for use in a respiratory system is disclosed, the connector comprising: a body comprising an engagement portion that is configured for receipt in a bore of a complementary connector component, wherein at least an outer surface of the engagement portion comprises a compliant material that is configured to at least generally conform to a wall of the bore of the complementary connector component when the engagement portion is received in the bore of the complementary connector component, and wherein the body comprises at least one alignment feature.

In some configurations, the at least one alignment feature is configured to cooperate with one or more complementary alignment features of the complementary connector component.

In some configurations, the at least one alignment feature comprises an external alignment feature that is provided on or in the outer surface of the engagement portion of the connector component.

In some configurations, the at least one alignment feature is configured to cooperate with one or more complementary internal alignment features in the bore of the complementary connector component.

In some configurations, the connector comprises a plurality of the alignment features.

In some configurations, the plurality of the alignment features are angularly spaced around a periphery of the engagement portion.

In some configurations, the connector comprises two, three, four, or more of the alignment features.

In some configurations, the body comprises a terminal end that is configured to be received in the bore of the complementary connector component.

In some configurations, the alignment feature(s) extend from the terminal end in a direction away from the terminal end.

In some configurations, the alignment feature(s) extend in an axial direction.

In some configurations, the body of the connector defines a bore that defines a gases lumen.

In some configurations, the bore of the connector extends from the terminal end of the body in a direction away from the terminal end.

In some configurations, the bore of the connector comprises a taper between opposed surfaces of the bore such that a portion of the bore proximal to the terminal end has a larger transverse dimension than a portion of the bore more distal from the terminal end.

In some configurations, the taper is between about 0 degrees and about 15 degrees.

In some configurations, the engagement portion is configured for receipt in the bore of the complementary connector component with a frictional engagement.

In some configurations, the complementary connector component comprises engagement fingers, and the bore of the complementary connector component is between the engagement fingers.

In a further aspect of the disclosure, in accordance with certain features, aspects and advantages of at least one of the embodiments disclosed herein, a connector for use in a respiratory system is disclosed, the connector comprising: a body defining a bore for receipt of a complementary connector component, the bore having a terminal end that provides an entry into the bore for the complementary connector component; at least one internal retaining feature; and at least one external alignment feature.

In some configurations, the at least one external alignment feature is/are configured to interact with complementary external alignment features(s) on the complementary connector component.

In some configurations, the at least one external alignment feature comprises one or more protrusions or recesses that is/are configured to interact with one or more complementary recesses or protrusions on the complementary connector component.

In some configurations, the one or more protrusions or recesses of the component is/are axially oriented.

In some configurations, the at least one external alignment feature is/are at or adjacent the terminal end of the bore.

In some configurations, the at least one internal retaining feature comprises at least one retaining protrusion that extends into the bore from an inner wall of the body.

In some configurations, the at least one internal retaining feature comprises a single retaining protrusion.

In some configurations, the single retaining protrusion has an annular configuration.

In some configurations, the at least one internal retaining feature comprises a plurality of retaining protrusions that are angularly spaced around the bore.

In some configurations, the at least one internal retaining feature is configured to engage with a least one engagement feature on the complementary connector component.

In some configurations, the at least one engagement feature comprises or is provided by an outwardly projecting flange.

In some configurations, the connector comprises a sealing region for engaging with a seal on the complementary connector component.

In some configurations, the sealing region is closer to the terminal end relative to the internal retaining feature(s).

In some configurations, the connector comprises a compliant material.

In a further aspect of the disclosure, in accordance with certain features, aspects and advantages of at least one of the embodiments disclosed herein, a connector for use in a respiratory system is disclosed, the connector comprising: a body defining a bore for receipt of a complementary connector component, the bore having a terminal end that provides an entry into the bore for the complementary connector component; at least one alignment feature; and at least one releasable latch member for engagement with the complementary connector component.

In some configurations, the at least one alignment feature is/are configured to align the at least one releasable latch member with at least one complementary engagement feature on the complementary connector component.

In some configurations, the at least one alignment feature comprises at least one internal alignment feature and/or at least one external alignment feature.

In some configurations, the at least one internal alignment feature comprises at least one alignment member that extends into the bore from an inner wall of the body.

In some configurations, the at least one alignment member is configured to be received in a recess in the complementary connector component.

In some configurations, the recess is located between engagement fingers of the complementary connector component.

In some configurations, the at least one external alignment feature is/are configured to interact with at least one complementary external alignment features on the complementary connector component.

In some configurations, the at least one external alignment feature comprises one or more protrusions or recesses that is/are configured to interact with one or more complementary recesses or protrusions on the complementary connector component.

In some configurations, the one or more protrusions or recesses of the connector is/are axially oriented.

In some configurations, the at least one external alignment feature is/are at or adjacent the terminal end of the bore.

In some configurations, the at least one releasable latch member is configured to engage with at least one complementary engagement feature on the complementary connector component.

In some configurations, the at least one engagement feature is on a complementary external alignment feature on the complementary connector component.

In some configurations, the at least one releasable latch member comprises a latch body with a latching portion for engaging with the complementary connector component, and wherein the latch body of the at least one releasable latch member is selectively movable from a latching configuration in which the latching portion is engaged with the complementary connector component to an unlatched configuration in which the latching portion is disengaged from the complementary connector component.

In some configurations, the at least one releasable latch member is in the latching configuration in an at-rest configuration of the at least one releasable latch member.

In some configurations, the at least one releasable latch member is biased to the latching configuration, optionally by a biasing device and/or by resilience of material of the at least one releasable latch member.

In some configurations, the at least one releasable latch member comprises an actuating surface to enable a user to selectively move the latch body to the unlatched configuration.

In some configurations, the actuating surface of the at least one releasable latch member is at an opposite end of the latch body to the latching portion.

In some configurations, the connector comprises a plurality of the releasable latch members.

In a further aspect of the disclosure, in accordance with certain features, aspects and advantages of at least one of the embodiments disclosed herein, a connector for use in a respiratory system is disclosed, the connector comprising: a body comprising a radially inner wall, a radially outer wall, and a cavity between the radially inner wall and the radially outer wall for receipt of the complementary connector component, the cavity having a terminal end that provides an entry into the cavity for the complementary connector component; and at least one external alignment feature.

In some configurations, the at least one external alignment feature is/are configured to interact with complementary external alignment features(s) on the complementary connector component.

In some configurations, the at least one external alignment feature comprises one or more protrusions or recesses that is/are configured to interact with one or more complementary recesses or protrusions on the complementary connector component.

In some configurations, the one or more protrusions or recesses of the connector is/are axially oriented.

In some configurations, the at least one external alignment feature is/are at or adjacent the terminal end of the cavity.

In some configurations, the body comprises at least one additional alignment feature.

In some configurations, the at least one additional alignment feature is/are configured to cooperate with one or more complementary additional alignment features of the complementary connector component.

In some configurations, the at least one additional alignment feature is/are provided on an outer surface of the radially inner wall of the body of the connector.

In some configurations, the at least one additional alignment feature is/are configured to cooperate with one or more complementary additional internal alignment features in a bore of the complementary connector component.

In some configurations, the connector comprises a plurality of the additional alignment features.

In some configurations, the plurality of the additional alignment features are angularly spaced around a periphery of the radially inner wall.

In some configurations, the connector comprises two, three, four, or more of the additional alignment features.

In some configurations, the additional alignment feature(s) extend(s) from the terminal end of the body in a direction away from the terminal end.

In some configurations, the additional alignment feature(s) extend(s) in an axial direction.

In some configurations, a bore of the connector extends from the terminal end in a direction away from the terminal end.

In some configurations, the bore of the connector comprises a taper between opposed surfaces of the bore such that a portion of the bore proximal to the terminal end has a larger transverse dimension than a portion of the bore more distal from the terminal end.

In some configurations, the taper is between about 0 degrees and about 15 degrees.

In some configurations, the cavity comprises a taper between the radially inner wall and the radially outer wall such that a portion of the cavity proximal to the terminal end has a larger transverse dimension than a portion of the cavity more distal from the terminal end.

In some configurations, the taper is between about 0 degrees and about 15 degrees.

In some configurations, the connector component comprises a sealing region for engaging with a seal on the complementary connector component.

In some configurations, the sealing region is provided by an inner surface of the radially outer wall.

In some configurations, the sealing region is adjacent to the terminal end.

In some configurations, the radially inner wall is configured for receipt in the bore of the complementary connector component with a frictional engagement therebetween.

In some configurations, the connector component comprises a compliant material.

In a further aspect of the disclosure, in accordance with certain features, aspects and advantages of at least one of the embodiments disclosed herein, a connection assembly for use in a respiratory system is disclosed, the connection assembly comprising: the connector as outlined above or herein, and a complementary connector component.

In a further aspect of the disclosure, in accordance with certain features, aspects and advantages of at least one of the embodiments disclosed herein, a connection assembly for use in a respiratory system is disclosed, the connection assembly comprising: a connector and a complementary connector component; wherein the complementary connector component has a bore providing a flow path therethrough, the bore having a central axis and a flow path dimension that is transverse to the central axis; and wherein the connector comprises a body defining a bore for receipt of the complementary connector component, the bore having a central axis and a terminal end that provides an entry into the bore for the complementary connector component, the connector comprising two diametrically opposed alignment members that extend into the bore from an inner wall of the body and that have a first distance between radially innermost surfaces thereof, wherein the first distance is smaller the flow path dimension of the complementary connector component.

In some configurations, a ratio of the first distance to the flow path dimension is between about 0.6 and about 0.95.

In some configurations, the first distance is between about 11.5 mm and about 18 mm.

In some configurations, the first distance is between about 12 mm and about 18 mm, between about 13 mm and about 18 mm, between about 14 mm and 18 mm, between about 15 mm and 18 mm, between about 16 mm and about 17 mm, about 11.1 mm, about 11.2 mm, about 11.3 mm, about 11.4 mm, about 11.5 mm, about 11.6 mm, about 11.7 mm, about 11.8 mm, about 11.9 mm, about 12 mm, about 12.1 mm, about 12.2 mm, about 12.3 mm, about 12.4 mm, about 12.5 mm, about 12.6 mm, about 12.7 mm, about 12.8 mm, about 12.9 mm, about 13 mm, about 13.1 mm, about 13.2 mm, about 13.3 mm, about 13.4 mm, about 13.5 mm, about 13.6 mm, about 13.7 mm, about 13.8 mm, about 13.9 mm, about 14 mm, about 14.1 mm, about 14.2 mm, about 14.3 mm, about 14.4 mm, about 14.5 mm, about 14.6 mm, about 14.7 mm, about 14.8 mm, about 14.9 mm, about 15 mm, about 15.1 mm, about 15.2 mm, about 15.3 mm, about 15.4 mm, about 15.5 mm, about 15.6 mm, about 15.7 mm, about 15.8 mm, about 15.9 mm, about 16 mm, about 16.1 mm, about 16.2 mm, about 16.3 mm, about 16.4 mm, about 16.5 mm, about 16.6 mm, about 16.7 mm, about 16.8 mm, about 16.9 mm, about 17 mm, about 17.1 mm, about 17.2 mm, about 17.3 mm, about 17.4 mm, about 17.5 mm, about 17.6 mm, about 17.7 mm, about 17.8 mm, about 17.9 mm, about 18 mm, or is any distance between any two of those values.

In some configurations, the flow path dimension is between about 10 mm and about 25 mm, between about 11 mm and about 24 mm, between about 12 mm and about 23 mm, between about 13 mm and about 22 mm, between about 14 mm and about 21 mm, between about 15 mm and about 20 mm, between about 16 mm and about 20 mm, between about 17 mm and about 20 mm, between about 18 mm and about 20 mm, about 10 mm, about 11 mm, about 12 mm, about 13 mm, about 14 mm, about 15 mm, about 16 mm, about 17 mm, about 18 mm, about 19 mm, about 20 mm, about 21 mm, about 22 mm, about 23 mm, about 24 mm, about 25 mm, or is about 19.1 mm, or is any value between any two of these values.

In some configurations, the radially innermost surfaces comprise centres of the alignment members, and wherein laterally outward edges of the alignment members connect to the inner wall of the bore of the connector.

In some configurations, an inner face of each alignment member has a convex or concave shape.

In some configurations, each of the alignment members has a substantial chevron shape.

In a further aspect of the disclosure, in accordance with certain features, aspects and advantages of at least one of the embodiments disclosed herein, a filter is disclosed, the filter comprising a filter housing comprising a cavity containing a filtration material in a flow path through the filter, the cavity having a first cross-sectional area, the filter further comprising a connector having a body defining a bore for receipt of a complementary connector component, the bore having a proximal end in fluid communication with the cavity and a terminal end that provides an entry into the bore for the complementary connector component, the filter provided in combination with the complementary connector component, the complementary connector component having a portion that is received in the bore in use, wherein the portion of the complementary connector component comprises a complementary connector component bore, wherein the complementary connector component bore has a second cross-sectional area, wherein a ratio of the second cross-sectional area to the first cross-sectional area is greater than 0 and less than 1.

In some configurations, the ratio of the second cross-sectional area to the first cross-sectional area is at least about 0.05.

In some configurations, the ratio of the second cross-sectional area to the first cross-sectional area is at least about 0.1.

In some configurations, a pressure drop through the filter in use is between about 80 Pa and about 490 Pa.

In some configurations, the connector is as outlined above or herein or is the connector of the connection assembly as outlined above or herein.

In some configurations, the connector is attached to or integrally formed with the filter housing.

In a further aspect of the disclosure, in accordance with certain features, aspects and advantages of at least one of the embodiments disclosed herein, a filter is disclosed, the filter comprising the connector as outlined above or herein or the connection assembly as outlined above or herein.

In some configurations, the filter further comprises a filter housing, wherein the connector is attached to or integrally formed with the filter housing.

In some configurations, the filter further comprises a filtration material in the filter housing.

In some configurations, the filtration material is hydrophobic.

In some configurations, the filter comprises a flow path through the filter housing, wherein a pressure drop through the flow path in use is between about 80 Pa and about 490 Pa.

In a further aspect of the disclosure, in accordance with certain features, aspects and advantages of at least one of the embodiments disclosed herein, a connector component for use in a respiratory system is disclosed, the connector component comprising: a plurality of male engagement fingers each comprising a length; and a cuff with a grip surface on an exterior thereof, wherein the grip surface is at least partly offset around an external surface of the connector component from the male engagement fingers, so as to be oriented at least partly in a lateral direction relative to the lengths of the fingers.

In some configurations, the grip surface is oriented in a direction that is substantially orthogonal to a plane that intersects the lengths of the male engagement fingers.

In some configurations, the grip surface is at least partially framed by a surface feature.

In some configurations, at least two opposite sides, and optionally at least three sides including the two opposite sides, of the grip surface are framed by the surface feature.

In some configurations, the surface feature comprises a recess in an outer wall of the cuff.

In some configurations, the connector comprises two diametrically opposed male engagement fingers, and the cuff further comprises two diametrically opposed grip surfaces.

In some configurations, the male engagement fingers are locking fingers.

In some configurations, the male engagement fingers are configured to be received by a female connector.

In some configurations, the male engagement fingers each comprise an engagement feature on an outer surface of the finger.

In some configurations, the engagement features each comprise a recess or aperture for receiving a respective retaining protrusion that extends from an inner wall of the female connector.

In some configurations, the male engagement fingers comprise engagement surfaces for engaging with complementary engagement surfaces of another connector, optionally of the female connector.

In some configurations, the engagement surfaces comprise a twist.

In some configurations, at least a portion of a tip of each male engagement finger has a chamfered configuration.

In some configurations, the connector component comprises a seal proximal to a base of the fingers.

In some configurations, the seal comprises a wiper seal, and optionally wherein the seal comprises a T-shaped wiper seal.

In some configurations, the seal is disposed in a recess.

In some configurations, the cuff has a tapered configuration in which a portion of the cuff proximal to the male engagement fingers has a larger lateral dimension than a portion of the cuff distal to the male engagement fingers.

In some configurations, the grip surface is substantially aligned with the portion of the cuff proximal to the male engagement fingers, in an axial direction of the connector component.

In some configurations, the connector component further comprises an external alignment feature.

In some configurations, the external alignment feature comprises a protrusion.

In some configurations, the connector component comprises reinforcing ribs along internal surface(s) of the male engagement fingers.

In some configurations, the connector component comprises a patient end electrical component disposed in a gas flow lumen.

In some configurations, an inner surface of the cuff comprises opposing protrusions to engage with opposing recesses on a body of the connector component, wherein the opposing protrusions are aligned with the grip surface(s).

In some configurations, the opposing recesses are provided in an overmould on the body.

In some configurations, the cuff comprises a thermoplastic elastomer material. In some configurations, the thermoplastic elastomer comprises thermoplastic vulcanizate for example.

In a further aspect of the disclosure, in accordance with certain features, aspects and advantages of at least one of the embodiments disclosed herein, a connection assembly is provided, the connection assembly comprising a connector outlined above or herein, and the connector component outlined above or herein.

In a further aspect of the disclosure, in accordance with certain features, aspects and advantages of at least one of the embodiments disclosed herein, a filter is disclosed, the filter comprising a connector outlined above or herein.

In a further aspect of the disclosure, in accordance with certain features, aspects and advantages of at least one of the embodiments disclosed herein, a filter is disclosed, the filter comprising: an inlet port for the flow of gases; an outlet port for the flow of gases; a cavity containing a filtration material in a flow path through the filter, wherein the filtration material has a first side proximal to the inlet port and a second side proximal to the outlet port; and a projection proximal to the outlet port and extending towards the second side of the filtration material.

In some configurations, the projection is configured to inhibit a flow of liquid between the cavity and the outlet port.

In some configurations, the inlet port and the outlet port are substantially coaxial.

In some configurations, the projection extends between about 1 mm and about 15 mm into the cavity, optionally between about 3 mm and about 10 mm, and optionally about 5 mm into the cavity.

In some configurations, the projection is substantially coaxial with the outlet.

In some configurations, the filter comprises a connector for connecting with a complementary connector component.

In some configurations, the connector is attached to or is integrally formed with a filter housing.

In some configurations, the connector comprises a body defining a bore for receipt of the complementary connector component, and wherein the body defines the inlet port.

In some configurations, the connector comprises one or more retaining features to assist with retaining the complementary connector component in engagement with the connector.

In some configurations, the connector comprises at least one internal alignment feature and/or at least one external alignment feature.

In some configurations, the connector is as outlined above or herein.

In a further aspect of the disclosure, in accordance with certain features, aspects and advantages of at least one of the embodiments disclosed herein, a respiratory conduit end cap is disclosed, the respiratory conduit end cap comprising: a body defining a bore for receipt of a complementary connector component of the respiratory conduit and defining a cavity for receipt of gases from the respiratory conduit; a support feature for supporting the end cap; and a connector for connecting with the complementary connector component, the connector comprising at least one alignment feature and one or more retaining features to assist with retaining the end cap on the complementary connector component of the respiratory conduit.

In some configurations, the at least one alignment feature comprises at least one internal alignment feature and/or at least one external alignment feature.

In some configurations, the respiratory conduit end cap comprises at least one internal alignment feature.

In some configurations, the at least one internal alignment feature comprises at least one alignment member that extends into the bore from an inner wall of the body.

In some configurations, the at least one alignment member comprises opposed engagement surfaces, the at least one alignment member comprising a tapered configuration in an axial direction of the bore wherein proximal ends of the opposed engagement surfaces that are more proximal to a terminal end of the bore are closer together than distal ends of the opposed engagement surfaces that are more distal from the terminal end of the bore, wherein the engagement surfaces comprise a twist along at least a substantial part of a length thereof between their proximal ends and their distal ends.

In some configurations, the opposed engagement surfaces are configured to interact with complementary engagement surfaces on the complementary connector component of the respiratory conduit to cause the complementary connector component to rotate into a correct alignment, if the complementary connector component is misaligned upon initial insertion into the bore of the respiratory conduit end cap.

In some configurations, the at least one alignment member comprises a substantial chevron shape.

In some configurations, the at least one alignment member comprises or is formed by a plurality of ribs that extend in an axial direction of the bore.

In some configurations, the at least one internal alignment feature is configured to provide haptic feedback during connection of the end cap to the complementary connector component.

In some configurations, the at least one alignment feature is configured to align the one or more retaining features with one or more complementary engagement features on the complementary connector component.

In some configurations, the respiratory conduit end cap further comprises at least one vent for a flow of gases from the cavity to an external surface of the respiratory conduit end cap.

In some configurations, the at least one vent comprises a channel or aperture in the body, or a gap between the body and a portion of the complementary connector component when the respiratory conduit end cap is on the complementary connector component of the respiratory conduit.

In some configurations, the one or more retaining features is/are configured with respect to the size of the at least one vent to enable a flow of gases through the conduit and the vent of the respiratory conduit end cap of up to a about 60 litres per minute and optionally of up to about 90 litres per minute.

In some configurations, the support feature is configured so that the respiratory conduit end cap and a connected respiratory conduit can be hung from another item by the support feature.

In some configurations, the support feature comprises a hook or a loop.

In a further aspect of the disclosure, in accordance with certain features, aspects and advantages of at least one of the embodiments disclosed herein, a respiratory conduit end cap is disclosed, the respiratory conduit end cap comprising: a body defining a bore for receipt of a complementary connector component of the respiratory conduit and defining a cavity for receipt of gases from the respiratory conduit; a support feature for supporting the end cap; at least one vent for a flow of gases from the cavity to an external surface of the respiratory conduit end cap; and a connector for connecting with the complementary connector component, the connector comprising one or more retaining features to assist with retaining the end cap on the complementary connector component of the respiratory conduit.

In some configurations, the one or more retaining features is/are configured with respect to the size of the at least one vent to enable a flow of gases through the conduit and the at least one vent of the respiratory conduit end cap of up to at least 60 litres per minute, and optionally of up to about 90 litres per minute.

In some configurations, the at least one vent comprises at least one channel in the body, and wherein the or each retaining feature is provided adjacent a respective channel.

In some configurations, the at least one channel defines a resiliently flexible tongue, and wherein the at least one retaining feature is provided on a respective resiliently flexible tongue.

In some configurations, the respiratory conduit end cap has one or more of the features outlined above or herein.

In a further aspect of the disclosure, in accordance with certain features, aspects and advantages of at least one of the embodiments disclosed herein, the combination of a respiratory conduit end cap and a complementary connector component is disclosed, the combination comprising: the respiratory conduit end cap outlined above or herein and the complementary connector component.

In some configurations, the complementary connector component has one or more of the features outlined above or herein.

In a further aspect of the disclosure, in accordance with certain features, aspects and advantages of at least one of the embodiments disclosed herein, a filter is disclosed, the filter comprising: an inlet port for the flow of gases; an outlet port for the flow of gases; a cavity containing a filtration material in a flow path through the filter; and a connector at the inlet port, the connector having a body defining a bore for receipt of a complementary connector component, the bore having a proximal end in fluid communication with the cavity and a terminal end that provides an entry into the bore for the complementary connector component, the connector comprising two or more retaining protrusions that extend into the bore from an inner wall of the body, the two or more retaining protrusions configured for engaging with engagement features on diametrically opposed male engagement fingers of the complementary connector component that are receivable in the bore.

In some configurations, the two or more retaining protrusions comprise two diametrically opposed retaining protrusions.

In some configurations, the connector comprises at least one internal alignment feature and/or at least one external alignment feature.

In a further aspect of the disclosure, in accordance with certain features, aspects and advantages of at least one of the embodiments disclosed herein, a filter is disclosed, the filter comprising: an inlet port for the flow of gases; an outlet port for the flow of gases; a cavity containing a filtration material in a flow path through the filter; and a connector at the inlet port, the connector having a body defining a bore for receipt of a complementary connector component, the bore having a proximal end in fluid communication with the cavity and a terminal end that provides an entry into the bore for the complementary connector component, wherein the connector comprises at least one internal alignment feature and/or at least one external alignment feature.

In some configurations, the at least one internal alignment feature comprises at least one alignment member that extends into the bore from an inner wall of the body.

In some configurations, the at least one external alignment feature comprises a recess.

In some configurations, the connector is as outlined above or herein

In a further aspect of the disclosure, in accordance with certain features, aspects and advantages of at least one of the embodiments disclosed herein, a respiratory therapy kit for use in a respiratory therapy system is disclosed, the respiratory therapy kit comprising: a patient interface comprising a nasal cannula; and a filter, wherein the filter comprises: an inlet port for the flow of gases; an outlet port for the flow of gases; a cavity containing a filtration material in a flow path through the filter; and the connector as outlined above or herein at the inlet port.

In some configurations, the connector is integrally formed with the filter.

In some configurations, the filter is as outlined above or herein.

In some configurations, the respiratory therapy kit comprises an interface tube for delivering gases from the outlet port to the nasal cannula.

In a further aspect of the disclosure, in accordance with certain features, aspects and advantages of at least one of the embodiments disclosed herein, a patient interface assembly is disclosed, the patient interface assembly comprising: a patient interface; a conduit; and a connector at a terminal end of the conduit for engagement with a complementary connector component, the connector comprising: a body defining a bore for receipt of the complementary connector component, the bore having a terminal end that provides an entry into the bore for the complementary connector component and a sealing region for engaging with a seal on the complementary connector component; and one or more retaining features to assist with retaining the complementary connector component in engagement with the connector.

In some configurations, the connector is coupled to or is formed with the conduit.

In some configurations, the terminal end of the conduit comprises a filter, and the connector is coupled to or is formed with the filter.

In some configurations, the one or more retaining features is/are in the bore.

In some configurations, the one or more retaining features comprise one or more retaining protrusions that extend into the bore from an inner wall of the body.

In some configurations, the one or more retaining protrusions comprise diametrically opposed retaining protrusions.

In some configurations, the sealing region is closer to the terminal end relative to the retaining protrusion(s).

In some configurations, the connector comprises a plurality of the retaining protrusions.

In some configurations, the retaining protrusions are configured for engaging with engagement features on male engagement fingers of the complementary connector component.

In some configurations, the connector comprises at least one internal alignment feature and/or at least one external alignment feature.

In some configurations, the connector is as outlined above or herein.

In some configurations, the patient interface comprises a nasal cannula.

The terms "respiratory system", "breathing assistance apparatus" and "respiratory apparatus" are used interchangeably herein.

Features from one or more embodiments or configurations may be combined with features of one or more other embodiments or configurations. Additionally, more than one embodiment may be used together in a respiratory system during a process of respiratory support of a patient.

As used herein the term "(s)" following a noun means the plural and/or singular form of that noun.

As used herein the term "and/or" means "and" or "or", or where the context allows both.

The term "comprising" as used in this specification means "consisting at least in part of". When interpreting each statement in this specification that includes the term "comprising", features other than that or those prefaced by the term may also be present. Related terms such as "comprise" and "comprises" are to be interpreted in the same manner.

It is intended that reference to a range of numbers disclosed herein (for example, 1 to 10) also incorporates reference to all rational numbers within that range (for example, 1, 1.1, 2, 3, 3.9, 4, 5, 6, 6.5, 7, 8, 9 and 10) and also any range of rational numbers within that range (for example, 2 to 8, 1.5 to 5.5 and 3.1 to 4.7) and, therefore, all sub-ranges of all ranges expressly disclosed herein are hereby expressly disclosed. These are only examples of what is specifically intended and all possible combinations of numerical values between the lowest value and the highest value enumerated are to be considered to be expressly stated in this application in a similar manner.

This disclosure may also be said broadly to consist in the parts, elements and features referred to or indicated in the specification of the application, individually or collectively, and any or all combinations of any two or more said parts, elements or features.

Where specific integers are mentioned herein which have known equivalents in the art to which this disclosure relates, such known equivalents are deemed to be incorporated herein as if individually set forth.

The disclosure consists in the foregoing and also envisages constructions of which the following gives examples only.

### BRIEF DESCRIPTION OF THE DRAWINGS

Specific embodiments and modifications thereof will become apparent to those skilled in the art from the detailed description herein having reference to the figures that follow, of which:
Figure 1 schematically shows a respiratory therapy system.
Figure 2 is a perspective view of a filter and a patient end of an inspiratory conduit that are coupled together by a connection assembly.
Figure 3 is a perspective view of the filter and the connection assembly.
Figure 4(a) is a front view of the filter and the connection assembly.
Figure 4(b) is a sectional view along line b-b of Figure 4(a).
Figure 5(a) is a front view of an inlet section of the filter and the connector of the connection assembly.
Figure 5(b) is an end view of the inlet section of the filter and the connector of the connection assembly.
Figure 5(c) is a right side view of the inlet section of the filter and the connector of the connection assembly.
Figure 5(d) is a sectional view along line d-d of Figure 5(a).
Figure 5(e) is a sectional view along line e-e of Figure 5(c).
Figure 6(a) is a fragmentary end view of the connection assembly.
Figure 6(b) is a sectional view along line b-b of Figure 6(a).
Figure 7 is a fragmentary end view of the connector.
Figure 8 is a sectional perspective view of the connector.
Figure 9 is a sectional front view of the connector.
Figure 10 is a partial end view of the connector.
Figure 11(a) is an exploded perspective view of the filter, patient end of the inspiratory conduit, and the connection assembly.
Figure 11(b) is another exploded perspective view of the filter, patient end of the inspiratory conduit, and the connection assembly.
Figure 11(c) is another exploded perspective view of the patient end of the inspiratory conduit and part of the connection assembly.
Figure 12(a) is a side view of the connection assembly during a connection process of the connector and complementary connector, with the connector and complementary connector misaligned.
Figure 12(b) is a sectional view of the connection assembly during connection of the connector and complementary connector, with the connector and complementary connector misaligned.
Figure 13(a) is an end view of the connection assembly showing first exemplary relative dimensions of components.
Figure 13(b) is an end view of the connection assembly showing second exemplary relative dimensions of components.
Figure 13(c) is an end view of the connection assembly showing third exemplary relative dimensions of components.
Figure 14 is a sectional side view of the filter, patient end of the inspiratory conduit, and the connection assembly.
Figure 15 shows operational parameters of the respiratory therapy device that may be used with the filter, inspiratory conduit, and connection assembly.
Figure 16(a) is a front view of the complementary connector component of the connection assembly.
Figure 16(b) is a sectional view along line b-b of Figure 16(a).
Figure 17(a) is an end view of a body of the complementary connector component.
Figure 17(b) is a side view of the body.
Figure 17(c) is a front view of the body.
Figure 18 is a perspective view of the body of Figures 17(a)-17(c).
Figure 19 shows a front view of the connector and complementary connector component during assembly, with the inset to Figure 19 showing details of the components.
Figure 20 shows alignment members of the connector and complementary connector component, and in particular shows the twisted engagement surfaces of those members, where parts of the alignment members 720 are shown as transparent for clarity.
Figure 21 is a perspective view of an alternative complementary connector component of the connection assembly.
Figure 22(a) is a front view of the alternative complementary connector component.
Figure 22(b) is a side view of the alternative complementary connector component.
Figure 22(c) is sectional view along line c-c of Figure 22(b).
Figure 23(a) is an end view of a body of the alternative complementary connector component.
Figure 23(b) is a side view of the body.
Figure 23(c) is a front view of the body.
Figure 24 is a perspective view of the body of Figures 23(a)-23(c).
Figure 25 is a perspective view of the cuff of the complementary connector component.
Figure 26 is a perspective view of the cuff with an overmould inside the cuff.
Figure 27 is a sectional part front view of the complementary connector component.
Figure 28 is a comparison view of the bodies of the complementary connector component 800 and the alternative complementary connector component 1800.
Figure 29(a) is a side view of an alternative filter.
Figure 29(b) is a sectional side view along line b-b of Figure 29(a).
Figure 29(c) is an enlarged view of the circled region of Figure 29(b).
Figure 30 is a sectional perspective view of the alternative filter.
Figure 31(a) is a sectional partial side view of another alternative filter.
Figure 31(b) is a sectional partial side view of another alternative filter.
Figure 31(c) is a sectional partial side view of another alternative filter.
Figure 31(d) is a sectional partial end view of projections of another alternative filter.
Figure 31(e) is a perspective partial view of a projection of another alternative filter.
Figure 32(a) is an overhead perspective view of a respiratory conduit end cap attached to a complementary connector of a respiratory conduit.
Figure 32(b) is an underside perspective view of the respiratory conduit end cap attached to the complementary connector of the respiratory conduit.
Figure 33(a) is a sectional view of the respiratory conduit end cap attached to the complementary connector of the respiratory conduit.
Figure 33(b) is another sectional view of the respiratory conduit end cap attached to the complementary connector of the respiratory conduit.
Figure 34(a) is an overhead perspective view of the respiratory conduit end cap.
Figure 34(b) is an underside perspective view of the respiratory conduit end cap.
Figure 35(a) is a sectional view of the respiratory conduit end cap.
Figure 35(b) is another sectional view of the respiratory conduit end cap.
Figure 36(a) is a sectional perspective view of the respiratory conduit end cap.
Figure 36(b) is another sectional perspective view of the respiratory conduit end cap.
Figure 37(a) is an overhead perspective view of an alternative respiratory conduit end cap.
Figure 37(b) is an underside perspective view of the alternative respiratory conduit end cap.
Figure 38(a) is a sectional view of the alternative respiratory conduit end cap.
Figure 38(b) is another sectional view of the alternative respiratory conduit end cap.
Figure 39(a) is a sectional perspective view of the alternative respiratory conduit end cap.
Figure 39(b) is another sectional perspective view of the alternative respiratory conduit end cap.
Figure 40(a) is an overhead perspective view of an alternative respiratory conduit end cap.
Figure 40(b) is an underside perspective view of the alternative respiratory conduit end cap.
Figure 41(a) is a sectional view of the alternative respiratory conduit end cap.
Figure 41(b) is another sectional view of the alternative respiratory conduit end cap.
Figure 42(a) is a sectional perspective view of the alternative respiratory conduit end cap.
Figure 42(b) is another sectional perspective view of the alternative respiratory conduit end cap.
Figure 43(a) is an overhead perspective view of another alternative respiratory conduit end cap attached to a complementary connector of a respiratory conduit.
Figure 43(b) is an underside perspective view of the alternative respiratory conduit end cap attached to the complementary connector of the respiratory conduit.
Figure 44(a) is a sectional view of the alternative respiratory conduit end cap attached to the complementary connector of the respiratory conduit.
Figure 44(b) is another sectional view of the respiratory conduit end cap attached to the complementary connector of the respiratory conduit.
Figure 45(a) is an overhead perspective view of the alternative respiratory conduit end cap.
Figure 45(b) is an underside perspective view of the respiratory conduit end cap.
Figure 46(a) is a sectional view of the alternative respiratory conduit end cap.
Figure 46(b) is another sectional view of the alternative respiratory conduit end cap.
Figure 47(a) is a sectional perspective view of the alternative respiratory conduit end cap.
Figure 47(b) is another sectional perspective view of the alternative respiratory conduit end cap.
Figure 48 is a schematic sectional view of an alternative respiratory conduit end cap.
Figure 49 is schematic sectional view of an alternative respiratory conduit end cap.
Figure 50 shows a perspective view of a patient interface in use on a user, the patient interface including a secondary supply conduit which connects between the outlet end of the gases supply conduit and the nasal cannula, and a neck tie or lanyard which in use loops around the neck of a patient and connects to the supply conduit at or close to the outlet end to support the weight of the supply conduit in use, the patient interface also including a head strap for securing the patient interface to a patient's head in use.
Figure 51 shows a front and a side view of an alternative patient interface in use, this alternative form having a pair of ear straps that loop around the ears of a user in use to hold the patient interface in place on the face of a user in use.
Figure 52 shows a perspective view from the front and to one side of one form of the nasal cannula, the nasal cannula of the preferred form having a face mount part and a manifold part, the manifold part removable from the face mount part, the secondary supply conduit connected to the manifold part.
Figure 53 shows the patient interface of Figure 52 with the manifold part removed from the face mount part.
Figure 54 shows a perspective view from the front and to one side of one form of the face mount part of the preferred form of nasal cannula, the face mount part including a section adapted to receive the manifold part, and a pair of nasal prongs extending from the face mount part.
Figure 55 shows a perspective view from the rear and to one side of one form of the face mount part of the nasal cannula, the face mount part having a pair of nasal prongs extending from the face mount part, each of the nasal prongs having a gases exit cut-out on their rear face, at the upper part of the prong.
Figure 56 shows a view directly from the rear of the manifold part of Figure 54, with the gases exit cut-out clearly shown.
Figure 57 shows a top view of one form of the face mount part of the nasal cannula, the view showing the preferred inwards angle of the nasal prongs relative to a vertical plane which bisects the face mount part, and alignment of a surface which defines the perimeter of the cut-out section in the preferred form.
Figure 58 shows a rear perspective view of the nasal cannula, the view showing one form and placement of the surface.
Figure 59 shows a view from under the nasal cannula.
Figure 60 shows an alternate form of the nasal cannula, the cannula includes a manifold with two carrier tubes extending from the manifold and looping around the user's ears and a prong connected to the end of each carrier tube.
Figure 61 shows a further form of the nasal cannula where the carrier tubes extend from the manifold, the carrier tubes connect to the face mount part and the prongs extending from the face mount part
Figure 62 shows a perspective view of an alternative connector for use in a respiratory system.
Figure 63 shows a side sectional view of the alternative connector.
Figure 64 shows an exploded perspective view of a connection assembly with the alternative connector.
Figure 65 shows a perspective sectional view of the connection assembly.
Figure 66 shows a perspective view of an alternative connector for use in a respiratory system.
Figure 67 shows an exploded perspective view of a connection assembly with the alternative connector.
Figure 68 shows a side sectional view of the connection assembly.
Figure 69 shows a perspective view of an alternative connector for use in a respiratory system.
Figure 70 shows another perspective view of the alternative connector.
Figure 71 shows a perspective view of a connection assembly with the alternative connector.
Figure 72 shows a side sectional view of the connection assembly.
Figure 73 shows a perspective view of an alternative connector for use in a respiratory system.
Figure 74 shows an exploded perspective view of a connection assembly with the alternative connector.
Figure 75 shows a side sectional view of the connection assembly.

### DETAILED DESCRIPTION

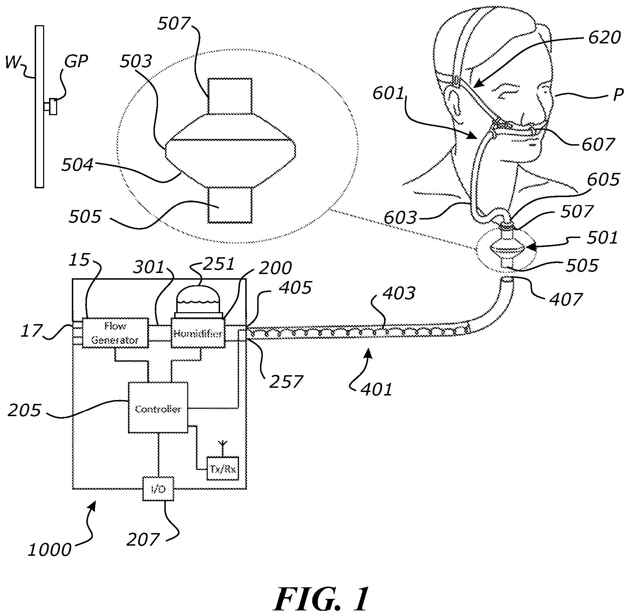

Figure 1 illustrates a respiratory circuit. A patient P is receiving gases through a nasal cannula assembly of a patient interface 601 that is operatively connected to a gases transportation pathway or a respiratory conduit which, in the configuration shown, is an inspiratory conduit 401, via a filter 501. The received gases may be high flow and/or pressurised and/or humidified. The inspiratory conduit 401 in turn is connected to a flow source of a breathing assistance apparatus 1000 comprising a flow generator or blower 15 and optionally a humidifier 200. Alternatively, the inspiratory conduit 401 may receive gases from another appropriate gases supply.

The inspiratory conduit 401 is connected to the outlet 257 of the flow source.

The flow source is provided with control means or electronic controller 205 which may comprise a microprocessor-based controller executing computer software commands stored in associated memory. Gases flowing through the inspiratory conduit 401 are passed to the patient by way of the filter 501 and patient interface 601.

The controller 205 receives input from sources such as user input means 207 through which a user of the device may, for example, set a predetermined required value (pre-set value) of humidity or temperature of the gases supplied to patient P.

The blower 15 may be provided with a variable speed pump or fan which draws air or other gases through the blower inlet 17. The speed of variable speed pump or fan may be controlled by the controller 205 (or alternatively by a further control means or electronic controller) in response to inputs from controller 205 and a user set predetermined required value (pre-set value) of pressure or fan speed or flow rate via an input device. Alternatively, the gases may be provided from a wall supply; i.e. a wall gas port GP in a wall W.

In some configurations, rather than using a blower 15, gases flow may be obtained from some other source(s) of gas. For example, in some configurations, source(s) of gas may comprise one or more containers of compressed air and/or another gas and one or more valve arrangements adapted to control the rate at which gases leave the one or more containers. As another example, in some configurations, gases may be obtained from an oxygen concentrator. The system may also include a supplementary gases source to provide an air and supplementary gas mixture. For example, the supplementary gas might be O2. In some configurations, the apparatus may be adapted to deliver a high flow therapy.

The humidifier 200 may be integral with the flow source. The flow source optionally comprises a blower.

The humidifier 200 comprises a humidifier chamber which contains a volume of liquid such as water. The humidifier chamber may be formed from a plastics material and may have a highly heat conductive base (for example an aluminium base) which is in direct contact with a heater plate of humidifier 200.

The humidifier chamber may be any suitable chamber that holds suitable liquid for use in humidifying gases, such as water for example. The humidifier chamber may be a manual fill chamber, and may be filled through a liquid inlet port. Alternatively, the humidifier chamber may be an automatically filling chamber, and liquid may be fed to the humidifier chamber from a liquid container, bag, or other liquid source. The humidifier chamber may comprise a float valve in the liquid reservoir, the float valve configured to control flow of liquid form the liquid container into the liquid reservoir.

A gases delivery conduit 301 is located upstream of the humidifier 200. The gases delivery conduit 301 is in fluid/pneumatic communication with the humidifier 200 or is configured to be placed in fluid/pneumatic communication with the humidifier 200 upstream of the humidifier chamber; i.e. with the humidifier 200 downstream of the gases conduit 301. The gases delivery conduit 301 is configured to receive one or more gases from a source of gas and deliver the gas(es) to a gases inlet port of the humidifier chamber.

The inspiratory conduit 401 extends from the humidifier 200 (or alternatively from the gases conduit 301 if a humidifier is not provided) to link the humidifier 200 or gases conduit 301 to the patient interface 601 via an in-line filter 501. The inspiratory conduit 401 may comprise a conduit heater 403 adapted to heat gases passing through the conduit 401. The conduit heater 403 will help minimise or prevent the formation of condensation in the inspiratory conduit, which could otherwise occur due to a temperature differential between the interior of the conduit wall and exterior of the conduit wall. In other configurations the conduit heater 403 may not be present.

The inspiratory conduit 401 comprises an upstream gases inlet port 405 at one end of the conduit and a downstream gases outlet port 407 at the opposite end of the conduit, with the conduit defining a gases flow path from the gases inlet port 405 to the gases outlet port 407.

The humidifier 200 is in fluid/pneumatic communication with the inspiratory conduit 401 upstream of the inspiratory conduit 401 or is configured to be placed in fluid/pneumatic communication with the inspiratory conduit 401 upstream of the inspiratory conduit; i.e. with the inspiratory conduit positioned downstream of the humidifier 200. The gases outlet port 257 of the humidifier chamber 251 and the gases inlet port 405 of the inspiratory conduit 401 may comprise complementary coupling features, to enable the inspiratory conduit to be coupled to the humidifier to provide fluid/pneumatic communication between the humidifier 200 and the inspiratory conduit 401. The complementary coupling features may be disconnectable from each other to enable the inspiratory conduit 401 to be decoupled from the humidifier 200. Alternatively, the complementary coupling features may be permanently or semi-permanently coupled.

The inspiratory conduit 401 will typically have a longer length than the gases delivery conduit 301.

The filter 501 comprises a filter housing 504 with an enlarged central body portion 503. The central body portion 503 could be a generally cylindrical shape or any other suitable shape. The central body portion 503 could be any suitable size including any suitable length and transverse dimension(s). The filter housing 504 may have a generally cylindrical shape or any other suitable shape. A leading edge of the enlarged central body portion comprises a tapering wall that terminates at an upstream gases inlet port 505, and a trailing edge of the enlarged central body portion terminates at a downstream gases outlet port 507. The gases inlet port 505 and gases outlet port 507 are in fluid/pneumatic communication via the central body portion 503. The filter may be a high-efficiency particulate arrestance (HEPA) filter.

The enlarged central body portion 503 of the filter housing defines a cavity that contains a suitable filter media/ filtration material. For example, the filtration material may comprise pleated paper, nano-fibers, or any other suitable filtration material, including sock filters, stacked disc filters, spiral filters, block(s) of filter material, a disc or discs of filter material with streams of filter material to free flow from or off the disc in fluid flow. The filter captures and prevents downstream passage therethrough of particulates, bacteria and/or other infectious material from the inspiratory conduit 401 to the patient P, and also captures and prevents upstream passage therethrough of bacteria and/or other infectious material from the patient P to the inspiratory conduit 401. In some embodiments where a humidified flow of gases is provided to the patient P and the inspiratory conduit 401 is positioned closer to a ground level relative to the patient interface 601 during use, condensate accumulating in the patient interface 601, for example in patient interface gases conduit 603 may tend to flow upstream towards the inspiratory conduit 401 due to gravity. Such condensate may carry particulates, bacteria and/or other infectious material. The filter 501 therefore captures and prevents upstream passage therethrough of bacteria and/or other infectious material from the patient P to the inspiratory conduit 401.

Further examples of filtration material types comprise of: ceramics, fabricated metals (e.g. woven wire cloth), porous plastics (such as, but not limited to, plastic powders moulded into porous rigid shapes), non-woven media (e.g. dry-formed, wet-laid or membrane).

While further examples of filtration materials may comprise of: Cellulose, cotton, wood pulp, glass, fibreglass, glass micro fibre, or composites; polymers, such as polytetrafluoroethylene PTFE, polycarbonate (PC), acrylics including modacrylics, rayon, fluoropolymers, thermoplastic polyurethane (TPU), polyethylene (PE), polyamides, polyester, polypropylene (PP), nylon, metals, such as galvanised steel, stainless steel, aluminium, copper, wool.

Composites may consist of polyamides, polyethersulfone, polysulfone, ceramic, carbon or any of the other polymers listed above, such as polytetrafluoroethylene PTFE, polycarbonate (PC), acrylics, rayon, fluoropolymers, thermoplastic polyurethane (TPU), polyethylene (PE), polyamides, polyester, polypropylene (PP), nylon. The composite may be a composite because it is of a multi-layer construction. Separate layers may have different functions, for example support or strength layers, different filtration efficiencies or pore sizes, for the absorption of gases, to contain particles and/or contaminants in the inner or different layers of a composite filter.

It will also be appreciated the filtration material may be or comprise of electrostatic, hydrophilic or hydrophobic characteristics or properties.

In some configurations, the filtration material may comprise a mechanical filter, to capture particles by direct interception. Alternatively, the filtration material may comprise an electrostatic material, to capture particles by electrical attraction. In other alternatives, the filtration material may be hydrophobic, so that the filter repels water and does not promote microbial growth. The filter should allow for air and water vapour (i.e. humidity) to pass through the filter without compromising filtration of infectious matter and condensate. In various examples, filtration materials or filter media composition may comprise one or more of the following: mineral fibres, glass fibres, ceramic fibres, polypropylene, expanded polytetrafluoroethylene (PTFE), acrylics including modacrylics and thermoplastic polyurethane (e.g. Estane), cellulose fibres, or electrostatic fibres.

The inspiratory conduit 401 is in fluid/pneumatic communication with the filter 501 upstream of the filter or is configured to be placed in fluid/pneumatic communication with the filter upstream of the filter; i.e. with the filter located downstream of the inspiratory conduit 401. The gases inlet port 505 of the filter 501 and the gases outlet port 407 of the inspiratory conduit 401 comprise complementary coupling features, to enable the inspiratory conduit to be coupled to the filter to provide fluid/pneumatic communication between the inspiratory conduit and the filter. The complementary coupling features of the gases inlet port 505 of the filter and the gases outlet port 407 of the inspiratory conduit are disconnectable from each other to enable the inspiratory conduit 401 to be decoupled from the filter 501.

The complementary coupling features may be any of the connector assemblies described herein.

The filter 501 is in fluid/pneumatic communication with the patient interface 601 upstream of the patient interface or is configured to be placed in fluid/pneumatic communication with the patient interface 601 upstream of the patient interface 601; i.e. with the patient interface located downstream of the filter. In one configuration, the filter 501 is coupled to the patient interface 601 or is configured to be coupled to the patient interface 601.

The patient interface 601 comprises a patient interface gases conduit 603 with an upstream gases inlet port 605 at one end of the conduit. The opposite downstream end of the patient interface gases conduit 603 is in fluid/pneumatic communication with a patient cannula 607 to deliver gases from the patient interface gases conduit 603 to a patient P.

In one configuration, the gases outlet port 507 of the filter 501 and the gases inlet port 605 of the patient interface gases conduit 603 comprise complementary coupling features to enable the filter 501 to be coupled to the patient interface 601 to provide fluid/pneumatic communication between the filter and the patient interface gases conduit, with the filter in-line with a gases flow path through the patient interface gases conduit. The complementary coupling features may be disconnectable from each other to enable the filter to be decoupled from the patient interface gases tube of the patient interface. Alternatively, the complementary coupling features may be permanently or semi-permanently coupled.

In one configuration, the complementary coupling features between the gases outlet port 507 of the filter 501 and the gases inlet port 605 of the patient interface 601 comprise a 22mm medical taper connection.

In an alternative configuration, the patient interface 601 comprises a patient interface gases conduit 603, and the filter 501 is integrally formed with the patient interface gases conduit 603 to provide fluid/pneumatic communication between the filter 501 and the patient interface gases conduit 603, with the filter in-line with a gases flow path through the patient interface gases conduit. That is, the filter and patient interface may be an integrated unit.

The patient interface 601 is shown to be a nasal cannula, although it should be understood that in some configurations, other patient interfaces may be suitable. For example, in some configurations, the patient interface may comprise a sealing or non-sealing interface, and may comprise a nasal mask, an oral mask, an oro-nasal mask, a full face mask, a nasal pillows mask, a nasal cannula, an endotracheal tube, a tracheostomy tube, a combination of the above or some other gas conveying system. In an embodiment, the patient interface 601 comprises a non-sealing interface such as a nasal cannula, which allows gases to be exchanged with the environment. For example, the non-sealing cannula allows carbon dioxide to be removed and/or cleared from the patient's airways while the patient receives flow therapy from the system. Further, in an embodiment, the patient interface is in the form of a nasal interface, such that the system does not interfere with other oral airway equipment and/or devices, for example, a tracheal tube in an intubation procedure. Accordingly, the patient may continue to receive flow therapy throughout the intubation procedure.

Headgear 620 may be provided to support and retain the patient interface against the patient's face.

In some configurations, there could be one or more therapy accessories downstream of the filter 501 and upstream of the patient interface 601, the one or more accessories in fluid communication with the filter 501 and the patient interface 601. For example, there could be one or more of a nebuliser, a sensor/sensor port, an exhalation port, an additional filter, and/or other accessories.

The patient interfaces, cannula manifolds, and/or conduits may have any one or more of the features described in PCT/IB2015/054585 (published as WO 2015/193833), US 2020/10077764, or US 8,997,747.

In alternative configurations, the patient interface could comprise a non-invasive ventilation (NIV) mask, or any other suitable type of patient interface. The filter 501 could be coupled directly to the patient interface 601 or alternatively could be coupled to the patient interface 601 by one or more accessories.

The patient interface gases conduit 603 forms a first gas lumen defined by a tubular wall. The first gas lumen is adapted to receive gases from the respiratory therapy system, via the inspiratory conduit 401 and filter 501, and channel the gases to the patient P.

In some configurations, the filter 501 is at a terminal end of the patient interface gases conduit 603.

The first gas lumen is defined at least in part by a wall within which gases can be channelled.

The first gas lumen may optionally comprise a reinforcement element adapted to strengthen and/or add rigidity to the first gas lumen to prevent deformation or collapse of the first gas lumen arising due to the application of forces against the first gas lumen. The reinforcement element may include a number of structures, including but not limited to plastic or metallic reinforcing beads that lie in or on the wall of the first gas lumen. Alternatively, the lumen may, in some configurations, comprise weakened sections, or sections which are unable to self-maintain their flow path or a fluid flow passage, to allow for a mask to seal over the patient interface, such as a nasal cannula or nasal mask, and to reduce or prevent the flow of gases to the patient interface.

The patient interface when in the form of a nasal cannula may utilise a pair of side arms extending from the main body (to which the manifold is to be put into connection with). The side arms may comprise of features allowing for the retention or securement or positioning of a gas supply tube to the side arm (to prevent the gas supply tube from uncontrollably moving about).

The patient interface 601 may further comprise mounts and/or supports, e.g., cheek supports, for attaching and/or supporting the gas lumen 603 and/or cannula 607 on the patient's face. For example, a releasable connection system may be utilised to position or locate the interface upon the patent's face, yet allow for a relatively rapid removal or re-positioning of the interface if necessary.

The patient interface or part(s) thereof may be provided in a respiratory therapy kit for use in the respiratory therapy system. The kit may comprise the patient interface (which may comprise a nasal cannula 601 or 5030 for example) and the filter 501 or 1501. The kit may further comprise the interface tube for delivering gases from the outlet port of the filter to the nasal cannula.

The filter 501 may be coupled to an interface tube for delivering gases to the patient interface, such as a patient interface gases conduit 603 or gas lumen, that is coupled to a manifold of the patient interface. The patient interface gases conduit or gas lumen may be a short section of tube or conduit. For example, the patient interface gases conduit or gas lumen may be about 20 cm to about 50 cm long, or about 25 cm to about 40 cm long, or about 30 cm to about 35 cm long, or may be about 32 cm long.

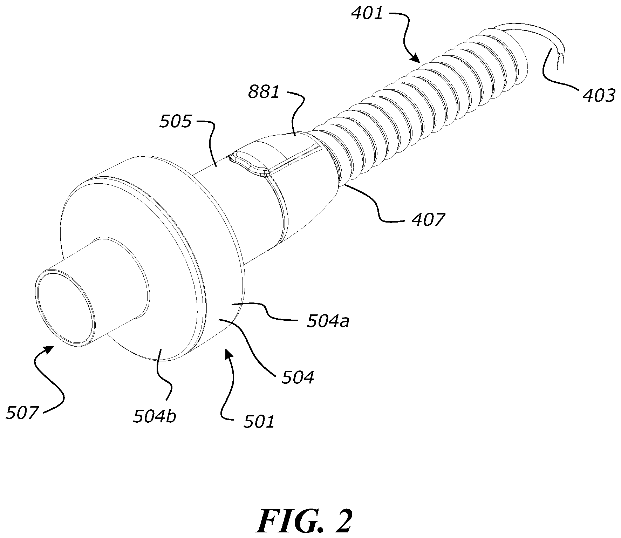

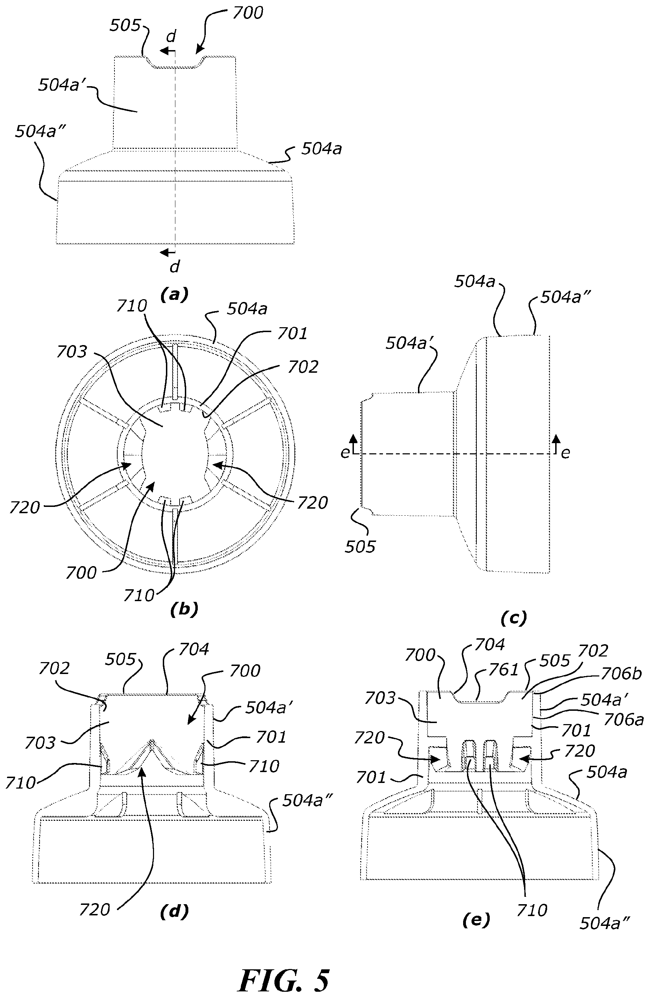

As shown in Figures 2-5 for example, the filter housing 504 of the filter 501 may comprise separate housing portions 504a, 504b that are fastened together to form the filter housing. That enables the filtration material (not shown) to be inserted into the filter housing 504 during assembly of the filter 501.

The filter housing 504 may comprise an upstream housing portion 504a that provides the filter inlet 505 and a downstream housing portion 504b that provides the filter outlet 507.

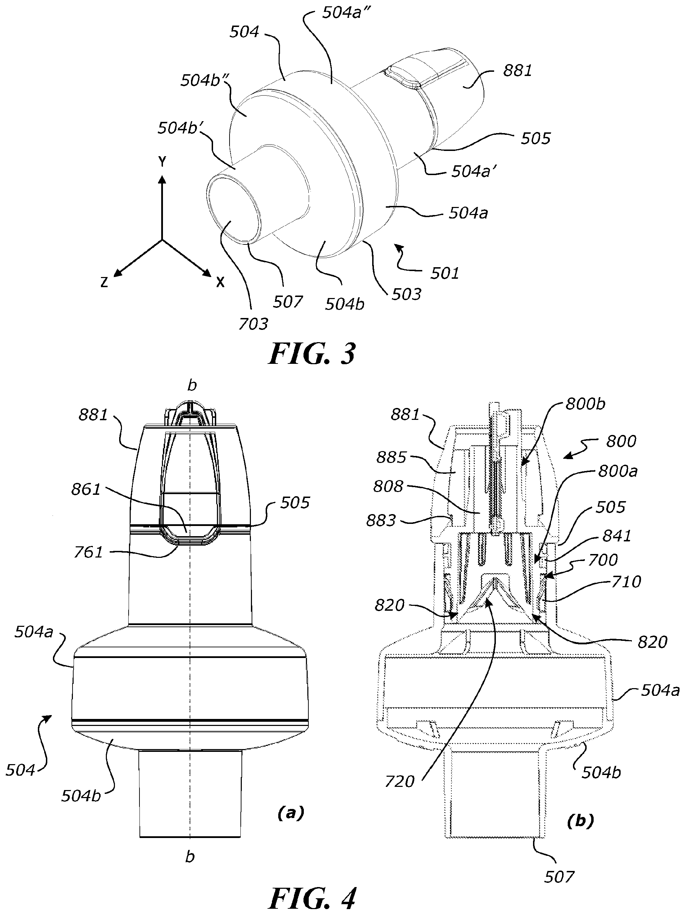

In the configuration shown in Figure 3 for example, the upstream housing portion 504a comprises first tubular body 504a' of relatively small cross-sectional dimension which defines the filter inlet 505 at its terminal end. The opposite end of the first tubular body 504a' connects to an enlarged portion 504a" that forms part of the enlarged central body portion 503 of the filter housing 504.

In the configuration shown, the downstream housing portion 504b comprises a second tubular body 504b' of relatively small cross-sectional dimension which defines the filter outlet 507 at its terminal end. The opposite end of the second tubular body 504b' connects to an enlarged portion 504b" that, together with the enlarged portion 504a", forms the enlarged central body portion 503 of the filter housing 504.

The housing portions 504a, 504b may be permanently connected together once assembled, or may be detachable from each other. The housing portions 504a, 504b may comprise any suitable fastening features to connect them together, such as clips, protrusions and recesses, fasteners, or screw threads for example.

The filter 501 may comprise a surrounding outer wall (not shown) around the filter housing 504 to provide an insulated filter. The filter housing 504 and the surrounding outer wall form therebetween closed air spaces. These closed air spaces insulate the filter housing 504 from the ambient conditions outside the surrounding outer wall. With the enclosed space within the filter 501 being substantially insulated from the ambient conditions outside the outer wall, condensation forming within the filter on the filter walls will be substantially reduced.

Additionally or alternatively, the filter 501 may have one or more other configurations to reduce temperature drop for humidified situations and variable environmental conditions including low ambient temperatures or drafts.

The insulated filter configuration may have any one or more of the features and functionality described in US 62/196,235, US 62/242,549, WO 2016/157101, or US 2018/0078728.

The filter 501 comprises a connector 700 for connecting a first respiratory system component comprising the filter 501 to a second respiratory system component comprising the inspiratory conduit 401. The connector 700 is shown in Figures 4(b), 5, 6, and 7 for example. In alternative configurations or applications, the first respiratory system component and second respiratory system component could comprise different components such as conduit(s), breathing tube(s), patient interface(s), non-invasive ventilation mask(s), T-piece(s) or Y-piece(s) for accessories such as nebulisers or other accessories, nebuliser(s), heat and moisture exchanger(s) (HMEs), cannula(s), or any two suitable components within a respiratory circuit. The connector 700 is provided at the filter inlet 505 of the filter 501.

The connector 700 comprises a body 701 with an inner wall 702 defining a bore 703 for receipt of a complementary connector component 800. The complementary connector component 800 is associated with the inspiratory conduit 401 and is described in more detail below. The connector 700 is a female connector and the complementary connector component 800 is a male connector component.

The bore 703 has a terminal end 704 that provides an entry into the bore 703 for the complementary connector component 800.

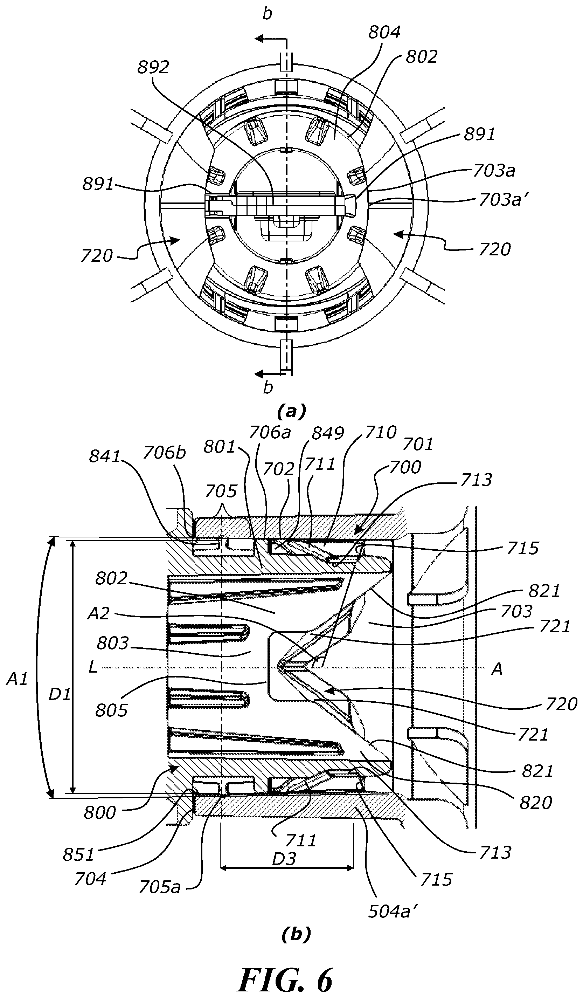

With reference to Figure 6(b), the body 701 of the connector 700 has a sealing region 705 in the bore 703 for engaging with a seal 841 on the complementary connector component 800. The sealing region 705 has a sealing region dimension D1 between opposed surfaces of the sealing region.

The sealing region dimension D1 is in a direction that is normal to the longitudinal axis LA of the bore 703. The longitudinal axis LA is a central axis.

In some configurations, the inner wall 702 that defines the bore 703 is an annular wall having a substantially circular cross-section. In such a configuration, the sealing region dimension D1 is a diameter. In other configurations, the inner wall 702 that defines the bore may have a non-circular cross-section. The geometry may be selected to provide desired flow properties. In those other configurations, the dimension D1 may be a hydraulic diameter. The complementary connector component 800 will be configured accordingly.

The bore 703 has a taper A1 between the opposed surfaces. In some configurations, the sealing region dimension is between about 25 mm and between about 27 mm and the taper A1 is between about 0 degrees and about 4 degrees.

In some configurations, the taper A1 is more than 0 degrees, optionally more than 0.5 degrees, optionally more than 1 degree, optionally more than 1.5 degrees.

In some configurations, the taper A1 is less than 4 degrees, optionally less than 3.5 degrees, optionally less than 3 degrees, optionally less than 2.5 degrees. In some configurations, the taper A1 is about 2 degrees.

In some configurations, the sealing region dimension D1 is between about 25.5 mm and about 26.5 mm, is about 25 mm, is about 25.1 mm, is about 25.2 mm, is about 25.3 mm, is about 25.4 mm, is about 25.5 mm, is about 25.6 mm, is about 25.7 mm, is about 25.8 mm, is about 25.9 mm, is about 26 mm, is about 26.1 mm, is about 26.2 mm, is about 26.3 mm, is about 26.4 mm, is about 26.5 mm, is about 26.6 mm, is about 26.7 mm, is about 26.8 mm, is about 26.9 mm, is about 27 mm, or is any value between any two of those values.

The sealing region 705 may have an effective sealing location 705a for contact by a surface of a seal 841 on the complementary connector component 800. In that configuration, the sealing region dimension D1 is the dimension of the effective sealing location 705a.

In the configuration shown, the seal 841 is a symmetrical T-shaped wiper seal, and the effective sealing location 705a is substantially at a centre of the sealing region 705, for contact by the surface of the seal which is at a centre of the seal. When the seal 841 is an asymmetric or different type of seal (for example having an L-shaped cross-section rather than a T-shaped cross-section), the effective sealing location 705a may not be at the centre of the sealing region 705.

In some configurations, the seal 841 may comprise a plurality of sealing features, and the sealing region may comprise a corresponding plurality of effective sealing locations. For example, the seal 841 could be a double T-seal or any other suitable configuration.

The seal 841 could be formed from any suitable material; including, but not limited, to silicone, nitrile, PTFE, EPDM rubber, or fluorocarbon material for example.

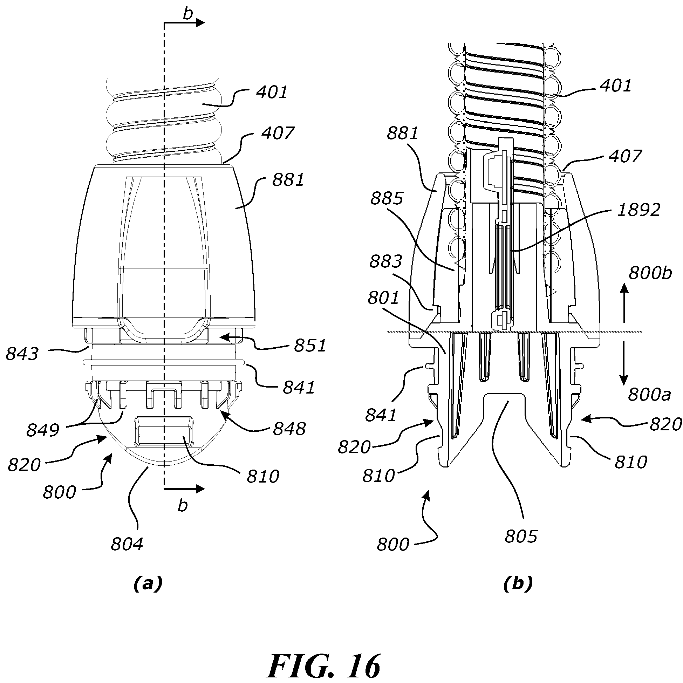

Having a relatively flat (or nearly parallel-walled) bore 703 enables a seal to be created with the seal 841. The small taper A1 is advantageous for manufacturing and also enables the option of interacting with anti-rocking features 851 (Figure 16) on the complementary connector component 800.

The connector 700 comprises at least one internal retaining feature, at least one internal alignment feature, and/or at least one external alignment feature.

The at least one internal retaining feature may comprise two or more retaining protrusions that extend into the bore 703 from an inner wall 702 of the body 701. In the configuration shown, with reference to Figures 4(b), 5(a), 5(e), 6, and 7, the at least one internal retaining feature comprises diametrically opposed retaining protrusions 710 that extend into the bore 703 from an inner wall 702 of the body 701. In alternative configurations, the two or more retaining protrusions may not be diametrically opposed.

The diametrically opposed retaining protrusions are configured for engaging with engagement features 810 on diametrically opposed male engagement fingers 820 of the complementary connector component 800.

The retaining protrusions 710 each have an angled or chamfered entry wall 711 to assist with guiding/deflecting locking fingers 820 radially inward and over the retaining protrusions 710 during insertion of the complementary connector component into the bore 703, a radially inward surface 713, and/or a retaining face 715 to assist with preventing unintended disengagement of the complementary connector component 800 from the bore 703. The radially inward end of the retaining face 715 terminates at the radially inward surface 713.

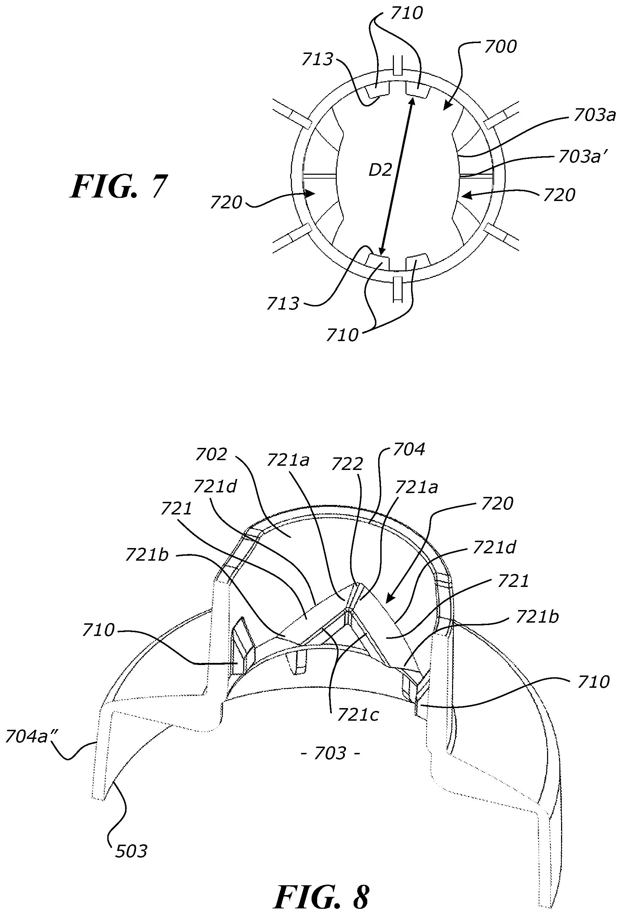

The diametrically opposed retaining protrusions 710 have a distance D2 (Figure 7) between the radially inward surfaces 713 thereof. In some configurations, the distance D2 is between about 21 mm and about 23 mm.

In some configurations, the distance D2 between the radially inward surfaces 713 is between about 21.5 mm and about 22.5 mm, is about 21 mm, is about 21.1 mm, is about 21.2 mm, is about 21.3 mm, is about 21.4 mm, is about 21.5 mm, is about 21.6 mm, is about 21.7 mm, is about 21.8 mm, is about 21.9 mm, is about 22 mm, is about 22.1 mm, is about 22.2 mm, is about 22.3 mm, is about 22.4 mm, is about 22.5 mm, is about 22.6 mm, is about 22.7 mm, is about 22.8 mm, is about 22.9 mm, is about 23 mm, or is any value between any two of those values.

Dimension D1 and distance D2 are related to provide an appropriate retention force and seal between the connector and the complementary connector component (when in use).

The sealing region 705 is closer to the terminal end 704 relative to the internal retaining feature(s) 710 and/or the internal alignment feature(s) 720.

With reference to Figure 6(b), the sealing region 705 comprises a first radial periphery 706a configured to engage a first support protrusion 849 of the complementary connector component 800.

The taper A1 is configured to provide the first radial periphery 706a with a dimension (transverse to the longitudinal axis LA) that is less than the sealing region dimension D1 of the effective sealing location 705a.

The effective sealing location 705a is closer to the terminal end 704 relative to the first radial periphery 706a.

The sealing region 705 comprises a second radial periphery 706b configured to engage a second support protrusion 851 of the complementary connector component 800.

The taper A1 is configured to provide the first radial periphery 706a with a dimension (transverse to the longitudinal axis LA) that is less than a dimension (transverse to the longitudinal axis LA) of the second radial periphery 706b.

The second radial periphery 706b is closer to the terminal end 704 relative to the effective sealing location.

Referring to Figure 6(b), in some configurations an angle A2 of the retaining face 715 is between about 90 degrees and about 125 degrees relative to a longitudinal axis LA of the bore 703.

In some configurations, the angle A2 of the retaining face is between about 95 degrees and about 120 degrees, is between about 100 degrees and 115 degrees, is between about 105 degrees and about 115 degrees, is about 90 degrees, is about 91 degrees, is about 92 degrees, is about 93 degrees, is about 94 degrees, is about 95 degrees, is about 96 degrees, is about 97 degrees, is about 98 degrees, is about 99 degrees, is about 100 degrees, is about 101 degrees, is about 102 degrees, is about 103 degrees, is about 104 degrees, is about 105 degrees, is about 106 degrees, is about 107 degrees, is about 108 degrees, is about 109 degrees, is about 110 degrees, is about 111 degrees, is about 112 degrees, is about 113 degrees, is about 114 degrees, is about 115 degrees, is about 116 degrees, is about 117 degrees, is about 118 degrees, is about 119 degrees, is about 120 degrees, is about 121 degrees, is about 122 degrees, is about 123 degrees, is about 124 degrees, or is about 125 degrees relative to the longitudinal axis LA of the bore, or is any angle between any two of those values.

In the configuration shown, the diametrically opposed retaining protrusions 710 comprise a first pair of adjacent retaining protrusions on one side of the bore and a second pair of adjacent retaining protrusions on an opposite side of the bore. A slot may be provided between the protrusions in each pair. This configuration may be utilised to reduce material usage and enhance strength of the retaining protrusions. In alternative configurations, the diametrically opposed retaining protrusions 710 may each comprise a single protrusion.

In alternative configurations, the at least one internal retaining feature may comprise a single retaining protrusion 710, or may comprise a plurality of retaining protrusions 710 that are not diametrically opposed. For example, three retaining protrusions may be provided around the bore. The three retaining protrusions 710 may be substantially evenly spaced around the bore 703 at about 120 degrees relative to each other.

The retaining face 715 may be provided at any suitable distance D3 from the sealing region 705. In some configurations, an axial distance between the sealing region 705 and the retaining face 715 is up to about 17 mm.

In some configurations, the axial distance between the sealing region 705 and the retaining face 715 is shorter. For example, the axial distance between the sealing region 705 and the retaining face 715 may be at least about 1 mm and up to about 17 mm, optionally more than about 3 mm and up to about 17 mm, optionally more than about 3 mm and up to about 14 mm.

In some configurations, the axial distance D3 between the sealing region 705 and the retaining face 715 is about 1 mm, about 2 mm, about 3 mm, about 4 mm, about 5 mm, about 6 mm, about 7 mm, about 8 mm, about 9 mm, about 10 mm, about 11 mm, about 12 mm, or about 13 mm, about 14 mm, about 15 mm, about 16 mm, about 17 mm, or is any distance between any two of those values. In some configurations, the distance D3 is between about 12.5 mm and about 15 mm, optionally between about 13 mm and 14.5 mm, optionally between about 13.5 mm and about 14 mm, optionally about 13.75 mm.

The retaining face 715 may be provided on at least one retaining protrusion 710 that extends into the bore 703 from the inner wall 702 of the body 701. Alternatively, the retaining face 715 may be provided elsewhere in the bore 703. For example, the retaining face could be provided in a slot or recess in the body 701, or on a different protrusion that protrudes into the bore 703 from the inner wall 702 of the body 701.

The axial distance D3 between the sealing region 705 and the retaining face 715 may be the axial distance between the effective sealing location 705a and the retaining face 715. Alternatively, if the seal 841 is of a type that contacts the sealing region 705 over a larger area (such as an O-ring for example), the axial distance D3 may be the axial distance between a centre of the sealing region 705 and the retaining face 715 or may be the axial distance between the retaining face 715 and the edge of the sealing region 705 that is closest to the retaining face 715.

The axial distance D3 between the sealing region 705 and the retaining face 715 will be selected for appropriate retention and sealing.

The sealing region 705 is closer to the terminal end 704 relative to the retaining face 715.

An axially oriented recess 761 located in the body 701 of the connector 700 at the terminal end 704 of the bore 703.

The retaining face 715 is axially aligned with the axially oriented recess 761; i.e. along the Y axis of Figure 3.

The axially oriented recess 761 extends from a radially inner surface of the body 701 to a radially outer surface of the body 701.

The at least one internal alignment feature may comprise at least one alignment member 720 as shown in Figures 4(b), 5(b), 5(d), 6, 7, 8, 9, 10, and 20 for example.

The alignment member 720 may be at a 90 degree offset around the bore from the retaining face 715.

The alignment member 720 extends into the bore 703 from the inner wall 702 of the body 701. The alignment member 720 comprises opposed engagement surfaces 721. The opposed engagement surfaces are outwardly facing. The alignment member 720 has a tapered configuration in an axial direction of the bore 703 wherein proximal ends 721a of the opposed engagement surfaces 721 that are more proximal to the terminal end 704 of the bore 703 are closer together than distal ends 721b of the opposed engagement surfaces 721 that are more distal from the terminal end 704 of the bore 703. The engagement surfaces 721 comprise a twist along at least a substantial part of a length thereof between their proximal ends 721a and their distal ends 721b.

In some configurations, the twist extends along substantially the entire length of each engagement surface 721.

In some configurations, the twist is substantially continuous along the length of each engagement surface 721.

The alignment member 720 comprises a tapered configuration in a radial direction of the bore, wherein inner edges 721c of the opposed engagement surfaces 721 more proximal to the longitudinal or central axis LA of the bore are positioned closer together than outer edges 721d of the opposed engagement surfaces 721 more distal from the longitudinal or central axis LA of the bore and more proximal to the inner wall 702.

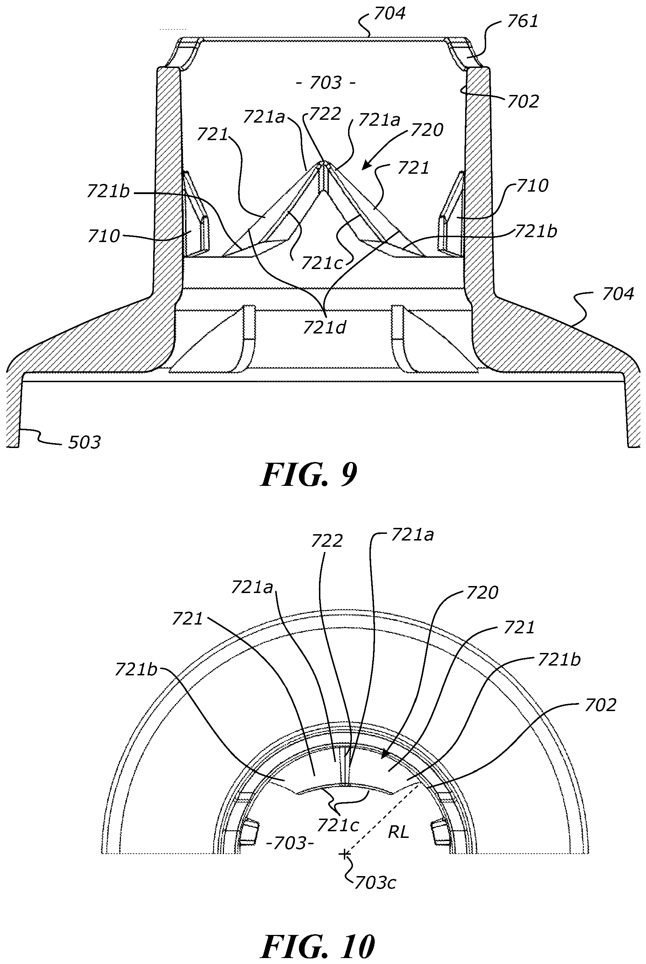

With reference to Figure 10, the inner edge 721c of each engagement surface 721 is positioned inwardly of an imaginary radial line RL extending from the outer edge 721d of the engagement surface to the longitudinal or central axis LA of the bore.

Each engagement surface 721 may be considered to be made up of an infinite number of inwardly projecting lines extending into the bore 703 from the outer edge 721d, with each line being rotated relative to an adjacent line

When viewed from the terminal end 704 of the bore, due to the twist, the proximal end 721a of the engagement surface 721 (i.e. the line that forms that proximal end) is angularly offset by an angle of greater than 0 degrees and less than 90 degrees, optionally at least about 30 degrees and less than 90 degrees, optionally between about 35 degrees and about 85 degrees, optionally between about 40 degrees and about 80 degrees, optionally between about 45 degrees and about 75 degrees, optionally between about 50 degrees and about 70 degrees, optionally between about 55 degrees and about 65 degrees, optionally at least about 60 degrees relative to the distal end 721b (i.e. the line that forms that distal end). The line at the proximal end 721a may extend in a radial direction of the bore, and the line at the distal end 721b may be offset from the radial direction.

In some configurations, the proximal end 721a is angularly offset by at least about 65 degrees, at least about 70 degrees, at least about 75 degrees, at least about 80 degrees, at least about 85 degrees, about 90 degrees, or any angle between any two of those angles, relative to the distal end 721b when viewed from the terminal end of the bore.

In the configuration shown, the alignment member 720 has a substantial chevron or inverted "V", generally "n", or uppercase delta "Δ" shape. However, because the underside of the alignment member 720 that is closer to the centre of the filter 501 does not contribute to the alignment of the complementary connector component 800 upon insertion into the bore, the underside of the alignment member could have a different shape. For example, the underside of the alignment member 720 could be solid to connect the two sides of the alignment member.

The connector 700 may comprise two diametrically opposed alignment members 720. Each alignment member 720 may have substantially the same shape and functionality.

The engagement surfaces 721 are configured to engage with complementary engagement surfaces 821 on the complementary connector component 800.

The engagement surfaces 721 are configured to interact with the complementary engagement surfaces 821 to cause the complementary connector component 800 to rotate into a correct alignment about a longitudinal axis LA of the connector, if the complementary connector component 800 is misaligned upon initial insertion into the bore 703 of the connector 700.

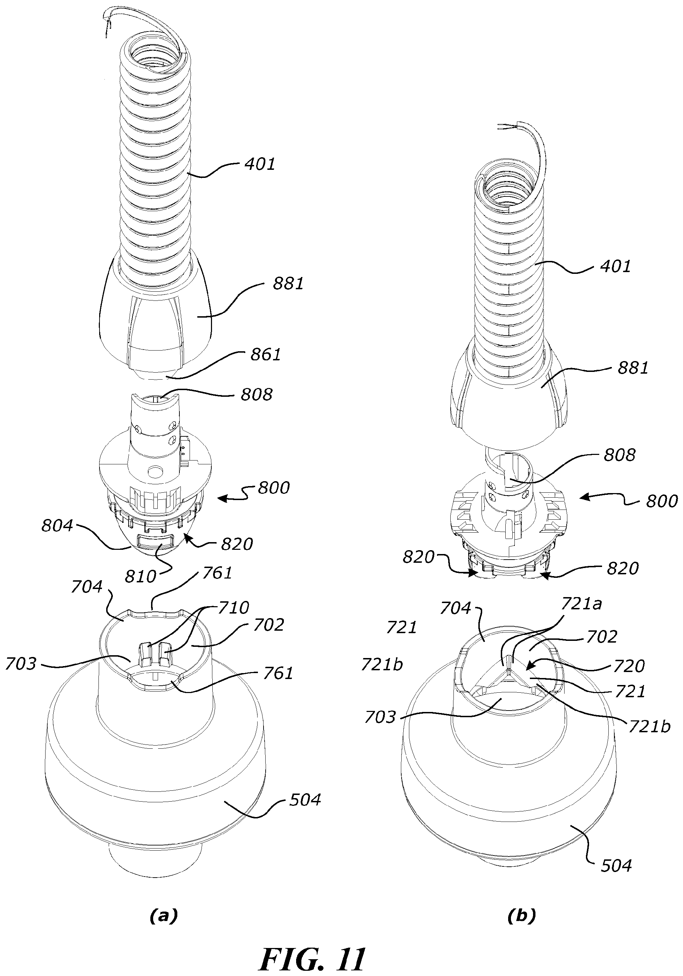

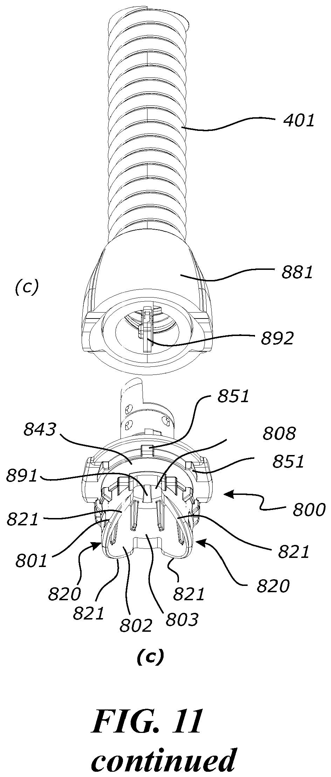

As shown in Figure 11, 16, 17, 18, and 20 for example, the complementary engagement surfaces 821 comprise an opposite tapered configuration in an axial direction of the complementary connector component wherein proximal portions 821a of the opposed engagement surfaces that are more proximal to the terminal end 804 of the complementary connector component 800 that is receivable in the bore 703 of the connector 700 are further apart than distal portions of the complementary engagement surfaces. The complementary engagement surfaces 821 comprise complementary twists along at least a substantial part of a length thereof between their proximal portions 821a and their distal portions 821b.

Each engagement surface 721 is configured to contact a respective one of the complementary engagement surfaces 821 over at least a major part of the length thereof when the connector 700 is engaged with the complementary connector component 800. In some configurations, each engagement surface 721 is configured to contact the respective one of the complementary engagement surface 821 over substantially the entire length thereof when the connector 700 is engaged with the complementary connector component 800.

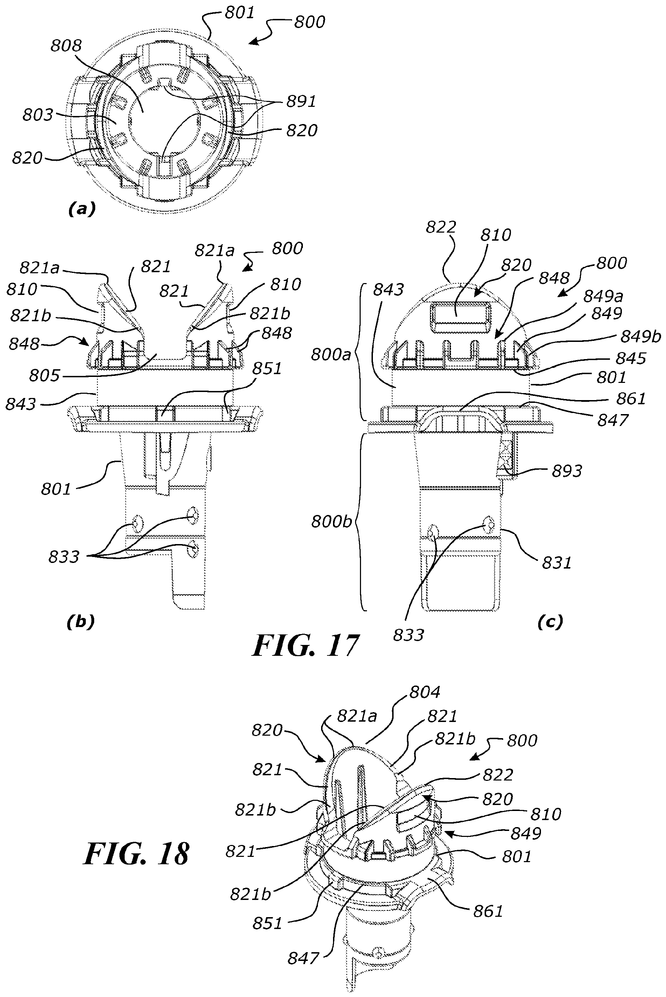

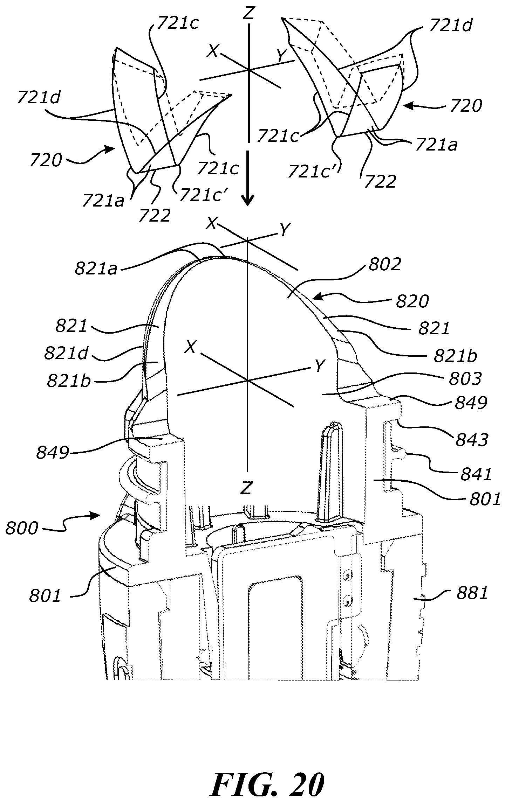

The twist of each engagement surface 721 and each complementary engagement surface 821 may be a substantially helical twist. Figure 20 shows the shape of one of the complementary engagement surfaces 821. As outlined above for engagement surface 721 the engagement surface 821 may be considered to be made up of an infinite number of inwardly projecting lines extending inwardly from the outer edge 821d of the engagement surface to an inner edge 821c of the engagement surface, with each inwardly projecting line being rotated relative to an adjacent line. In the configuration shown in Figure 17, the distal portion 821b is up to 90 degrees offset (e.g. is oriented at or close to a y-axis direction) relative to the proximal portion 821a (which e.g. is oriented at or close to an x-axis direction) with the twist continuing along the length of the engagement surface 821 in the z-direction.

Similarly, Figure 20 shows the shape of one of the engagement surfaces 721. In the configuration shown in Figure 20, the distal portion 721b is up to 90 degrees offset (e.g. is oriented at or close to an x-axis direction) relative to the proximal portion 721a (which e.g. is oriented at or close to y-axis direction) with the twist continuing along the length of the engagement surface 721 in the z-direction.

The angular offset for the ends of the engagement surfaces 721 and complementary engagement surfaces 821 may be smaller for a larger number of engagement surfaces. For example, if the connector 700 has three alignment members 720 and the complementary connector component 800 has three locking fingers 820 with opposed complementary engagement surfaces 821, the angular offset may be up to about 60 degrees.

In the configuration shown, the two proximal ends 721a of the alignment members 720 meet at a tip 722. The tip 722 may be substantially pointed. Alternatively, that tip 722 may be rounded or filleted and may have a radius between the proximal ends 721a. By having a point or a curve at the tip, contact between the tip and a surface of the locking finger(s) 820 at the proximal end 804 of the complementary connector component 800 will cause the complementary connector component 800 to rotate into alignment (about the longitudinal axis LA) during insertion into the bore 703 if that is initially misaligned.

The alignment members 720 may be angled differently from what is shown. The alignment members may be formed from non-continuous sections.

The connector 700 and connector component 800 may be provided together in a connection assembly for use in a respiratory system.

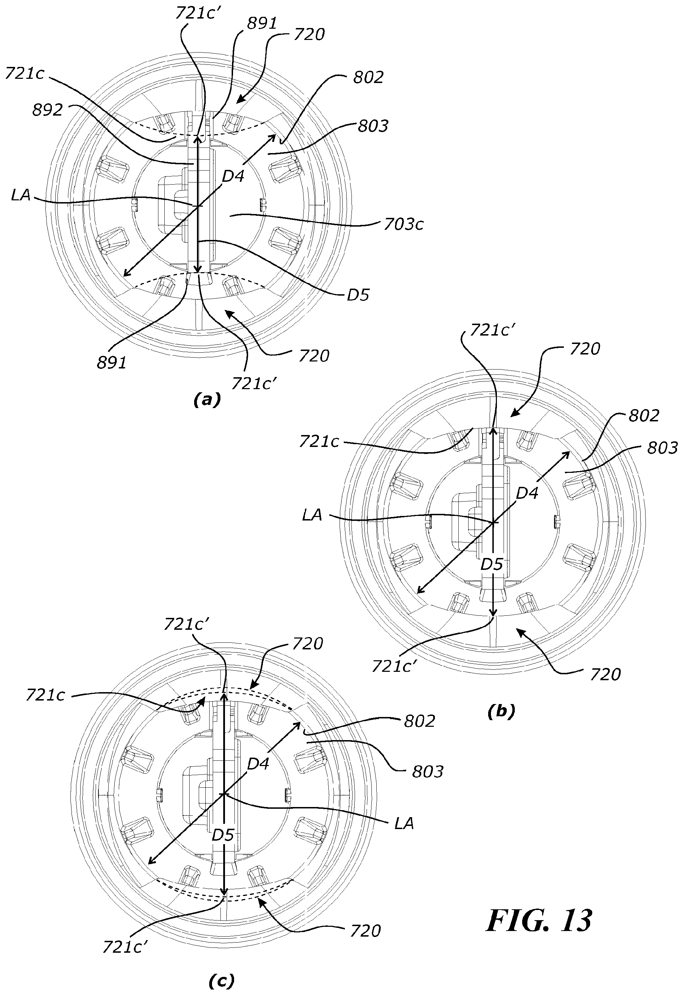

Referring to Figures 13(a) to (c), the bore 803 of the complementary connector component 800 has a flow path dimension D4 that is transverse to the central axis LA.

The flow path dimension D4 may be defined by an inner wall 802 of the locking fingers 820.

A first distance D5 between the radially innermost surfaces 721c' of the two diametrically opposed alignment members 720 is smaller than the flow path dimension D4 of the complementary connector component.

In some configurations, a ratio of the first distance D5 to the flow path dimension D4 is between about 0.6 and about 0.95.

In some configurations, the ratio may be about 0.6, about 0.65, about 0.7, about 0.75, about 0.8, about 0.85, about 0.9, about 0.95, or any ratio between any two of those values.

In some configurations, the first distance D5 is between about 11.5 mm and about 18 mm.

In some configurations, the first distance D5 is between about 12 mm and about 18 mm, between about 13 mm and about 18 mm, between about 14 mm and 18 mm, between about 15 mm and 18 mm, between about 16 mm and about 17 mm, about 11.1 mm, about 11.2 mm, about 11.3 mm, about 11.4 mm, about 11.5 mm, about 11.6 mm, about 11.7 mm, about 11.8 mm, about 11.9 mm, about 12 mm, about 12.1 mm, about 12.2 mm, about 12.3 mm, about 12.4 mm, about 12.5 mm, about 12.6 mm, about 12.7 mm, about 12.8 mm, about 12.9 mm, about 13 mm, about 13.1 mm, about 13.2 mm, about 13.3 mm, about 13.4 mm, about 13.5 mm, about 13.6 mm, about 13.7 mm, about 13.8 mm, about 13.9 mm, about 14 mm, about 14.1 mm, about 14.2 mm, about 14.3 mm, about 14.4 mm, about 14.5 mm, about 14.6 mm, about 14.7 mm, about 14.8 mm, about 14.9 mm, about 15 mm, about 15.1 mm, about 15.2 mm, about 15.3 mm, about 15.4 mm, about 15.5 mm, about 15.6 mm, about 15.7 mm, about 15.8 mm, about 15.9 mm, about 16 mm, about 16.1 mm, about 16.2 mm, about 16.3 mm, about 16.4 mm, about 16.5 mm, about 16.6 mm, about 16.7 mm, about 16.8 mm, about 16.9 mm, about 17 mm, about 17.1 mm, about 17.2 mm, about 17.3 mm, about 17.4 mm, about 17.5 mm, about 17.6 mm, about 17.7 mm, about 17.8 mm, about 17.9 mm, about 18 mm, or is any distance between any two of those values.

In some configurations, the flow path dimension D4 is between about 10 mm and about 25 mm, between about 11 mm and about 24 mm, between about 12 mm and about 23 mm, between about 13 mm and about 22 mm, between about 14 mm and about 21 mm, between about 15 mm and about 20 mm, between about 16 mm and about 20 mm, between about 17 mm and about 20 mm, between about 18 mm and about 20 mm, about 10 mm, about 11 mm, about 12 mm, about 13 mm, about 14 mm, about 15 mm, about 16 mm, about 17 mm, about 18 mm, about 19 mm, about 20 mm, about 21 mm, about 22 mm, about 23 mm, about 24 mm, about 25 mm, or is about 19.1 mm, or is any value between any two of those values. The radially innermost surfaces 721c' comprise centres of the alignment members 720. That is, the radially innermost surfaces 721c' are positioned at or adjacent the proximal ends 721a of the engagement surfaces 720 or at or adjacent the tip 722. Laterally outward edges of the alignment members (which are at the distal ends 721b of the engagement surfaces 721) connect to the inner wall 702 of the bore 703 of the connector 700.

When viewed from the terminal end 704 of the bore 703, the inner edge 721c of the alignment member could have any suitable shape, including concave relative to the longitudinal or central axis LA of the bore (as shown in the intermediate configuration of Figure 13(b) or the maximum dimension configuration shown in broken lines in Figure 13(c)) or convex (as shown in the minimum dimension configuration in broken lines in Figure 13(a)) for example.

By having a reduced distance between the radially innermost surfaces 721c' of the alignment members 720, oblique angle misconnection of the complementary connector component 800 to the connector 700 is inhibited if the alignment features are misaligned.

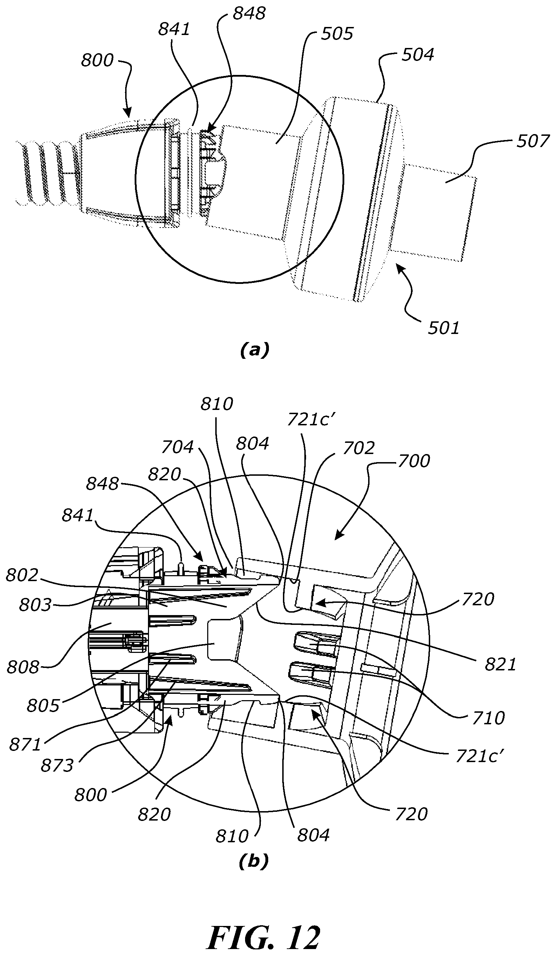

As shown in Figure 12(b), the tip of the locking finger 820 will impact on the alignment member 720 rather than the engagement feature 810 on the locking finger coupling with the peak of the alignment member 720. However, this is balanced against having a sufficiently large first distance D5 to not significantly increase flow resistance through the connection assembly. An upper bound of the first distance D5 is determined to inhibit undesired misconnection occurring by tilting the complementary connector component 800 to an oblique angle relative to the connector 700.

In one exemplary configuration, the minimum first distance D5 is 11.5 mm, an exemplary first distance is 16.4 mm, and a maximum first distance is 18 mm. The locking fingers 820 may, in this exemplary configuration, have an outer dimension of 21.5 mm and an inner dimension of 19.5 mm.

In some configurations, the first respiratory system component (which in the configuration shown comprises the filter 501) has a pressure drop (resistance to flow) in use through the first respiratory system component of between about 80 Pa and about 490 Pa.

In some configurations, the one or more retaining features of the connector 700 are configured to provide a retention force in an axial direction of the connector of between about 10 N and about 100 N, optionally between about 10 N and about 75 N, optionally between about 10 N and about 50 N. That is, an axial pull out force required to remove the complementary connector component 800 from the bore 703 of the connector 700 is between about 10 N and about 100 N, optionally between about 10 N and about 75 N, optionally between about 10 N and about 50 N.

In some configurations, the one or more retaining features are configured to provide a retention force of about 10 N, 11 N, about 12 N, about 13 N, about 14 N, about 15 N, about 16 N, about 17 N, about 18 N, about 19 N, about 20 N, about 21 N, about 22 N, about 23 N, about 24 N, about 25 N, about 26 N, about 27 N, about 28 N, about 29 N, about 30 N, about 31 N, about 32 N, about 33 N, about 34 N, about 35 N, about 36 N, about 37 N, about 38 N, about 39 N, about 40 N, about 41 N, about 42 N, about 43 N, about 44 N, about 45 N, about 46 N, about 47 N, about 48 N, about 49 N, about 50 N, about 51 N, about 52 N, about 53 N, about 54 N, about 55 N, about 56 N, about 57 N, about 58 N, about 59 N, about 60 N, about 61 N, about 62 N, about 63 N, about 64 N, about 65 N, about 66 N, about 67 N, about 68 N, about 69 N, about 70 N, about 71 N, about 72 N, about 73 N, about 74 N, about 75 N, about 76 N, about 77 N, about 78 N, about 79 N, about 80 N, about 81 N, about 82 N, about 83 N, about 84 N, about 85 N, about 86 N, about 87 N, about 88 N, about 89 N, about 90 N, about 91 N, about 92 N, about 93 N, about 94 N, about 95 N, about 96 N, about 97 N, about 98 N, about 99 N, about 100 N, or is any value between any two of those values.

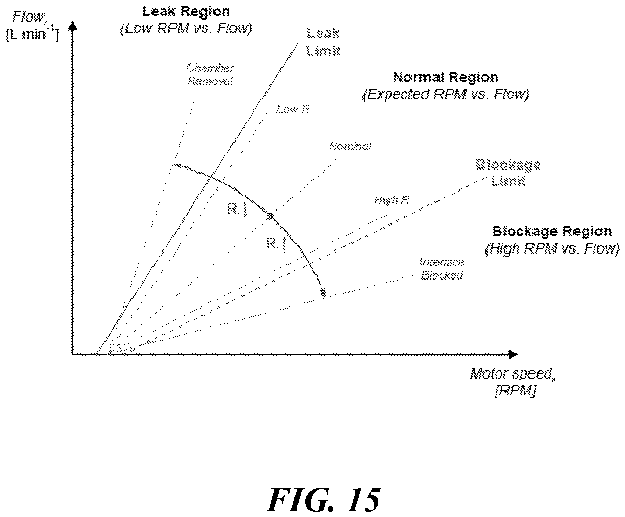

An exemplary dry filter has a pressure drop of about 80-460 Pa through the filter over flow rates from 20-70 L/min, providing an axial force applied to the filter 501 of about 1.5-11 N (+/- 10%). The pressure drops and forces for a given flow rate will be higher for a saturated filter. The axial forces applied to the filter 501 will be resisted by the one or more retaining features 710 of the connector 700.As shown in Figure 15, the respiratory therapy device may include a leak limit (defining maximum allowable leak) and a blockage limit (defining maximum resistance to flow) that are based on allowable motor speeds for the blower 15 to achieve a given flow.

Resistance to flow of the downstream component (e.g. the filter) results in the seal 841 being used to seal between the connector 700 and the complementary connector component 800. The seal 841 improves the seal between the terminal end 407 of the inspiratory conduit 401 and the filter inlet 505.

The sealing region 705 may have a sealing region dimension D1 as outlined above.

The body 701 may have diametrically opposed retaining protrusions 710 as outlined above. The distance D2 between radially inward surfaces 713 of the diametrically opposed retaining protrusions 710 may be between about 21 mm and about 23 mm.

In some configurations, the distance between the radially inward surfaces 713 is between about 21.5 mm and about 22.5 mm, or is about 21 mm, is about 21.1 mm, is about 21.2 mm, is about 21.3 mm, is about 21.4 mm, is about 21.5 mm, is about 21.6 mm, is about 21.7 mm, is about 21.8 mm, is about 21.9 mm, is about 22 mm, is about 22.1 mm, is about 22.2 mm, is about 22.3 mm, is about 22.4 mm, is about 22.5 mm, is about 22.6 mm, is about 22.7 mm, is about 22.8 mm, is about 22.9 mm, is about 23 mm, or is any value between any two of those values.

Each of the diametrically opposed retaining protrusions 710 may have a retaining face 715 to assist with preventing disengagement of the complementary connector component 800 from the bore 703, wherein the angle A2 of the retaining face is between about 90 degrees and about 125 degrees relative to a longitudinal axis of the bore.

In some configurations, the angle A2 of the retaining face 715 is between about 95 degrees and about 120 degrees, is between about 100 degrees and 115 degrees, is between about 105 degrees and about 115 degrees, is about 90 degrees, is about 91 degrees, is about 92 degrees, is about 93 degrees, is about 94 degrees, is about 95 degrees, is about 96 degrees, is about 97 degrees, is about 98 degrees, is about 99 degrees, is about 100 degrees, is about 101 degrees, is about 102 degrees, is about 103 degrees, is about 104 degrees, is about 105 degrees, is about 106 degrees, is about 107 degrees, is about 108 degrees, is about 109 degrees, is about 110 degrees, is about 111 degrees, is about 112 degrees, is about 113 degrees, is about 114 degrees, is about 115 degrees, is about 116 degrees, is about 117 degrees, is about 118 degrees, is about 119 degrees, is about 120 degrees, is about 121 degrees, is about 122 degrees, is about 123 degrees, is about 124 degrees, or is about 125 degrees relative to a longitudinal axis of the bore, or is any angle between any two of those values.

The diametrically opposed retaining protrusions 710 may comprise a first pair of adjacent retaining protrusions on one side of the bore 703 and a second pair of adjacent retaining protrusions on an opposite side of the bore 703.

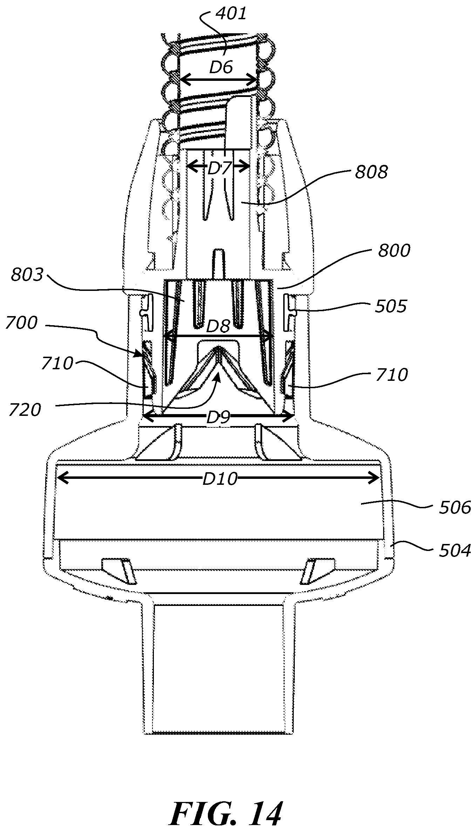

Referring to Figure 14, the filter housing 504 comprises a cavity 506 containing the filtration material (not shown) in a flow path through the filter housing. The cavity 506 has a first cross-sectional area in the region of the filtration material, the first cross-sectional area related to dimension D10 in Figure 14. The filter 501 comprises the connector 700 having a body 701 defining the bore 703 for receipt of the complementary connector component 800. The bore has a proximal end in fluid communication with the cavity 506 and the terminal end 704 that provides an entry into the bore 703 for the complementary connector component 800.

The filter 501 is provided in combination with the complementary connector component 800, the complementary connector component having a portion 800a that is received in the bore 703 via the terminal end 704 in use. That portion 800a of the complementary connector component comprises a complementary connector component bore 803. The complementary connector component bore 803 has a second cross-sectional area related to dimension D8 in Figure 14.

A ratio of the second cross-sectional area to the first cross-sectional area is greater than 0 and less than 1.

In some configurations, the ratio is at least about 0.05, optionally at least about 0.1. optionally about 0.11. The bore 803 of the complementary connector component 800 may be an outlet bore 803 of the complementary connector component 800. The outlet bore 803 may be in fluid communication with an inlet bore 808 of the complementary connector component.

The inlet bore 808 has a third cross-sectional area related to dimension D7 in Figure 14. A ratio of the third cross-sectional area to the second cross-sectional area may be at least about 0.36.

The cross-sectional areas may progressively increase from the inlet bore 808 of the complementary connector component to the filter cavity 506. This has implications for controlling resistance to flow and establishing a residence time and/or velocity for the respiratory gases interacting with the filtration material. For example, by increasing the cross-sectional area prior to the filtration material, jetting of gases at the surface of the filtration material is inhibited, which could otherwise adversely affect filtration.

As outlined above, the resistance to flow through the filter may be between about 80 Pa and about 490 Pa.

**Table 1**

| **Ref** | **Description** | **Value (mm)** | **Cross-sectional area (mm²)** |
|---|---|---|---|
| D6 | Tube transverse dimension/diameter (inspiratory conduit 401) | 13.0 | 132.7 |
| D7 | Complementary connector component inlet bore inner transverse dimension/diameter | 11.3 | 100.2 |
| D8 | Complementary connector component outlet bore inner transverse dimension/diameter | 18.7 | 274.5 |
| D9 | Filter inlet transverse dimension/diameter | 25.6 | 514.5 |
| D10 | Filtration material transverse dimension/diameter | 54.8 | 2358.6 |

Table 1 outlines one exemplary set of dimensions for the components. However, it will be appreciated that the dimensions could vary without departing from the scope of this concept. For example, D6 could be up to about 18 mm or larger, D7 could proportionally larger, and so on.

The filter 501 disclosed herein may comprise or be provided in combination with the connector 700 or the connection assembly comprising the connector 700 and the complementary connector component 800.

The connector 700 may be attached to or integrally formed with the filter housing 504 of the filter 501.

The filter 501 may be provided with a suitable filtration material in the filter housing 504 as discussed above.

While the connection assembly comprising the connector 700 and complementary connector component 800 are described with reference to connecting a filter inlet 505 to an outlet 407 of an inspiratory conduit 401, the connection assembly could be used to connect any suitable first respiratory system component to any suitable second respiratory system component. For example, the first respiratory system component and the second respiratory system component could comprise different components such as conduit(s), breathing tube(s), patient interface(s), non-invasive ventilation mask(s), T-piece(s) or Y-piece(s) for accessories such as nebulisers or other accessories, nebuliser(s), heat and moisture exchanger(s) (HMEs), cannula(s), or any two suitable components within a respiratory circuit.

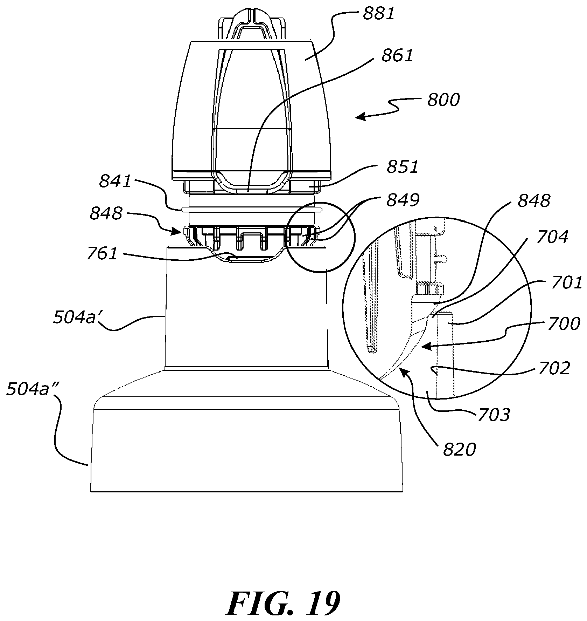

Figures 4, 6, 11, 12, 13, 14, and 16 to 20 show details of the complementary connector component 800 that can be used with the connector 700 in the connection assembly.

The complementary connector component 800 has a proximal portion 800a that is received in the bore 703 of the connector 700 when assembled with the connector 700. A distal portion 800b is provided at the terminal end 407 of the inspiratory conduit 401. The distal portion 800b can be directly connected to the terminal end 407 of the inspiratory conduit or could be connected thereto by one or more accessories.

As shown in Figure 17, in some configurations the distal portion 800b may comprise a lead in region or connection portion 831 for receipt of the terminal end 407 of the inspiratory conduit 401.

The lead in region 831 may comprise one or more protrusions 833 to assist with maintaining the inspiratory conduit 401 in engagement with the complementary connector component 800.

When a plurality of protrusions 833 are provided, the protrusions 833 may be formed along a generally helical pathway such that the inspiratory conduit 401, which have an internally threaded surface, may be threaded onto the connection portion 831.

In some configurations, the protrusions 833 may be axially arranged on one side of the connection portion 831. That is, there may be two or more protrusions 833 on one side of the connection portion 831. The axially aligned protrusions 833 will also fall within the helical pathway.

In alternative configurations, the connection portion 831 may include an external thread. The thread may be a full thread that extends about the tubular member. Alternatively, the thread may be a partial thread having short portions of thread with gaps or spaces between the short portions.

The inspiratory conduit 401 may be spirally wound, with the path of the protrusions 833 on the connection portion 831 being arranged to generally match the taper/helical arrangement of the spirally wound conduit. In some embodiments, the path of the protrusions does not precisely match the helical arrangement of the spirally wound conduit, but the path is designed such that the location of the protrusions will engage with the thread of the inspiratory conduit 401.The protrusions may be arranged such that they are positioned at an edge/boundary of the internal threaded portion of the inspiratory conduit 401. Using a series of protrusions 833 instead of a typical helical thread makes the assembly more tolerant of variances in tube dimensions.

In some configurations, the protrusions 833 would be arranged to match a single revolution of the taper, with only the first and last protrusion being axially aligned. The first and last protrusion 833 being axially aligned also prevents, or at least substantially inhibits, axial movement of the inspiratory conduit.

The inspiratory conduit 401 may be a composite structure made of two or more distinct components that are spirally wound to form an elongate tube. A suitable conduit is the tube described in WO 2012/164407 or US 10,080,866. The protrusions 833 would be placed to line up with this tube, such that the protrusions engage with the member by causing the hollow lumen to be compressed.

A second spirally wound member may be interwoven with the first member, with the second member being made of solid plastic. The windings of the second member on either side of the first member would prevent axial movement of the inspiratory conduit 401 once the protrusions 833 are engaged with first member, as the second member would be substantially incompressible and therefore unable to pass over the protrusion. Slight axial movement could still be possible due to the first member being slightly wider than the protrusions themselves, thereby allowing the protrusions 833 to shift between the boundaries created by the two adjacent windings of the second member.

This axial movement may be prevented/limited by the two axially aligned protrusions 833 described above. The placement of the protrusions 833 can be altered such that one of the protrusions 833 engages with the lower boundary of the first spiral member, while the other protrusion 833 engages with an upper boundary of the first spiral member.

In some configurations, the location of the protrusions 833 may be chosen or designed such that the protrusions pinch adjacent/neighbouring helical portions of the tube. During manufacturing, before the inspiratory conduit 401 and complementary connector component 800 are connected, the wires of the conduit are exposed. The complementary connector component 800 could have an alignment or lead in feature that locates the exposed wires of the inspiratory conduit 401. The alignment or lead in feature could comprise tapered channels, which taper from a wide entrance down to a narrow exit. This allows the wires to be reliably guided towards the desired location, such that the electrical contacts on the terminal end of the wires may be soldered onto the electrical pins.

The proximal portion 800a comprises one or more male connection features or locking fingers 820 that extend to the proximal end 804 of the complementary connector component 800.

In the configuration shown, the complementary connector component 800 comprises a pair of diametrically opposed locking fingers 820. In some configurations, the complementary connector component 800 comprises one, two, three, four, or more locking fingers 820.

The locking finger(s) 820 comprise an engagement feature 810 on an outer surface of the finger (s) 820. The engagement feature(s) 810 is/are configured to engage with the retaining protrusion(s) 710 of the connector 700.

In some configurations, the engagement feature 810 comprises a recess or aperture for receiving the respective retaining protrusion 710.

When engaged, an edge of the engagement feature 810 that is closest to the proximal end 804 of the complementary connector component (and closest to a tip 822 of the locking finger) engages with the retaining face 715 of the retaining protrusion 710 to assist with preventing disengagement of the complementary connector component from the bore.

The engagement feature(s) 810 interact with the retaining face(s) 715 of the retraining protrusion(s) 710 in use to provide the retention forces described above. The retention forces are selected to enhance intentional connection and disconnection, and to minimise the likelihood of unintentional disconnection.

The tip 822 of the locking finger(s) 820 may be of an at least partially chamfered configuration. That is, at least a portion of the tip 822 of the locking finger(s) 820 may have a chamfered configuration.

Interaction of the engagement surfaces 721 and complementary engagement surfaces 821 evoke a relative rotation between the components when an axial force is applied during insertion, causing the complementary connector component 800 to rotate into alignment (about the longitudinal axis LA) during insertion into the bore 703 of the connector 700 if the complementary connector component 800 and the connector 700 are initially rotationally misaligned. This assists with guiding the engagement feature(s) 810 into alignment with the retaining protrusion(s) 710 if they are initially misaligned.

Each locking finger 820 has the opposed complementary engagement surfaces 821 as described above for engaging with the engagement surfaces 721 of the alignment member(s) 720. The complementary engagement surfaces 821 comprise twists as described above. As well as providing a guiding feature, the twists of the engagement surfaces 721 and the complementary engagement surfaces 821 can provide haptic feedback during connection.

The interaction of the alignment member(s) 720 and the locking finger(s) 820 may provide a small torsional coupling between the filter inlet 505 and the terminal end 407 of the inspiratory conduit 401 which inhibits unintended disconnection owing to torque applied on the filter body 504.

The engagement surfaces 721 and complementary engagement surfaces 821 are configured so that a user can rotate the connector 700 about the longitudinal axis LA relative to the complementary connector component 800 to disconnect those components from one another. This method of disconnection is intended to be easier to implement than directly pulling the components apart in an axial direction. The engagement surfaces 721 and 821 will interact to separate the connector 700 and the complementary connector component 800 in an axial direction. Due to the twists and extensive contact between the engagement surfaces 721 and the complementary engagement surfaces 821, rotation of the connector 700 relative to the complementary connector component 800 will advantageously apply a substantially constant axial force to the connector 700 and the complementary connector component 800 in the axial direction to separate them. Alternatively, a user can simply pull the connector 700 and complementary connector component 800 apart in the axial direction.

In some configurations, the engagement surfaces 721, 821 may be asymmetric, so that the complementary connector component 800 and connector 700 can only rotate into engagement (if initially misaligned) in one direction (e.g. clockwise), and can only be rotated out of engagement in the opposite direction (e.g. anti-clockwise).

A recess 805 is provided between the bases of the locking fingers 820. The recess 805 is configured to receive the tip 722 of the alignment feature 721 when the proximal portion 800a is fully received in the bore 703 of the connector 700. In the configuration shown there are two recesses 805 between two locking fingers 820 to receive two tips 722 of two alignment members 720. In alternative configurations, there can be a different number of recesses 805 (e.g. three) between a corresponding number of locking fingers 820 to receive a corresponding number of tips 722 of a corresponding number of alignment members 720.

The recess 805 assists with assembly and reduces the chance of the alignment members 720 being overly constrained.

As shown in Figure 17, the proximal portion 800a comprises an annular seal recess 843 for receipt of the seal 841.

The seal recess 843 is located between the locking fingers 820 and the distal portion 800b proximal to a base of the locking fingers. In some configurations, the seal recess is adjacent to the base of the locking fingers.

The seal recess 843 is defined between a first outwardly projecting flange 845 that is more proximal to the locking fingers 820 and a second outwardly projecting flange 847 that is more proximal to the distal portion 800b. The flanges 845, 847 may be substantially continuous annular members, or may be discontinuous. The flanges may comprise one or more axial recesses therethrough.

The seal 841 can be stretched over the first outwardly projecting flange 845 to install the seal.

In the configuration shown, a radial protrusion array 848 comprising a plurality of angularly spaced, outwardly projecting radial support protrusions 849 is provided proximal to the seal recess 843. The radial protrusion array 848 can assist with protecting the seal 841 from damage during connection of the complementary connector component 800 with the connector 700.

The radial protrusion array 848 may form, or may be positioned adjacent to, the first outwardly projecting flange 845.

The radial protrusion array 848 may be substantially continuous around the periphery of the body 801 or may comprise one or more discontinuities.

The radial support protrusions 849 of the radial protrusion array 848 may be tapered or chamfered, so that leading ends 849a of the radial support protrusions 849 have a smaller dimension than trailing ends 849b of the radial protrusions. The tapered configuration can assist with guiding the proximal portion 800a into engagement in the bore 703 of the connector, particularly if the proximal portion 800a is misaligned upon initial insertion into the bore 703.

The radial support protrusions 849 of the radial protrusion array 848 can be a close fit or in contact with the inner wall 702 of the body 701 of the connector when the complementary connector component 800 and connector 700 are connected, to limit rocking of those components relative to each other in use.

In the configuration shown in Figures 4 and 12, the complementary connector component 800 comprises a plurality of reinforcing ribs 871, 873.

The reinforcing ribs 871, 873 may extend in a longitudinal or axial direction of the complementary connector component 800, along internal surface(s) of the complementary connector component.

There may be any number of reinforcing ribs, but for example there may be 1-10 ribs, or 2-8 ribs, or 4-6 ribs, or 2 ribs, or 3 ribs, or 4 ribs, or 6 ribs, or 8 ribs, or 10 ribs.

At least some of the reinforcing ribs, and in particular reinforcing ribs 873 may be provided in the locking finger(s) 820 to reinforce the locking fingers 820 while still allowing deflection of the locking fingers 820.

The reinforcing ribs 873 in the locking finger(s) 820 may extend along at least a major part of a length of the locking finger(s). The reinforcing ribs 820 may terminate short of the tips 822 of the locking finger(s).

The reinforcing ribs 873 may extend along at least 2/3 of the length of the locking finger(s) 820, optionally along at least 3/4 of the length of the locking finger(s), optionally along at least 4/5 of the length of the locking finger(s) for example.

At least some of the reinforcing ribs 871, 873 may be tapered so that ends of the reinforcing ribs located more proximal to the terminal end 804 of the complementary connector component 800 have a smaller radial dimension between a radially inner edge and radially outer edge of the respective reinforcing ribs than portions of the reinforcing ribs located more distal from the terminal end 804

In the configuration shown in Figures 11, 16, 17, and 18, the complementary connector component 800 comprises one or more anti-rocking features 851. The anti-rocking features 851 inhibit rocking of the complementary connector component 800 relative to the connector 700 when they are connected together. For example, they inhibit small relative rotational displacements around the X or Y axis shown in Figure 3. They also prevent or reduce movement of the central axes of the complementary connector component 800 and connector 700 relative to each other.

The one or more anti-rocking features 851 may comprise a plurality of angularly spaced radial support protrusions 851 that are between the sealing recess 843 and the distal portion 800b. There may, for example, be 2, 3, 4, 5, 6, or more angularly spaced radial support protrusions 851.

The plurality of angularly spaced radial support protrusions 851 may form, or may be positioned adjacent to, the second outwardly projecting flange 847.

The anti-rocking feature(s) 851 can be a close fit or in contact with the inner wall 702 of the body 701 of the connector when the complementary connector component 800 and connector 700 are connected, to limit rocking of those components relative to each other in use.

The anti-rocking feature(s) 851 can be positioned at or adjacent the terminal end 704 of the bore 703 when the complementary connector component 800 and the connector 700 are connected to each other.

The body 701 of the connector 700 comprises one or more external alignment feature(s) 761 that is/are configured to interact with complementary external alignment feature(s) 861 on the complementary connector component 800.

The external alignment feature(s) 761 comprise one or more recesses and the complementary external alignment feature(s) 861 may comprise one or more complementary recesses or protrusions. The protrusion(s) will be arranged to be received in the recess(es).

The protrusion(s) and recess(es) are axially oriented.

The external alignment feature(s) 761 comprise one or more axially oriented recesses located in the body 701 of the connector at the terminal end 704 of the bore 703. This corresponds to the inlet 505 of the filter 501.

The complementary external alignment feature(s) 861 comprise one or more axially oriented protrusions protruding from a portion of the body 801 in the region between the proximal portion 800a and the distal portion 800b.

The external alignment feature(s) 761 and complementary external alignment feature(s) 861 may have a tapered configuration in the axial direction to assist with guiding the feature(s) 761, 861 into contact with one another.

The external alignment feature(s) 761, 861 act to physically align the connector 700 and complementary connector component 800 as they are connected with each other. Additionally, they can provide an externally visible aid for alignment of the connector 700 and complementary connector component 800.

The connection assembly may comprise one, two, three, four, or more external alignment features 761 and complementary external alignment features 861.

The connector 700 and complementary connector component 800 described herein or at least their bodies may be formed of any suitable materials. Example materials include rigid polymeric materials that may be biocompatible. Example materials include Polycarbonate (PC), Polyethylene (PE), Acrylonitrile Butadiene Styrene (ABS) or polypropylene (PP).

In the configuration shown, a cuff 881 is provided at least substantially about the distal portion 800b of the complementary connector component 800, said cuff 881 extending substantially longitudinally down at least a part of a length of the body 801 and inspiratory conduit 401.

The cuff 881 may comprise a compliant material. The cuff 881 may, for example, comprise an elasticised or elastic-type material, such as silicone or a thermoplastic elastomer (TPE) type material.

A portion of the cuff 881 may comprise a latching feature 883 as shown in Figure 4 for example, to engage the cuff with another portion of the complementary connector component 800. In the configuration shown in Figure 4, the latching feature engages with an optional overmould 885 on the distal portion 800b of the complementary connector component 800.

The cuff 881 may comprise a shaped portion corresponding to the shape of the external alignment feature 861.

In the configuration shown in Figures 6(a), 13, and 17 for example, the complementary connector component 800 comprises a recess 891 for receipt of, or containing, a patient end electrical component 1892. The patient end electrical component 1892 may comprise a printed circuit board (PCB).

For example, the PCB may be provided upon a relatively planar plate and may be inserted within the body bore of the connector component 1800. The PCB may be used for various reasons, and may in some particular configurations have circuitry to facilitate: control, sensing (e.g. temperature, humidity, flow rate), heating (e.g. heater wires) or other electronic components for a breathing conduit to be used as a part of a breathing circuit.

The patient end electrical component 1892 may be permanently connected or removable or made integral with the connector component.

The recess 891 comprises two diametrically opposed radially extending slots that extend outwardly from the inner wall of the body 801. The slots are configured to hold the patient end electrical component 892 in position across the bore 808 and/or bore 803.

As shown in Figure 17, the complementary connector component 800 may comprise electrical and/or data connection(s) 893 to provide electrical and/or data connection to the patient end electrical component 892.

The patient end electrical component 892 can comprise any suitable type of sensor. For example, the patient end electrical component may comprise a transducer, thermistor, pressure sensor, temperature sensor, humidity sensor, or other sensor. The patient end electrical component 892 may be in communication with the controller 205, to monitor characteristics of the gases flow and/or operate the apparatus in a manner that provides suitable therapy. The gases flow characteristics can include gases' concentration, flow rate, pressure, temperature, humidity, or others. The controller 205 can receive output from the sensors to assist it in operating the breathing assistance apparatus in a manner that provides suitable therapy, such as to determine a suitable target temperature, flow rate, and/or pressure of the gases flow. Providing suitable therapy can include meeting a patient's inspiratory demand.

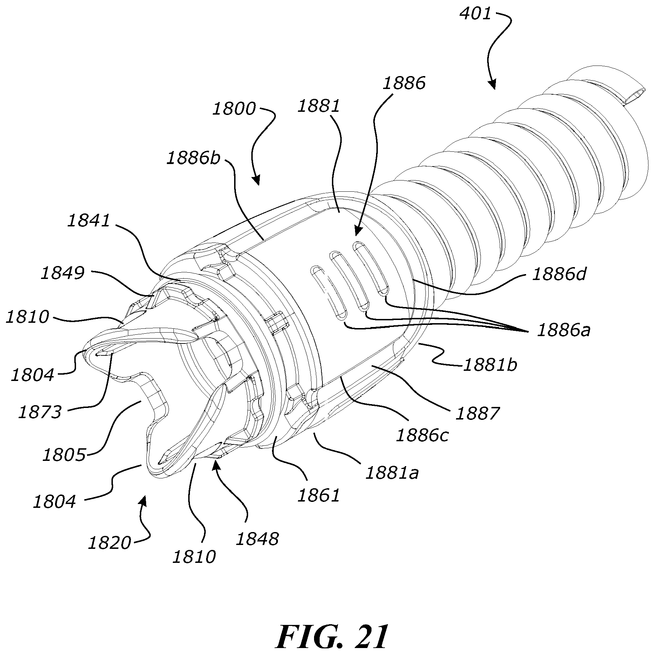

Figures 21 to 27 show a connector component 1800 that provides an alternative complementary connector component of the connection assembly. The connector component 1800 can be used in the connection assembly with the connector 700 in substantially the same manner as described above for the connector component 800. Unless described as otherwise below, the features, functionality, and options are the same as for the connector component 800. Like reference numerals indicate like parts with the addition of 1000.

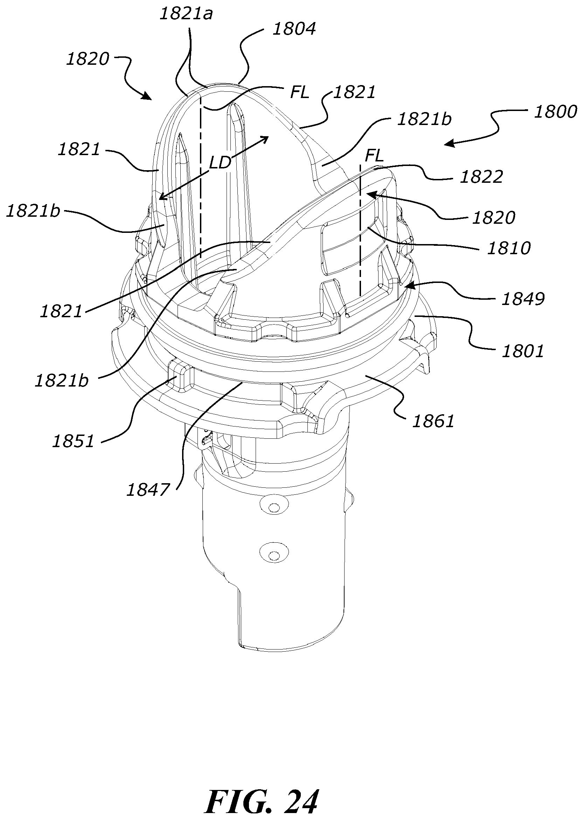

The connector component 1800 comprises a plurality of male engagement fingers 1820 which can also be referred to as locking fingers in the specification. Each of the male engagement fingers 1820 comprises a length FL (Figure 24) in an axial direction of the connector component 1800. A cuff 1881 with a grip surface 1886 is provided on an exterior of the connector component 1800.

The grip surface 1886 is at least partly offset around an external surface of the connector component from the male engagement fingers 1820, so as to be oriented at least partly in a lateral direction LD relative to the lengths FL of the male engagement fingers.

This allows a user to perform a "roll-out" disconnection between the connector 700 and the connector component 1800, where the user moves the connector component 1800 in a lateral direction (transverse to the male engagement fingers 1820) by applying a moment to the cuff 1881 and thereby to the connector component 1800, to disconnect the connector 700 and the complementary connector component 1800.

In the configuration shown, the grip surface 1886 is oriented in a direction LD that is substantially orthogonal to a plane (not shown) that intersects the lengths FL of the male engagement fingers 1820.

In the configuration shown, the grip surface 1886 is oriented in a direction LD that is substantially orthogonal to a plane (not shown) that intersects the lengths FL of the male engagement fingers 1820.

The grip surface 1886 is provided on an exterior of the cuff 1881.

The grip surface 1886 may comprise a surface texture to provide a grasping surface for a user. In the configuration shown, the grip surface 1886 comprises one or a plurality of protrusions 1886a that project(s) outwardly from a wall of the cuff 1881. Additionally, or alternatively, the grip surface 1886 may comprise one or a plurality of recesses that extend(s) inwardly into a wall of the cuff 1881. The protrusion(s) and/or recess(es) may partially extend in transverse direction across the periphery of the wall of the cuff 1881, to enable an axial force to be applied to the connector component 1800. For example, the protrusion(s) and/or recess(es) may comprise a spline or waveform or curved configuration that has a portion that extends in the transverse direction. In another configuration, the protrusion(s) and/or recess(es) may extend substantially in the transverse direction. In that configuration, the protrusion(s) and/or recess(es) may be substantially straight.

Additionally, or alternatively, the grip surface 1886 may comprises a rough or high friction surface finish.

The grip surface 1886 is at least partially framed by a surface feature 1887. In some configurations, the grip surface is 1886 is substantially framed by the surface feature 1887. In the configuration shown, at least two opposite sides 1886b, 1886c, and optionally at least three sides 1886b, 1886c, 1886d including the two opposite sides 1886b, 1886c, of the grip surface 1886 are framed by the surface feature 1887. Substantially the entire grip surface 1886 may be framed by the surface feature 1887.

In the configuration shown, the surface feature 1887 comprises a recess or channel in an outer wall of the cuff 1886. The recess or channel may be continuous or discontinuous. In an alternative configuration, the surface feature 1887 could comprise a protrusion (not shown) that extends outwardly, optionally radially outwardly, from the outer wall of the cuff. The protrusion may be continuous or discontinuous.

The surface feature 1887 at least partly frames the grip surface 1886 to indicate to a user where to grasp the connector component 1800. When the surface feature 1887 comprises a recess or channel, the recess or channel may act as a safety feature to inhibit the chance of misconnection of a larger connector over the connector and cuff 1886 of the connector component 1800, and may provide a leak pathway. The recess or channel acts as a safety mechanism to provide an intentional leak when an incorrect connection has been made. Such intentional leak could trigger leak alarms in some flow control devices, indicating to a user that the system has not been properly setup.

In the configuration shown, the connector component 1800 comprises two diametrically opposed male engagement fingers 1820, and the cuff 1881 comprises two diametrically opposed grip surfaces 1886. Each of the grip surfaces 1886 may have any one or more of the features described herein.

In the configuration shown, the grip surfaces 1886 are offset from the male engagement fingers 1820 around the periphery of the cuff 1881. Therefore, when viewed from the free end of the connector component 1800, the grip surfaces 1886 are aligned with the recesses 1805 between the bases of the male engagement fingers 1820.

In the configuration shown, a plane extending through longitudinal centres of the grip surfaces 1886 would be orthogonal to a plane extending through the lengths FL of the male engagement fingers, with the intersection of the two planes being coaxial with a longitudinal axis LA of the connector component 1800.

In an alternative configuration, the connector component 1800 could have a different number of male engagement fingers 1820 and a different number of grip surfaces 1886. For example, the connector component 1800 could have three male engagement fingers 1820 spaced around the connector component 1800, and may further comprise three grip surfaces 1886 that are offset from those male engagement fingers 1820 around the periphery of the cuff 1881.

The male engagement fingers 1820 are configured to be received by the female connector 700.

As discussed above for the connector component 800, the male engagement fingers 1820 may each comprise an engagement feature 1810 on an outer surface of the finger.

In the configuration shown, the engagement features 1810 each comprise a recess or aperture for receiving a respective retaining protrusion 710 that extends from an inner wall of the female connector 700.

As discussed above for the connector component 800, the male engagement fingers 1820 may each comprise engagement surfaces 1821 for engaging with complementary engagement surfaces 721 of the connector 700.

Each of the engagement surfaces 721, 821 comprises a twist.

A tip 1821a or a portion of the tip of each male engagement finger 1820 has a chamfered configuration.

The connector component 1800 comprises a seal 1841 adjacent a base of the male engagement fingers 1820. The seal 1821 may comprise a wiper seal, and optionally the seal 1821 may comprise a T-shaped wiper seal.

The seal 1841 is disposed in a recess 1843.

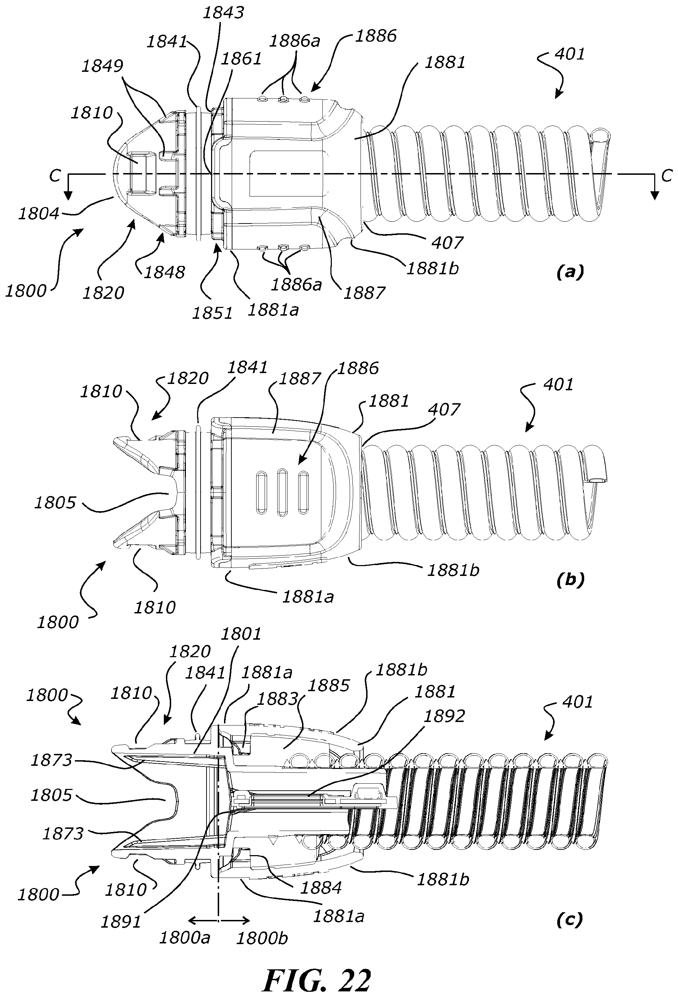

As shown in Figure 22(c) for example, the cuff 1881 has a tapered configuration in which a portion 1881a of the cuff proximal to the male engagement fingers 1820 has a larger lateral dimension than a portion 1881b of the cuff more distal to the male engagement fingers 1820.

Comparing Figure 22(a) to Figures 22(b) and (c), the edges of the cuff having the grip surfaces 1886 may be substantially parallel or slightly tapered, and the edges of the cuff through the non-grip section (i.e. normal to the grip sections) may be more tapered.

The grip surface 1886 is substantially aligned with the portion of the cuff proximal to the male engagement fingers, in an axial direction of the connector component. That is, although the surfaces of the cuff having the grip surfaces 1886 may be substantially parallel or only slightly tapered, they grip surfaces 1886 are aligned with the wider portion of the cuff.

Because the grip surface 1886 is aligned or positioned on a wider portion of the tapered cuff 1881, alongside the inclusion of a surface texture, this evokes in the mind of the user where to grasp the assembly to engage/disengage the connector component 1800 with/from the connector 700.

The connector component 1800 comprises at least one external alignment feature 1861.

The external alignment feature 1861 may comprises a recess or protrusion to engage with a complementary protrusion or recess 761 on the connector.

The connector component comprises reinforcing ribs 1873 along internal surfaces of the male engagement fingers 1820.

In the configuration shown, the connector component 1800 has a reduced number of ribs 1873 compared to the ribs 871, 873 of the connector component 800. Additionally, rather than being oriented radially inwardly from an inner wall of the connector component 1800, the ribs 1873 are in opposed relationship where a rib 1873 on one side of the bore of the connector component is oriented toward a rib 1873 on the other side of the bore in a substantially planar arrangement.

The connector component 1800 comprises a patient end electrical component 1892 disposed in a gas flow lumen. The patient end electrical component 1892 may comprise a printed circuit board (PCB).

For example, the PCB may be provided upon a relatively planar plate and may be inserted within the body bore of the connector component 1800. The PCB may be used for various reasons, and may in some particular configurations have circuitry to facilitate: control, sensing (e.g. temperature, humidity, flow rate), heating (e.g. heater wires) or other electronic components for a breathing conduit to be used as a part of a breathing circuit.

The patient end electrical component 1892 may be permanently connected or removable or made integral with the connector component.

The recess 1891 comprises two diametrically opposed radially extending slots that extend outwardly from the inner wall of the body 1801. The slots are configured to hold the patient end electrical component 1892 in position across the bore of the connector component 1800.

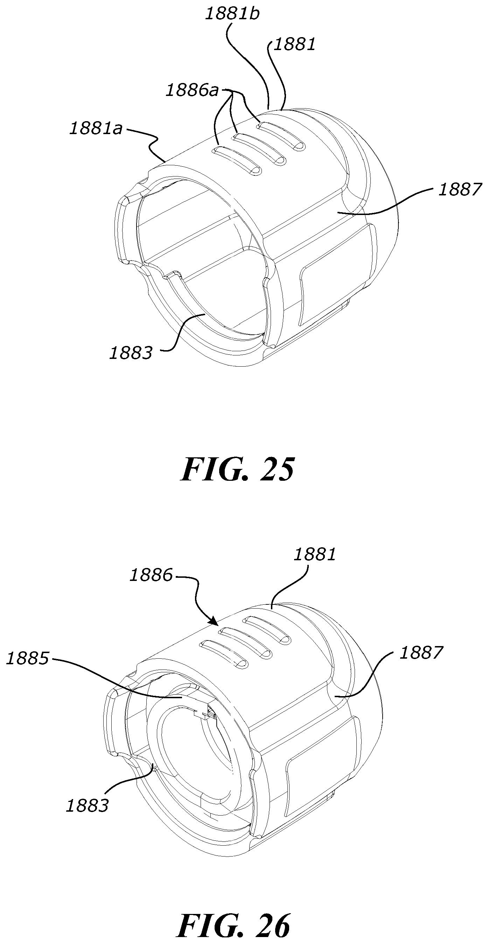

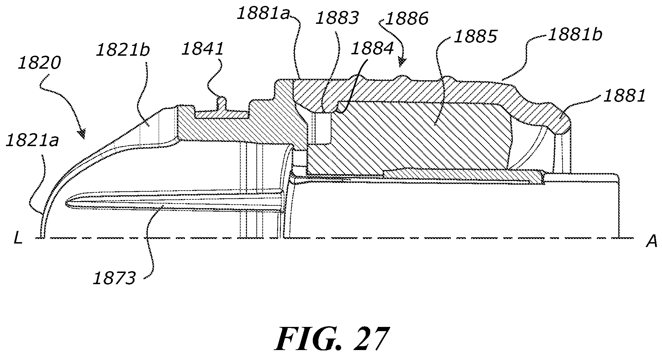

With reference to Figures 25 and 27, an inner surface of the cuff 1881 comprises latching feature(s) to engage the cuff with another portion of the connector component 1800.

In the configuration shown, the latching features comprise opposing protrusions 1883 on an inner surface of the cuff 1881 to engage with opposing recesses 1884 on a body of the connector component. The opposing protrusions 1883 are aligned with the grip surface(s) 1886. That is, when viewed from the free end of the connector component 1800, the protrusions 1883 are radially inward (preferably directly) of the grip surfaces 1886. When force towards central axis LA is applied to the grip surface(s) 1886 of the cuff, the force may be partially or completely transferred through the protrusions 1883 and recesses 1884 to the body 1801. This inward force (towards central axis LA) strengthens the user's grip on the connector component 1800 for example for disengaging the connector component 1800 from the connector 700.

The connector component 1800 may comprise an overmould 1885 on the body 1801, and the opposing recesses 1884 may be provided in the overmould 1885. Alternatively, the opposing recesses 1884 may be provided directly on the body 1801.

The cuff 1881 may be formed from any suitable material such as a compliant material. The cuff 1881 may, for example, comprise an elasticised or elastic-type material, such as silicone or a thermoplastic elastomer (TPE) type material. In some configurations, the cuff 1881 comprises a thermoplastic elastomer material. In some configurations, the thermoplastic elastomer comprises thermoplastic vulcanizate for example.

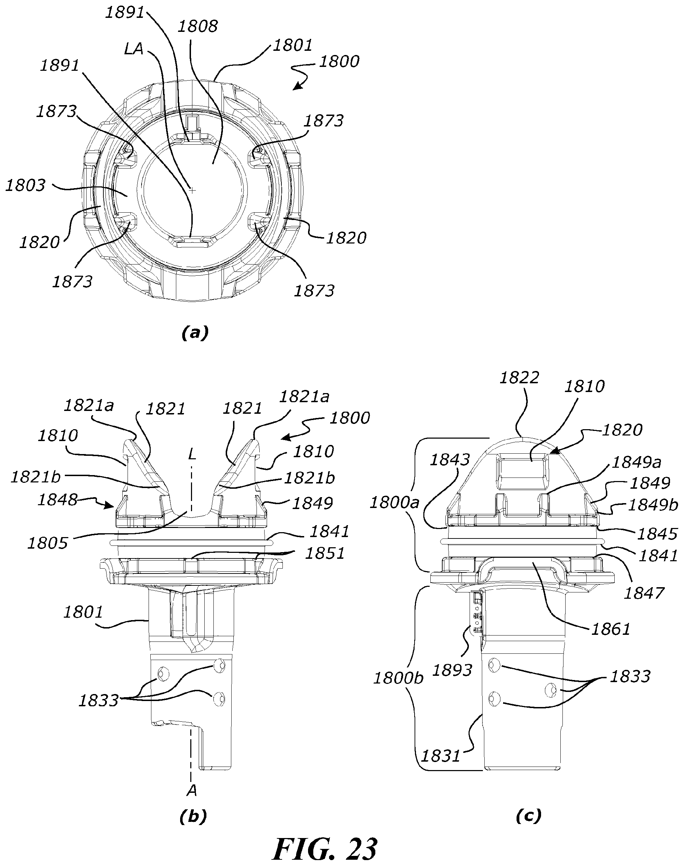

Referring to Figure 23(b) for example, the base of the recess 1805 is more concave than that of the recess 805 which is substantially planar.

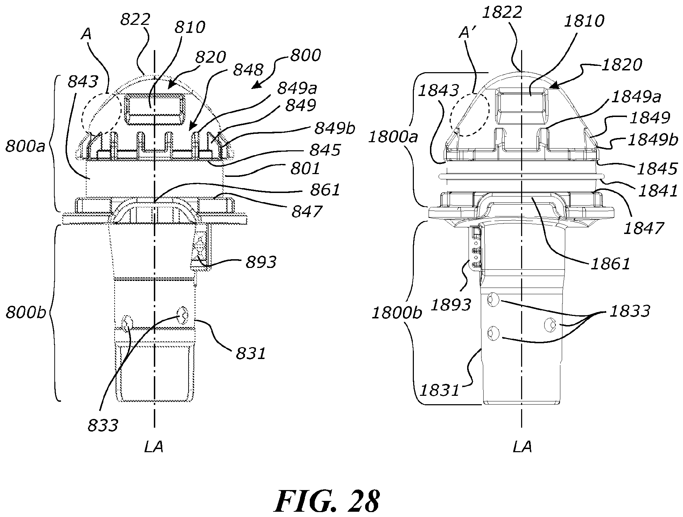

Referring to Figure 28, the engagement surfaces 1821 of the male engagement fingers 1820 have a different shape to the engagement surfaces 821 of the locking fingers 820. In particular, the engagement surfaces 1821 have a longer (in the direction of the longitudinal axis LA) and narrower (in a direction transverse to the longitudinal axis LA) overall configuration than the engagement surfaces 821. The bases of the engagement surfaces 821 (in circled region A and on the opposite side) have a laterally wider and more outwardly bowed configuration. The bases of the engagement surfaces 1821 (in circled region A' and on the opposite side) have a laterally narrower and more inwardly bowed configuration. That is, the bases of the engagement surfaces 821 are laterally convex when viewed from either side of the fingers 820. The bases of the engagement surfaces 1821 are laterally concave when viewed from either side of the fingers 1820. Overall, the sides and tip of the engagement surfaces 1821 are a more sinuous shape than the sides and tip of the engagement surfaces 821.

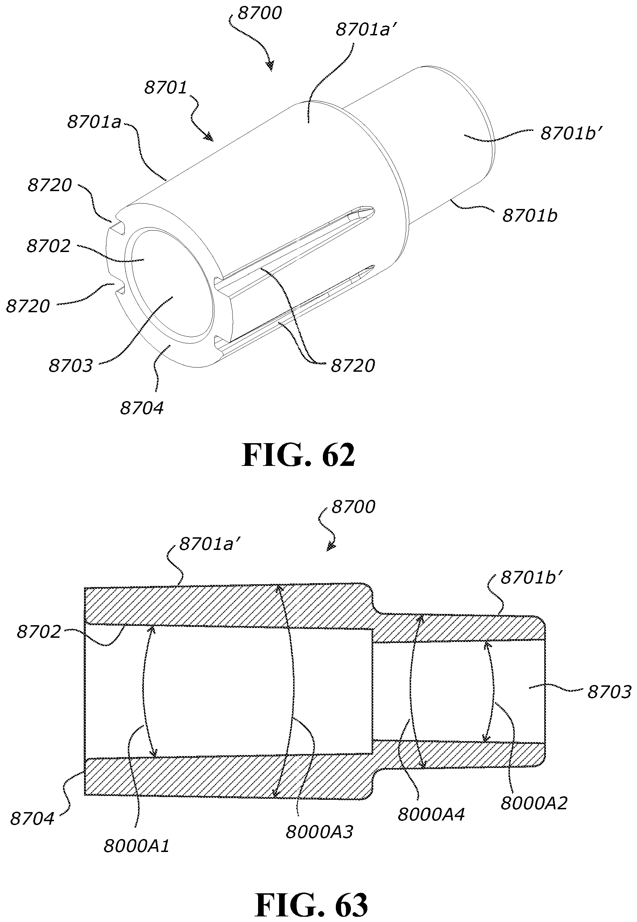

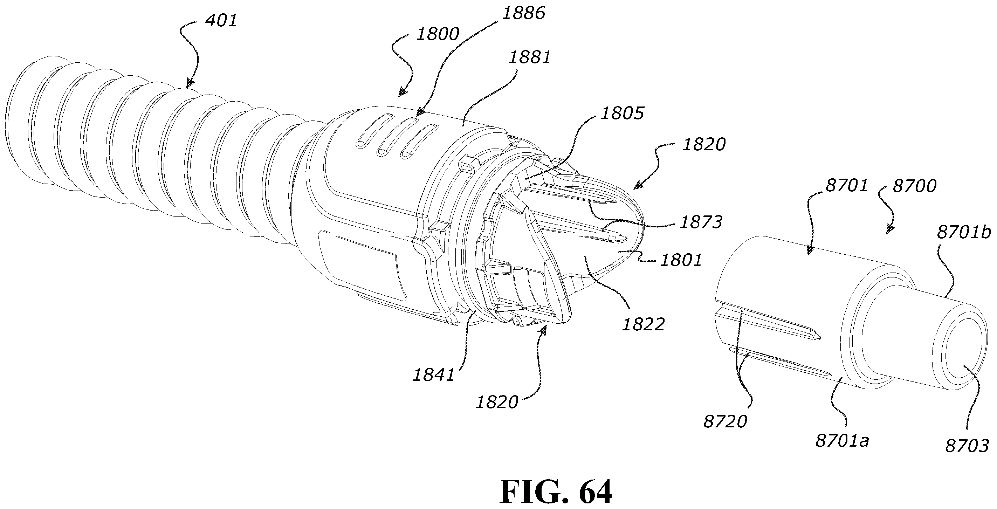

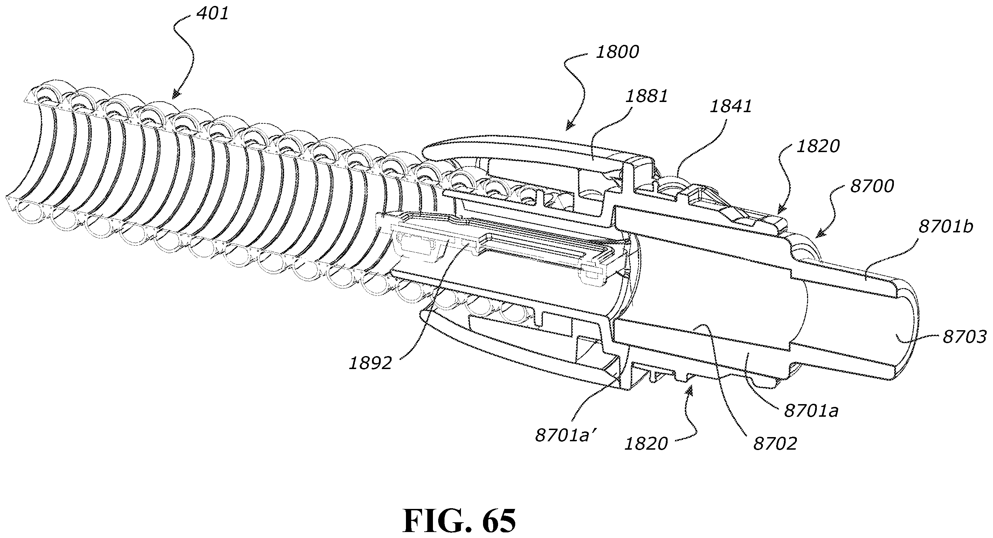

Figures 62 to 65, 66 to 68, 69 to 72, and 73 to 75 show alternative connectors 8700, 9700, 10700, 11700 for use in a respiratory system. The connectors 8700, 9700, 10700, 11700 are for connecting a first respiratory system component to a second respiratory system component. For example, the connectors 8700, 9700, 10700, 11700 could be used for connecting the filter 501, 1501 to the inspiratory conduit 401. In alternative configurations or applications, the first respiratory system component and second respiratory system component could comprise different components such as conduit(s), breathing tube(s), patient interface(s), non-invasive ventilation mask(s), T-piece(s) or Y-piece(s) for accessories such as nebulisers or other accessories, nebuliser(s), heat and moisture exchanger(s) (HMEs), cannula(s), or any two suitable components within a respiratory circuit. The connectors 8700, 9700, 10700, 11700 could be used as, or in, an adapter.

The connectors 8700, 9700, 10700, 11700 may be provided at the filter inlet 505, 1505 of the filter 501, 1501. The connectors 8700, 9700, 10700, 11700 may be attached to or integrally formed with the filter housing 504, 1504.

In the configuration shown, the connectors 8700, 9700, 10700, 11700 are configured for use in a connection assembly with the complementary connector component 1800. In an alternative configuration, the connectors 8700, 9700, 10700, 11700 may be configured for use with the complementary connector component 800 or any other suitable complementary connector component.

Figures 62 to 65 show a first alternative connector 8700.

The connector 8700 comprises a body 8701 comprising an engagement portion 8701a that is configured for receipt in a bore 1822 of a complementary connector component 1800. At least an outer surface 8701a' of the engagement portion 8701a comprises a compliant material. The compliant material is configured to at least generally conform to a wall 1801 of the bore 1822 of the complementary connector component 1800 when the engagement portion 8701a is received in the bore 1822 of the complementary connector component 1800.

The compliant material may be configured to substantially conform to the wall 1801 when the engagement portion 8701a is received in the bore 1822 of the complementary connector component.

The compliant material may, for example, comprise an elasticised or elastic-type material, such as silicone or a thermoplastic elastomer (TPE) type material. In some configurations, the compliant material comprises a thermoplastic elastomer material. In some configurations, the thermoplastic elastomer comprises thermoplastic vulcanizate for example.

In some configurations, the entire connector 8700 comprises the compliant material. In alternative configurations, at least part of the connector 8700 other than the engagement portion 8701a comprises a less compliant material.

In some configurations, the less compliant material comprises a rigid polymeric material that may be biocompatible. Example materials include Polycarbonate (PC), Polyethylene (PE), Acrylonitrile Butadiene Styrene (ABS) or polypropylene (PP).

The body 8701 comprises at least one alignment feature 8720.

In the configuration shown, the at least one alignment feature 8720 is configured to cooperate with one or more complementary alignment features 1873 of the complementary connector component 1800.

The at least one alignment feature 8720 is an external alignment feature that is provided on or in the outer surface 8720 of the engagement portion 8701a of the connector component 8700.

The at least one alignment feature 8720 is configured to cooperate with one or more complementary internal alignment features 1873 in the bore 1822 of the complementary connector component 1800.

The at least one alignment feature 8720 may be a female alignment feature or may be a male alignment feature. The at least one complementary alignment feature 1873 may be a male alignment feature or may be a female alignment feature. Each of the connector 8700 and the complementary connector component 1800 may comprise a combination of male and female alignment features/complementary alignment features.

In the configuration shown, the complementary alignment features 1873 comprise the reinforcing ribs along internal surfaces of the male engagement fingers 1820. In the configuration shown, the complementary alignment features 1873 are reinforcing ribs that extend in an axial direction of the complementary connector component 1800. Alternatively, the complementary alignment features 1873 may comprise different members.

In some configurations, the connector 8700 comprises a plurality of the alignment features 8720. In some configurations, the complementary connector component 1800 comprises a corresponding plurality of the complementary alignment features 1873.

The plurality of the alignment features 8720 are angularly spaced around a periphery of the engagement portion 8701a. The alignment features 8720 may be substantially evenly spaced around the periphery or may be unevenly spaced.

The connector 8700 may comprise two, three, four, or more of the alignment features 8720. Alternatively, the connector 8700 may comprise a single alignment feature 8720.

The body comprises a terminal end 8704 that is configured to be received in the bore 1822 of the complementary connector component 1800.

The alignment feature(s) 8720 extend from the terminal end 8704 in a direction away from the terminal end 8704.

The alignment feature(s) 8720 extend in an axial direction of the connector 8700.

In the configuration shown, the complementary connector component 1800 comprises engagement fingers 1820, and the bore 1822 of the complementary connector component 1800 is between the engagement fingers 1820. In alternative configurations, the bore 1822 could be provided elsewhere in the complementary connector component 1800.

The body 8701 of the connector defines a bore 8703 that defines a gases lumen.

The bore 8703 of the connector extends from the terminal end 8704 of the body 8701 in a direction away from the terminal end 8704.

As shown in Figure 63, the bore 8703 of the connector comprises a taper 8000A1 between opposed surfaces of the bore such that a portion of the bore 8703 proximal to the terminal end 8704 has a larger transverse dimension than a portion of the bore 8703 more distal from the terminal end 8704.

In some configurations, the taper 8000A1 is between about 0 degrees and about 15 degrees. In some configurations, the taper 8000A1 is more than 0 degrees and up to about 15 degrees.

The engagement portion 8701a is configured for receipt in the bore 1822 of the complementary connector component 1800 with a frictional engagement. Once the engagement portion 8701a is received in the bore 1822, it is retained therein in use by a frictional engagement between the engagement portion 8701a and the wall 1801 of the bore 1822.

The outer surface 8701a' of the engagement portion 8701a may be configured to form a seal with a wall 1801 of the bore 1822 of the complementary connector component.

The outer surface 8701a' of the engagement portion 8701 may comprise a taper such that such that a portion of the outer surface 8701a' proximal to the terminal end 8704 has a smaller transverse dimension than a portion of the outer surface 8701a' more distal from the terminal end 8704.

A distal portion 8701b of the body 8701 that is distal from the terminal end 8704 may be configured to be engaged with, for example received in, or may be in, the first respiratory system component. For example, the distal portion 8701b of the body may be configured to be engaged with, for example to be received in, or may be in, the filter inlet 505, 1505 of the filter 501, 1501.

Similarly to the engagement portion 8701a, an outer surface 8701a' of the distal portion 8701b may comprise a compliant material. The compliant material is configured to at least generally conform to a wall of the first respiratory system component. The complaint material may, for example, be of the type described above for the outer surface 8701a'.

The compliant material may be configured to substantially conform to a the wall of the first respiratory system component when the distal portion 8701b is engaged with the first respiratory system component.

In the configuration shown, the outer surface 8701a' has a larger transverse outer dimension than the outer surface 8701b'. Alternatively, the outer surface 8701a' may have a smaller transverse outer dimension than the outer surface 8701b', or they may have the same dimensions.

The bore 8703 extends through the engagement portion 8701a of the body and through the distal portion 8701b of the body.

In the configuration shown, the portion of the bore 8703 in the distal portion 8701b comprises a taper 8000A2 between opposed surfaces of the bore that is opposed to the taper 8000A1 in the engagement portion. That is, within the distal portion 8701b, the portion of the bore 8703 adjacent the engagement portion 8701a has a smaller transverse dimension than a portion of the bore 8703 more distal from the engagement portion 8701a.

In an alternative configuration, the bore 8703 may have the same dimension throughout from the terminal end 8704 to the opposite end of the bore 8703. In another alternative configuration, the taper 8000A1 or 8000A2 may continue along the entire length of the bore 8703.

In some configurations, the taper 8000A2 is between about 0 degrees and about 15 degrees. In some configurations, the taper 8000A2 is more than 0 degrees and up to about 15 degrees.

The engagement portion 8701a and distal portion 8701b may comprise external tapers 8000A3, 8000A4. The tapers may, for example, be standard medical tapers.

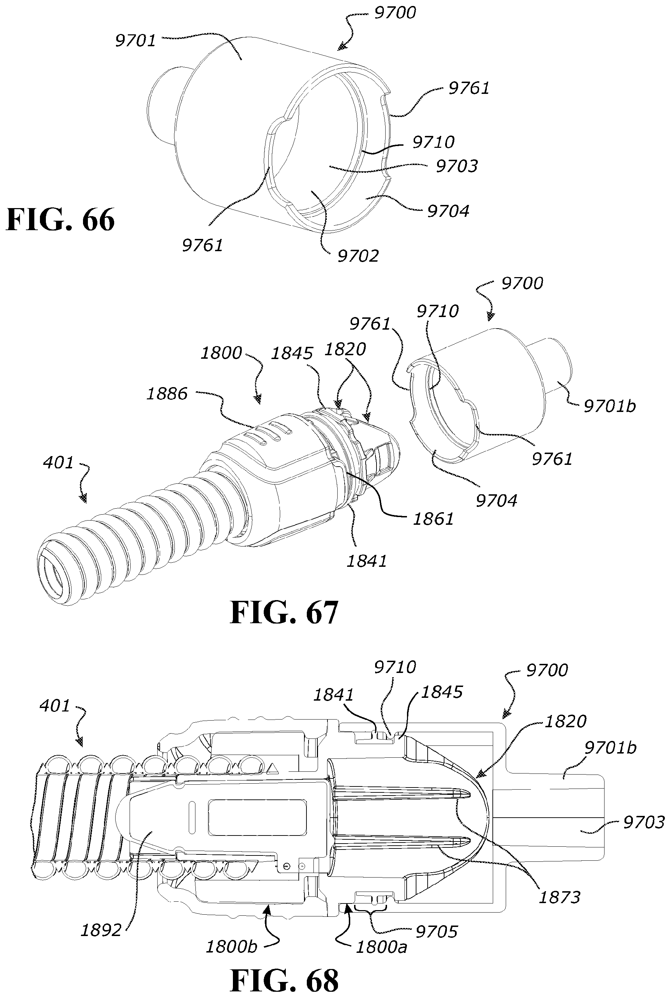

Figures 66 to 68 show a second alternative connector 9700.

The connector 9700 comprises a body 9701 defining a bore 9703 for receipt of a complementary connector component 1800. The bore 9703 has a terminal end 9704 that provides an entry into the bore 9703 for the complementary connector component 1800.

The bore 9703 defines a gases lumen.

The connector comprises at least one internal retaining feature 9710 and at least one external alignment feature 9761.

The at least one external alignment feature 9761 is/are configured to interact with complementary external alignment features(s) 1861 on the complementary connector component 1800.

The at least one external alignment feature 9761 comprises one or more protrusions or recesses that is/are configured to interact with one or more complementary recesses or protrusions on the complementary connector component 1800.

The one or more protrusions or recesses of the connector 9700 may be axially oriented.

The protrusion(s) will be arranged to be received in the recess(es).

In the configuration shown, the external alignment feature(s) 9761 comprise one or more axially oriented recesses located in the body 9701 of the connector 9700 at the terminal end 9704 of the bore 9703.

The complementary external alignment feature(s) 1861 comprise one or more axially oriented protrusions protruding from a portion of the body 1801 in the region between the proximal portion 1800a and the distal portion 1800b.

The external alignment feature(s) 9761 and complementary external alignment feature(s) 1861 may have a tapered configuration in the axial direction to assist with guiding the feature(s) 9761, 1861 into contact with one another.

The external alignment feature(s) 9761 and complementary external alignment feature(s) 1861 may have an axial dimension that is less than a circumferential dimension. In some configurations, the axial dimension is less than half of the circumferential dimension.

The external alignment feature(s) 9761, 1861 act to physically align the connector 9700 and complementary connector component 1800 as they are connected with each other. Additionally, they can provide an externally visible aid for alignment of the connector 9700 and complementary connector component 1800.

The at least one external alignment feature 9761 is/are at or adjacent the terminal end 9704 of the bore 9703.

The connection assembly may comprise one, two, three, four, or more external alignment features 9761 and complementary external alignment features 1861.

The at least one internal retaining feature 9710 comprises at least one retaining protrusion that extends into the bore 9703 from an inner wall 9702 of the body 9701.

In the configuration shown, the at least one internal retaining feature 9710 comprises a single retaining protrusion.

The single retaining protrusion 9710 has an annular configuration.

In an alternative configuration, the single retaining protrusion may extend around only part of the bore 9703.

In alternative configurations, the at least one internal retaining feature 9710 may comprise a plurality of retaining protrusions that are angularly spaced around the bore. The plurality of retaining protrusions may approximate an annular configuration, or may comprise more substantial angular spaces between the retaining protrusions.

The at least one internal retaining feature 9710 is configured to engage with a least one engagement feature on the complementary connector component 1800.

In the configuration shown, the at least one engagement feature comprises or is provided by the outwardly projecting flange 1845 of the complementary connector component 1800.

In alternative configurations, the at least one engagement feature could be provided by one or more different members on the complementary connector component 1800.

The connector comprises a sealing region 9705 (Figure 68) for engaging with the seal 1841 on the complementary connector component 1800.

The sealing region 9705 is closer to the terminal end 9704 of the bore 9703 relative to the internal retaining feature(s) 9710.

The connector 9700 may comprise a compliant material. The compliant material may be of the type described above for the compliant material of the connector 8700.

In the configuration shown, the body 9701 of the connector 9700 comprises a distal portion 9701b. The distal portion 9701b may have any one or more of the features described above for the distal portion 8701b of the connector 8700.

In the configuration shown, the complementary connector component 1800 comprises engagement fingers 1820. The engagement fingers 1822 of the complementary connector component 1800 are received in the bore 9703 when the connection assembly is assembled.

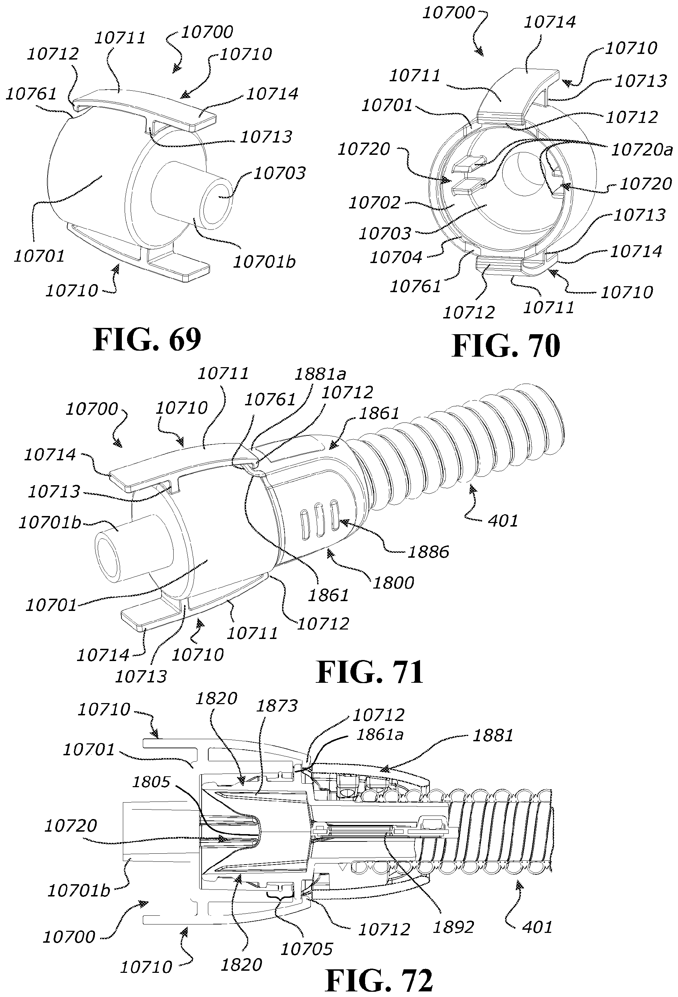

Figures 69 to 72 show a third alternative connector 10700.

The connector 10700 comprises a body 10701 defining a bore 10703 for receipt of a complementary connector component 1800. The bore 10703 has a terminal end 10704 that provides an entry into the bore 10703 for the complementary connector component 1800.

The bore 10703 defines a gases lumen.

The connector 10700 comprises at least one alignment feature 10720, 10761.

The connector 10700 comprises at least one releasable latch member 10710 for engagement with the complementary connector component 1800.

The at least one alignment feature 10720, 10761 is/are configured to align the at least one releasable latch member 10710 with at least one complementary engagement feature 1861 on the complementary connector component 1800.

The at least one alignment feature comprises at least one internal alignment feature 10720 and/or at least one external alignment feature 10761.

The at least one internal alignment feature 10720 comprises at least one alignment member 10720a that extends into the bore 10703 from an inner wall 10702 of the body 10701.

The at least one alignment member 10720a is configured to be received in a recess 1805 in the complementary connector component 1800.

In the configuration shown, the recess 1805 is located between engagement fingers 1820 of the complementary connector component 1800. Alternatively, the recess 1805 may be provided elsewhere in the complementary connector component 1800.

The connector 10700 may comprise a plurality of angularly spaced internal alignment features 10720, and the complementary connector component 1800 may comprise a corresponding plurality of angularly spaced recesses 1805. In an alternative configuration, the connector 10700 may comprise a single internal alignment feature 10720, and the complementary connector component 1800 may comprise s single recess 1805. In an alternative configuration, there may be no internal alignment features, and instead the connector 10700 and complementary connector component may comprise only external alignment feature(s).

The internal alignment features 10720 may be substantially evenly spaced around the periphery or may be unevenly spaced.

The at least one external alignment feature 10761 is/are configured to interact with at least one complementary external alignment features 1861 on the complementary connector component 1800.

The at least one external alignment feature 10761 comprises one or more protrusions or recesses that is/are configured to interact with one or more complementary recesses or protrusions on the complementary connector component 1800.

The one or more protrusions or recesses of the connector may be axially oriented.

The protrusion(s) will be arranged to be received in the recess(es).

In the configuration shown, the external alignment feature(s) 10761 comprise one or more axially oriented recesses located in the body 10701 of the connector 10700 at the terminal end 10704 of the bore 9703.

The complementary external alignment feature(s) 1861 comprise one or more axially oriented protrusions protruding from a portion of the body 1801 in the region between the proximal portion 1800a and the distal portion 1800b.

The external alignment feature(s) 10761 and complementary external alignment feature(s) 1861 may have a tapered configuration in the axial direction to assist with guiding the feature(s) 10761, 1861 into contact with one another.

The external alignment feature(s) 10761, 1861 act to physically align the connector 10700 and complementary connector component 1800 as they are connected with each other. Additionally, they can provide an externally visible aid for alignment of the connector 10700 and complementary connector component 1800.

The connection assembly may comprise one, two, three, four, or more external alignment features 9761 and complementary external alignment features 1861.

In some configurations, the at least one external alignment feature 10761 is/are at or adjacent the terminal end 10704 of the bore 10703.

The at least one releasable latch member 10710 is configured to engage with at least one complementary engagement feature on the complementary connector component 1800.

When the at least one releasable latch member 10710 is engaged with the at least one complementary engagement feature on the complementary connector component 1800, the connector 10700 is retained in connection with the complementary connector component 1800. The at least one releasable latch member 10710 is releasable to enable disconnection of the connector 10700 from the complementary connector component 1800.

In the configuration shown, the at least one engagement feature is on a complementary external alignment feature 1861 on the complementary connector component 1800.

More specifically, the at least one complementary engagement feature comprises a wall 1861a of the complementary external alignment feature 1861. The wall may be located at a terminal end of the complementary external alignment feature 1861.

The cuff 1881 may comprise a compliant material as described above, to enable the cuff 1881 to flex out of the way of the releasable latch member(s) 10710 when engaged with the complementary connector component 1800. Alternatively, the cuff 1881 may not be present or may comprise cut-out(s) to provide recesses for receipt of latching portions of the releasable latch members 10710.

In some configurations, the at least one releasable latch member 10710 comprises a latch body 10711 with a latching portion 10712 for engaging with the complementary connector component 1800.

The latching portion 10712 extends radially inwardly from the latch body 10711.

The latch body 10711 of the at least one releasable latch member 10710 is selectively movable from a latching configuration in which the latching portion 10712 is engaged with the complementary connector component 1800 to an unlatched configuration in which the latching portion 10712 is disengaged from the complementary connector component 1800.

The releasable latch members are shown in the engaged configuration in Figures 69-72. In the unlatched configuration, the latching portions 10712 will be radially outward from the positions shown.

The at least one releasable latch member 10710 is in the latching configuration in an at-rest configuration of the at least one releasable latch member 10710.

The at least one releasable latch member 10710 is biased to the latching configuration. The at least one releasable latch member 10710 may be so biased by a biasing device and/or by resilience of material of the at least one releasable latch member 10710.

A connector portion 10713 may connect the latch body 10711 to the body 10701 of the connector 10700. The connector portion 10713 may be configured to enable selective movement of the latch body 10711 relative to the body 10701 of the connector 10700.

In one exemplary configuration, the connector portion 10713 may comprise a pivot. A biasing device, such as a torsion spring, tension spring, compression spring, leaf spring, or resilient member for example, may be associated with the pivot. The biasing device will be configured to bias the at least one releasable latch member 10710 to the latching configuration.

In another exemplary configuration, the connector portion 10713 may comprise a resilient member that can be flexed to enable selective movement of the latch body 10711 relative to the body 10701 of the connector 10700.

In some configurations, the at least one releasable latch member 10710 may comprise a rigid polymeric material that may be biocompatible. Example materials include Polycarbonate (PC), Polyethylene (PE), Acrylonitrile Butadiene Styrene (ABS) or polypropylene (PP). In some configurations, the entire connector 10700 may comprise such a material.

In some configurations, the body 10701 may comprise a compliant material. The compliant material may, for example, comprise an elasticised or elastic-type material, such as silicone or a thermoplastic elastomer (TPE) type material. In some configurations, the compliant material comprises a thermoplastic elastomer material. In some configurations, the thermoplastic elastomer comprises thermoplastic vulcanizate for example.

The at least one releasable latch member 10710 comprises an actuating surface 10714 to enable a user to selectively move the latch body 10711 to the unlatched configuration.

The actuating surface 10714 of the at least one releasable latch member 10710 is at an opposite end of the latch body 10711 to the latching portion 10712.

The actuating surface 10714 can be pushed radially inwardly (towards the centre of the connector 10700) to move the latching portion 10712 radially outwardly.

In some configurations, the at least one releasable latch member 10710 is releasable by applying a significantly high force to pull the connector 10700 and the complementary connector component 1800 apart in an axial direction. In other configurations, the at least one releasable latch member 10710 is not releasable by applying high force to the connector 10700 and complementary connector component 1800 in the axial direction, in the absence of selectively moving the at least one releasable latch member 10710 to the unlatched configuration.

The connector 10700 may comprise one, two, three, or more of the releasable latch members 10710.

In the configuration shown, the body 10701 of the connector 10700 comprises a distal portion 10701b. The distal portion 10701b may have any one or more of the features described above for the distal portion 8701b of the connector 8700.

The connector comprises a sealing region 10705 (Figure 72) for engaging with the seal 1841 on the complementary connector component 1800.

The sealing region is provided by the inner wall 10702 of the body 10701 of the connector 10700.

The sealing region 10705 is at or adjacent to the terminal end 10704 of the bore 10703.

In the configuration shown, the complementary connector component 1800 comprises engagement fingers 1820. The engagement fingers 1822 of the complementary connector component 1800 are received in the bore 10703 when the connection assembly is assembled.

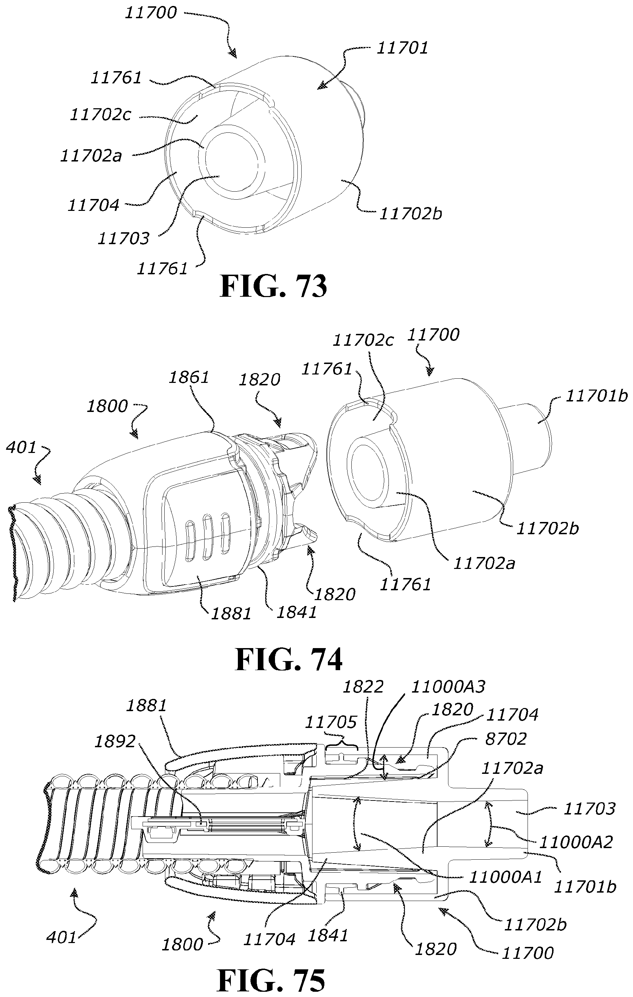

Figures 73 to 75 show a fourth alternative connector 11700.

The connector 11700 comprises a body 11701 comprising a radially inner wall 11702a, a radially outer wall 11702b, and a cavity 11702c between the radially inner wall 11702a and the radially outer wall 11702b for receipt of the complementary connector component 1800. In the configuration shown, the cavity receives the engagement fingers 1820 of the complementary connector component 1800. The cavity 11703c has a terminal end 11704 that provides an entry into the cavity 11703c for the complementary connector component 1800. The connector comprises at least one external alignment feature 11761.

The at least one external alignment feature 11761 is/are configured to interact with complementary external alignment features(s) 1861 on the complementary connector component 1800.

The at least one external alignment feature 11761 comprises one or more protrusions or recesses that is/are configured to interact with one or more complementary recesses or protrusions on the complementary connector component.

The one or more protrusions or recesses of the connector may be axially oriented.

The protrusion(s) will be arranged to be received in the recess(es).

In the configuration shown, the external alignment feature(s) 11761 comprise one or more axially oriented recesses located in the body 11701 of the connector 11700 at the terminal end 11704 of the cavity 11702c.

The complementary external alignment feature(s) 1861 comprise one or more axially oriented protrusions protruding from a portion of the body 1801 in the region between the proximal portion 1800a and the distal portion 1800b.

The external alignment feature(s) 11761 and complementary external alignment feature(s) 1861 may have a tapered configuration in the axial direction to assist with guiding the feature(s) 11761, 1861 into contact with one another.

The external alignment feature(s) 11761, 1861 act to physically align the connector 11700 and complementary connector component 1800 as they are connected with each other. Additionally, they can provide an externally visible aid for alignment of the connector 11700 and complementary connector component 1800.

The connection assembly may comprise one, two, three, four, or more external alignment features 11761 and complementary external alignment features 1861.

The at least one external alignment feature 11761 is/are at or adjacent the terminal end 11704 of the cavity 11702c.

The body 11701 may comprise at least one additional alignment feature (not shown in Figures 73 to 75). For example, the body 11701 may comprise alignment features(s) 8720 as discussed above for the connector 8700.

The at least one additional alignment feature 8720 may be configured to cooperate with one or more complementary additional alignment features 1873 of the complementary connector component 1800.

The at least one additional alignment feature 8720 may be provided on an outer surface of the radially inner wall 11702a of the body 11701 of the connector 11700.

The features, options, and functionality of the at least one additional alignment feature may be as described above for the alignment feature(s) 8720 of the connector 8700.

A bore 11703 of the connector component extends from the terminal end 11704 in a direction away from the terminal end 11704.

The bore 11703 defines a gases lumen.

As shown in Figure 75, the bore 11703 of the connector 11700 comprises a taper 11000A1 between opposed surfaces of the bore such that a portion of the bore 11703 proximal to the terminal end 11704 has a larger transverse dimension than a portion of the bore 11703 more distal from the terminal end 11704.

In some configurations, the taper 11000A1 is between about 0 degrees and about 15 degrees. In some configurations, the taper 11000A1 is more than 0 degrees and up to about 15 degrees.

As shown in Figure 75, the cavity 11702c comprises a taper 11000A3 between the radially inner wall 11702a and the radially outer wall 11702b such that a portion of the cavity proximal to the terminal end 11704 has a larger transverse dimension than a portion of the cavity more distal from the terminal end 11704. The taper may assist with enabling insertion of the complementary connector component 1800 into the cavity 11702c.

In some configurations, the taper 11000A3 is between about 0 degrees and about 15 degrees. In some configurations, the taper 11000A3 is more than 0 degrees and up to about 15 degrees.

The connector 11700 comprises a sealing region 11705 for engaging with a seal 1841 on the complementary connector component 1800.

The sealing region 11705 is provided by an inner surface of the radially outer wall 11702b.

The sealing region 11075 is adjacent to the terminal end 11704 of the cavity.

The radially inner wall 11702a is configured for receipt in the bore 1822 of the complementary connector component 1800 with a frictional engagement therebetween. Once the radially inner wall 11702a is received in the bore 1822, it is retained therein in use by a frictional engagement between the radially inner wall 11802a and the wall 8702 of the bore 8722.

In the configuration shown, the complementary connector component 1800 comprises engagement fingers 1820, and the bore 1822 of the complementary connector component 1800 is between the engagement fingers 1820. In alternative configurations, the bore 1822 could be provided elsewhere in the complementary connector component 1800.

The engagement fingers 1822 of the complementary connector component 1800 are received in the cavity 11702c when the connection assembly is assembled.

In some configurations, the connector component comprises a compliant material. In some configurations, a portion the radially inner wall 11702a comprises the compliant material. In some configurations, the radially outer wall 11702b comprises the compliant material. In some configuration, a portion of the radially inner wall 11702a and a portion of the radially outer wall 11702b comprise the compliant material. The compliant material may, for example, comprise an elasticised or elastic-type material, such as silicone or a thermoplastic elastomer (TPE) type material. In some configurations, the compliant material comprises a thermoplastic elastomer material. In some configurations, the thermoplastic elastomer comprises thermoplastic vulcanizate for example.

In the configuration shown, the body 11701 of the connector 11700 comprises a distal portion 11701b. The distal portion 10701b may have any one or more of the features described above for the distal portion 8701b of the connector 8700.

The bodies 8701, 9701, 10701, 11701 are shown as being generally circular in a cross-section that is transvers to an axial direction of the bodies. It will be appreciated that the bodies 8701, 9701, 10701, 11701 could have a different cross-sectional shape, depending on the shape of the complementary connector component.

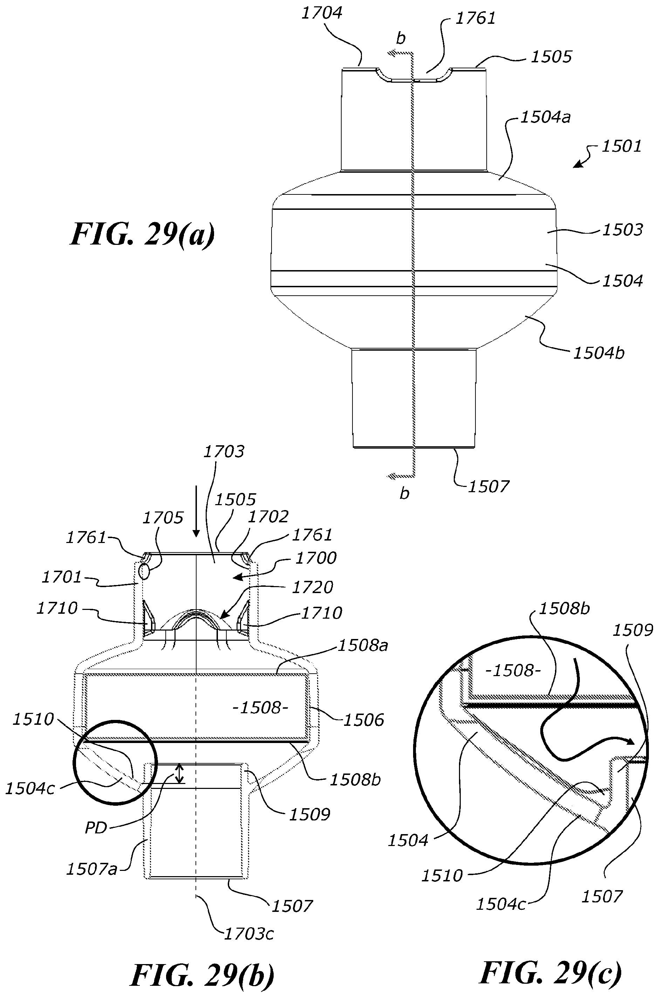

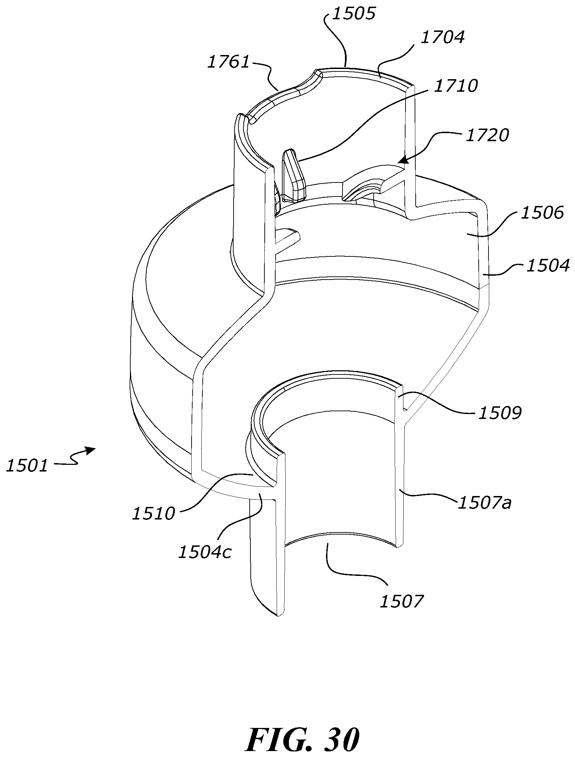

Figures 29(a) to 31(e) shows an alternative filter 1501 for use in a respiratory system. Unless described as otherwise below, the features, functionality, and options are the same as for the filter 501. Like reference numerals indicate like parts with the addition of 1000.

The filter 1501 comprises a filter housing 1504, an inlet port 1505 for the flow of gases, and an outlet port 1507 for the flow of gases. The filter housing 1504 comprises a cavity 1506 containing a filtration material 1508 in a flow path through the filter. The filtration material 1508 has a first side 1508a proximal to the inlet port 1505 and a second side 1508b proximal to the outlet port 1507. The filter 1501 further comprises a projection 1509 proximal to the outlet port 1507 and extending towards the second side 1508b of the filtration material 1508.

Condensate can form in a filter owing to environmental factors such as ambient temperature surrounding the filter housing. When therapy is provided with humidified gases, mobile condensate may form due to the saturation of filtration material and temperature gradients.

The projection 1509 is configured to inhibit a flow of liquid between the cavity 1506 and the outlet port 1507. That enables the projection 1509 to inhibit mobile condensate from exiting the filter housing 1504 via the outlet port 1507 and being propelled to the patient interface. The projection 1509 can also inhibit the movement of condensate which may arise due to reorientation of the filter housing 1504.

In the configuration shown, the inlet port 1505 and the outlet port 1507 of the filter 1501 are substantially coaxial. In alternative configurations, the inlet port 1505 and the outlet port 1507 may not be coaxial. By way of example, one of the inlet port 1505 and the outlet port 1507 may be in the side of the filter, and the other of the inlet port 1505 and the outlet port 1507 may be in the end of the filter.

In the configuration shown, the filter 1501 has one inlet port 1505 and one outlet port 1507. In alternative configurations, the filter 1501 may have more than one inlet port 1505 and/or more than one outlet port 1507. When the filter has more than one outlet port 1507, the projection 1509 may be provided for one of the outlet ports or may be provided for some or each of the outlet ports.

The projection 1509 extends into the cavity 1506 from the location where the body 1507a that defines the outlet port 1507 intersects with an adjacent wall portion 1504c of the filter housing 1504.

Referring to Figure 29(c), the projection 1509 and the adjacent wall portion 1504c provide a condensate accumulation zone 1510. That helps prevent condensate being carried out of the outlet port 1507 in the gases flow, with the direction of gases flow being shown by the arrows in Figures 29(b) and 29(c).

Referring to Figure 29(b), the projection 1509 extends into the cavity by distance PD. Distance PD may be selected to reduce resistance to flow while retaining a sufficient volume of condensate. The sufficient volume of condensate may relate to the application and/or length of time to which the filter 1501 is used.

The distance PD may be sufficient for the projection 1509 to contact the second side 1508b of the filtration material 1508. Alternatively, the distance PD may be shorter so that the projection 1509 does not contact the second side 1508b of the filtration material 1508. Increasing the distance PD may increase the resistance to flow through the filter housing 1504.

The projection 1509 could have any distance PD in practice. The distance PD may depend on the overall size of the filter 1501. If the filter 1501 is too large, it can take up undesired space, particularly if the filter is used close to the patient. That could make the use of the breathing circuit unwieldy.

For example, the distance PD may be between about 1 mm and about 15 mm, optionally between about 3 mm and about 10 mm, and optionally about 5 mm. In some configurations, the distance PD may be about 1 mm, about 2 mm, about 3 mm, about 4 mm, about 5 mm, about 6 mm, about 7 mm, about 8 mm, about 9 mm, about 10 mm, about 11 mm, about 12 mm, about 13 mm, about 14 mm, or about 15 mm, or may be any value between any two of those values.

In the configuration shown, the projection 1509 is contiguous with the body 1507a that defines the outlet port 1507 and is an extension of the body 1507a. However, as described below, the projection 1509 may have different configurations.

The projection 1509 can inhibit the majority of mobile condensate but allow for some degree of drainage by a practitioner by tilting or inverting the filter 1501 when the filter 1501 is disconnected from the downstream component(s).

The projection 1509 is shown in a filter that has a connector 1700 for connecting with a complementary connector component 800, 1800. Alternatively the projection 1509 could be used an a filter that has a different connector arrangement for connecting the filter to a different respiratory system component.

As outlined above for connector 700 of filter 501, the connector 1700 may be attached to or integrally formed with the filter housing 1504.

The connector 1700 comprises a body 1701 defining a bore 1703 for receipt of the complementary connector component 800, 1800, and the body 1701 defines the inlet port 1505 of the filter 1501.

The connector 1700 comprises one or more retaining features 1710 to assist with retaining the complementary connector component 800, 1800 in engagement with the connector 1700. Additionally, or alternatively, the connector 1700 comprises at least one internal alignment feature 1720 and/or at least one external alignment feature 1761.

The connector 1700 may have any one or more of the features outlined herein for the connector 700.

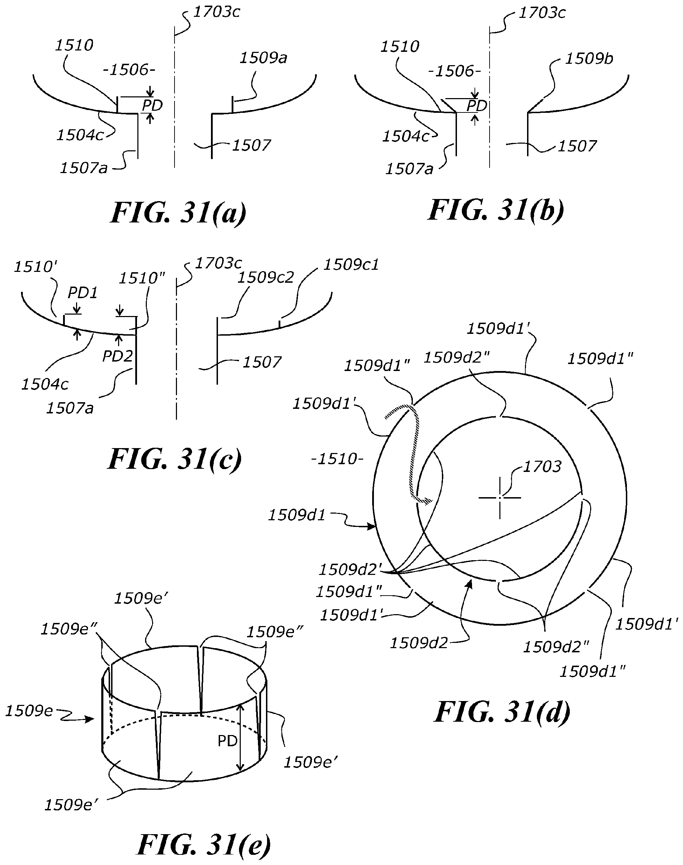

Figures 31(a) to 31(e) show alternative configurations of the projection 1509 for inhibiting the flow of liquid to between the cavity 1506 and the outlet port 1507.

In the configuration of Figure 31(a), the projection 1509a is coaxial with the outlet port 1507. However, the projection 1509a has a larger transverse dimension and is offset from the body 1507a that defines the outlet port 1507.

The projection 1509a can inhibit the majority of mobile condensate but allow for some degree of drainage by a practitioner by tilting or inverting the filter 1501 when the filter 1501 is disconnected from the downstream component(s).

In the configuration of Figure 31(b), the projection 1509b is frustoconical or angled so as to be non-parallel with an axis 1703c extending through the outlet port 1507. The portion of the projection 1509b proximal to the filtration material 1508 is wider than the portion of the projection 1509b proximal to the outlet port 1507, which may enhance gases flow through the filter 1501 and/or may minimise any increase to resistance to flow at the outlet port 1507. The projection 1509b may be contiguous with the body 1507a or may be offset from the body 1507a as described for Figure 31(a).

The projection 1509b can inhibit the majority of mobile condensate but allow for some degree of drainage by a practitioner by tilting or inverting the filter 1501 when the filter 1501 is disconnected from the downstream component(s).

In the configuration of Figure 31(c), the filter is provided with two projections 1509c1, 1509c2. The first projection 1509c1 is an outer projection and the second projection 1059c2 is an inner projection. Both projections could be substantially solid around their peripheries, or could be provided with one or more gaps in their peripheries. The configuration of Figure 31(c) provides a first condensate accumulation zone 1510' distal from the outlet port 1507 and a second condensate accumulation zone 1510" proximal to the outlet port 1507. The two projections may have different depths PD1, PD2 or may have the same depths.

The projections 1509c1, 1509c2 can inhibit the majority of mobile condensate but allow for some degree of drainage by a practitioner by tilting or inverting the filter 1501 when the filter 1501 is disconnected from the downstream component(s).

The configuration of Figure 31(d) is similar to that of Figure 31(c) in that it has two projections 1509d1, 1509d2 to provide two condensate accumulation zones 1510', 1510". In this configuration, the outer projection 1509d1 comprises a plurality of wall segments 1509d1' with gaps or splits 1509d1" between adjacent wall segments. The inner projection 1509d2 comprises a plurality of wall segments 1509d2' with gaps or splits 1509d2" between adjacent wall segments.

The outer projection 1509d1 may comprise two, three, four, or more wall segments. The inner projection 1509d2 may comprise two, three, four, or more wall segments.

The outer gaps or splits 1509d1" are offset from the inner gaps or splits 1059d2". There is no direct radial path for condensate from the outer condensate accumulation zone 1510', through the inner condensate accumulation zone 1510", to the outlet port 1507. Instead, any condensate would need to follow a tortuous path from the outer condensate accumulation zone 1510' to the outlet port 1507, as indicated by the arrow in Figure 31(d) for example.

The projections 1509d1, 1509d2 can inhibit the majority of mobile condensate but allow for some degree of drainage by a practitioner by tilting or inverting the filter 1501 when the filter 1501 is disconnected from the downstream component(s).

In the configuration of Figure 31(e), the projection 1509e comprises a plurality of wall segments 1509e' with crenellations provided by gaps or splits 1509e" that extend at least part of the depth of the projection 1509e. The gaps or splits 1509e" are provided between adjacent wall segments 1509e'. This provides a projection 1509e with a non-constant or varying height (e.g. distance PD) around the periphery of the projection 1509e.

The projection 1509e can inhibit the majority of mobile condensate but allow for some degree of drainage by a practitioner by tilting or inverting the filter 1501 when the filter 1501 is disconnected from the downstream component(s). This type of configuration could alternatively be used in a filter having two or more projections, such as that shown in Figure 31(c) for example.

In the configurations shown, the projections 1509, 1509a-1509e have a generally annular configuration. The projections 1509, 1509a-1509e are shown as being circular. The projections 1509, 1509a-1509e could have any suitable shape depending on the shape of the outlet port 1507 and/or the desired shape of the condensate accumulation zone(s).

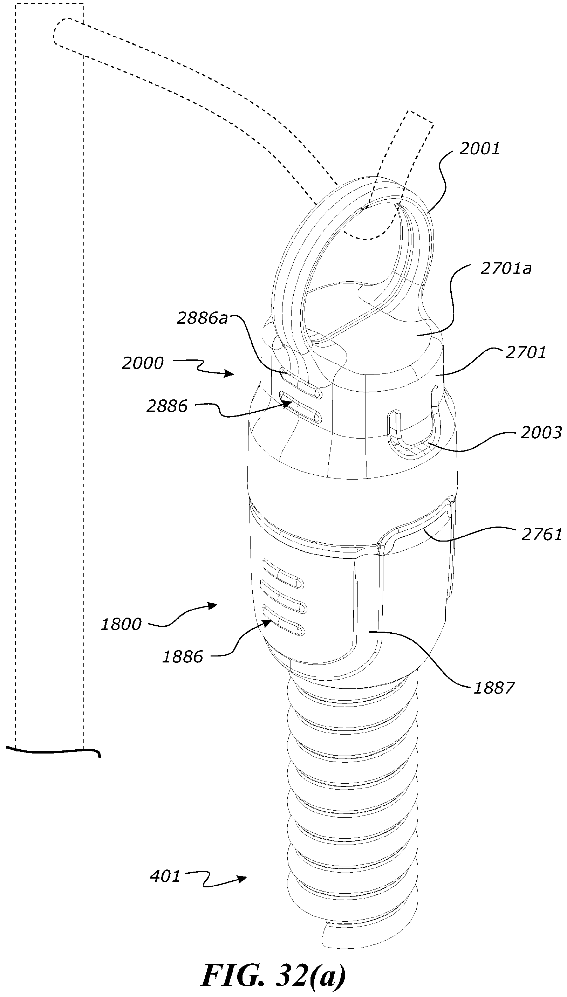

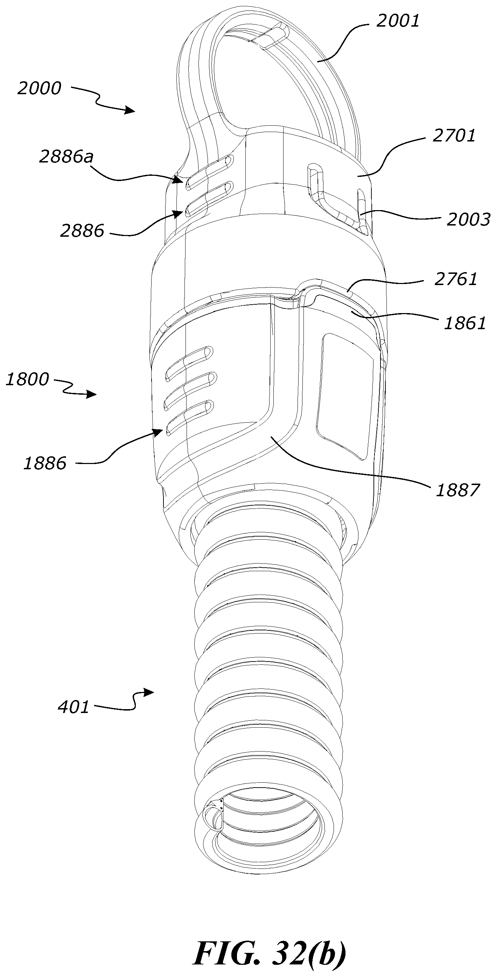

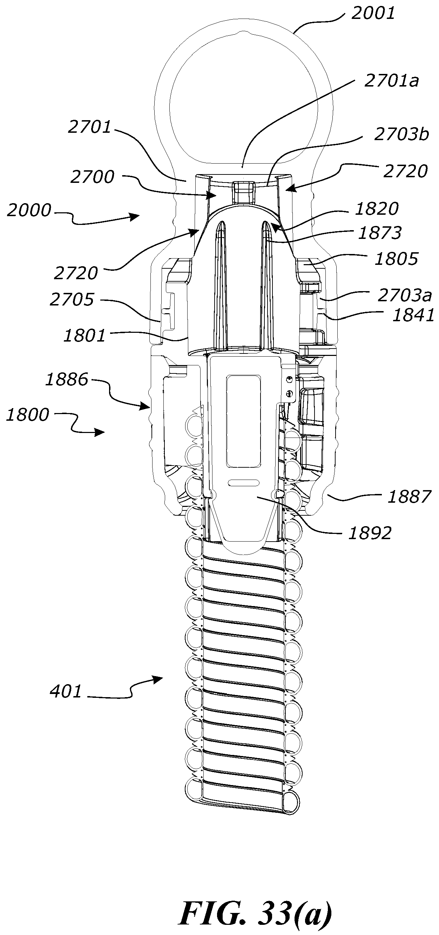

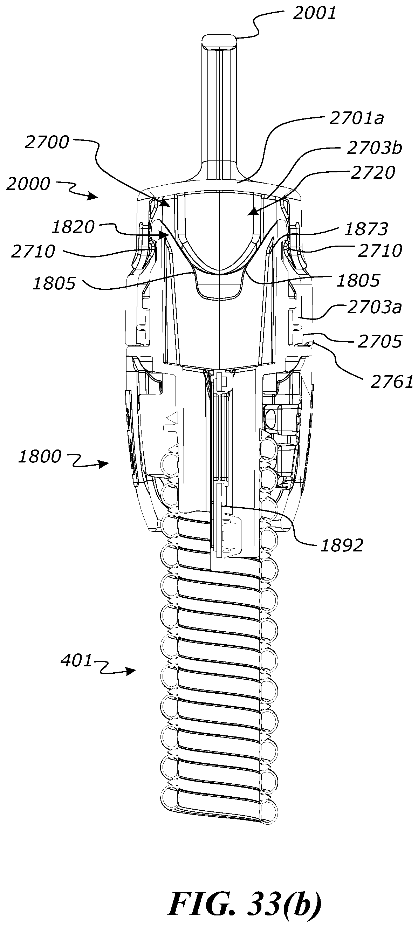

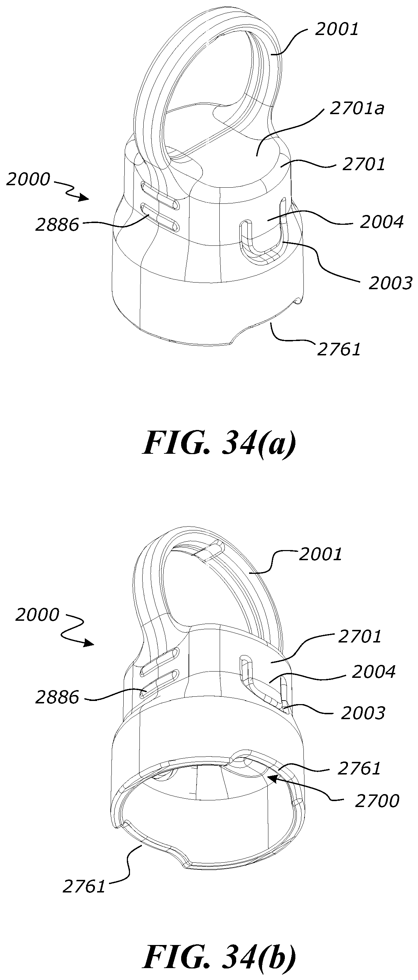

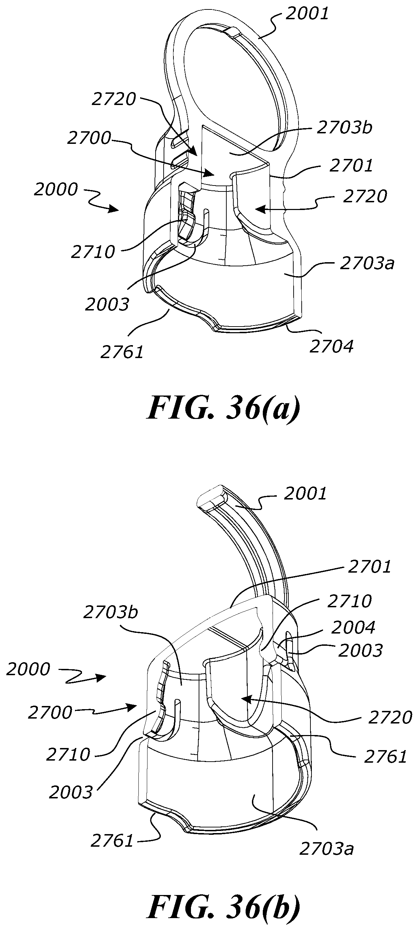

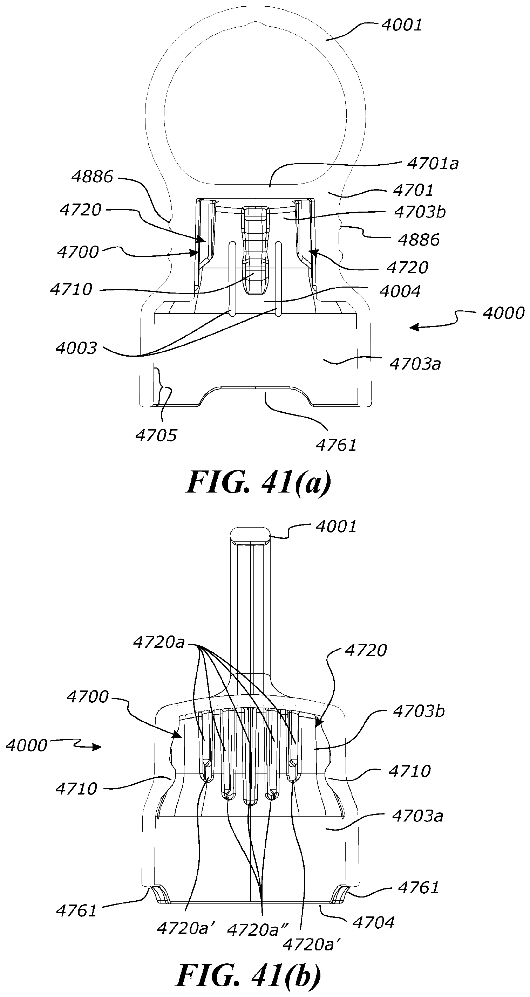

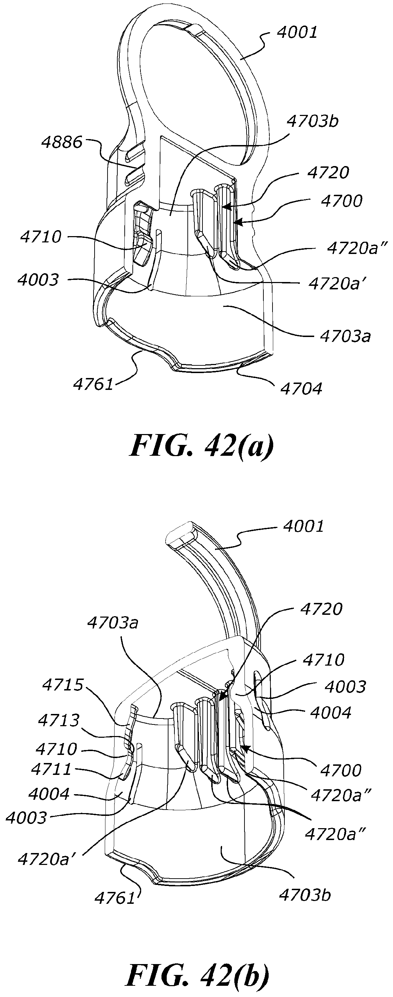

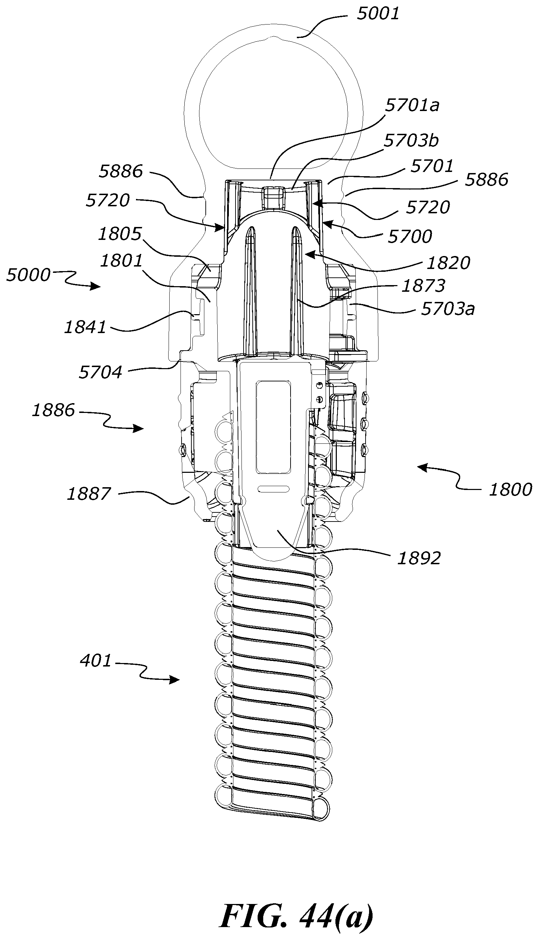

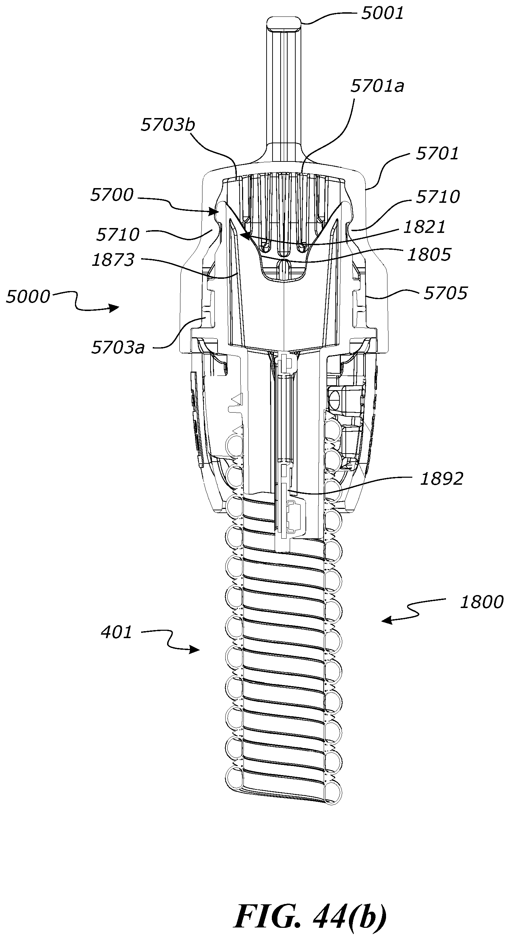

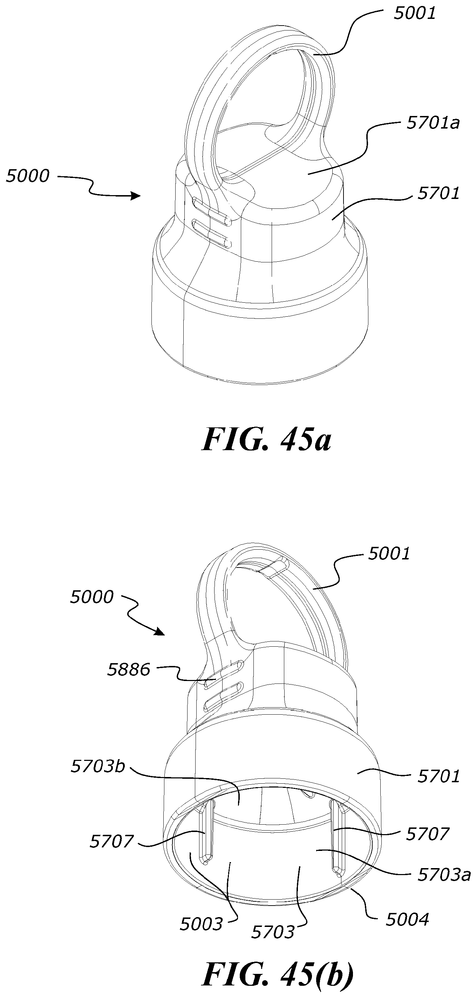

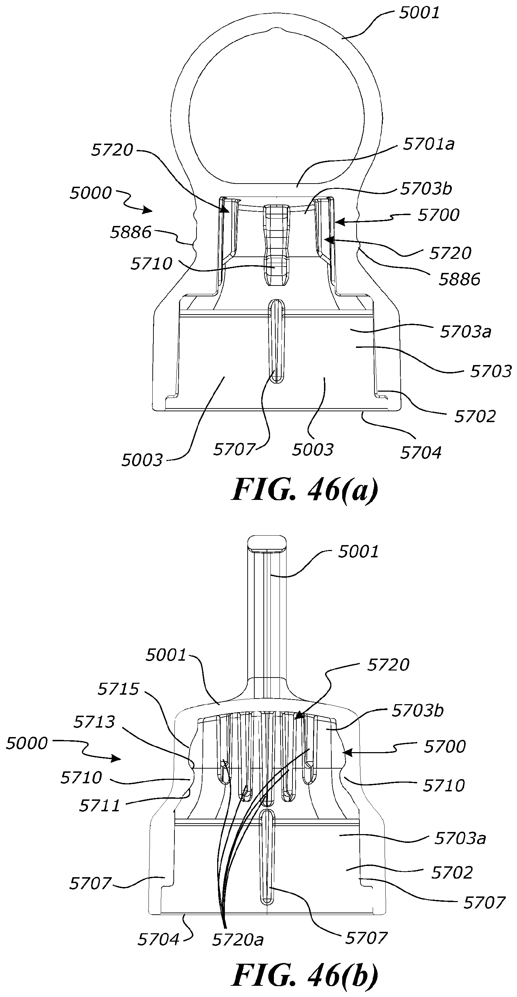

Figures 32(a) to 49 show respiratory conduit end caps 2000, 3000, 4000, 5000, 6000, 7000 (also referred to herein more simply as "end cap" or "end caps") for use with respiratory conduits such as inspiratory conduit 401. The end caps 2000, 3000, 4000, 5000, 6000, 7000 each comprise a connector 2700, 3700, 4700, 5700, 6700, 7700 for connecting with the complementary connector component 1800 of the conduit 401. Unless described as otherwise below, the features, functionality, and options of the connectors 2700, 3700, 4700, 5700, 6700 are the same as for the connector 700 of the filter 501, and like reference numbers indicate like parts with the addition of 2000, 3000, 4000, 5000, 6000, and 7000 respectively.

Although the end caps are shown as connecting to the complementary connector component 1800, they could alternatively connect to the complementary connector component 800 or any other suitable complementary connector component.

Referring to Figures 32(a) to 36(b), the end cap 2000 comprises a body 2701 defining a bore 2703 for receipt of a complementary connector component 1800 of the respiratory conduit 401. The bore 2703 defines a cavity 2703b for receipt of gases from the respiratory conduit 401.

In the configuration shown, the bore 2703 comprises a terminal end 2704.

An enlarged entry portion of the bore 2703a is provided proximal to the terminal end 2704. A reduced size portion that forms the cavity 2703b is provided distal from the terminal end 2704. An end wall 2701a provides a substantially closed end of the cavity 2703b. The bore 2703 could have different configurations. For example, the entry portion 2703a and the cavity 2703b could have a substantially constant cross-sectional size.

The end cap 2000 comprises a support feature 2001 for supporting the end cap 2000. The support feature 2001 may be integrally formed with the body 2701 or may be coupled to the body 2701.

The support feature 2001 can be used to support the end cap 2000 from another item.

The connector 2700 for connecting with the complementary connector component 1800 comprises at least one alignment feature and one or more retaining features to assist with retaining the end cap 2000 on the complementary connector component 1800 of the respiratory conduit 401.

The at least one alignment feature comprises at least one internal alignment feature and/or at least one external alignment feature.

The at least one alignment feature is configured to align the one or more retaining features with one or more complementary engagement features on the complementary connector component.

In the configuration shown, the at least one internal alignment feature comprises at least one alignment member 2720 that extends into the bore 2703 from an inner wall 2702 of the body 2701.

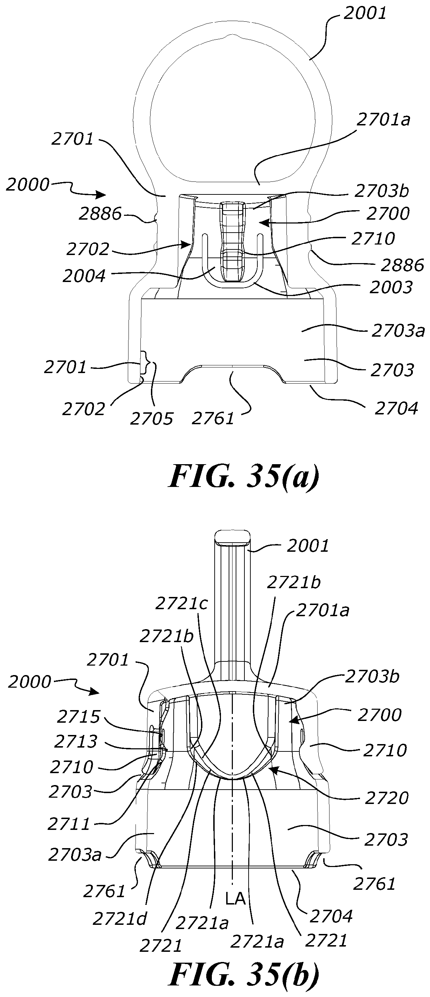

With reference to Figure 35(b), the alignment member 2720 comprises opposed engagement surfaces 2721. The opposed engagement surfaces are outwardly facing. The alignment member 2720 has a tapered configuration in an axial direction of the bore 2703 wherein proximal ends 2721a of the opposed engagement surfaces 2721 that are more proximal to the terminal end 2704 of the bore 2703 are closer together than distal ends 2721b of the opposed engagement surfaces 2721 that are more distal from the terminal end 2704 of the bore 2703. The engagement surfaces 2721 comprise a twist along at least a substantial part of a length thereof between their proximal ends 2721a and their distal ends 2721b.

In some configurations, the twist extends along substantially the entire length of each engagement surface 2721.

In some configurations, the twist is substantially continuous along the length of each engagement surface 2721.

The twist may have any one or more features described herein in relation to the twist 721.

The alignment member 2720 comprises a tapered configuration in a radial direction of the bore, wherein inner edges 2721c of the opposed engagement surfaces 2721 more proximal to the longitudinal or central axis LA of the bore are positioned closer together than outer edges 2721d of the opposed engagement surfaces 2721 more distal from the longitudinal or central axis LA of the bore and more proximal to the inner wall 2702.

The engagement surfaces 2721 are configured to interact with the complementary engagement surfaces 1821 to cause the complementary connector component 1800 to rotate into a correct alignment about a longitudinal axis LA of the connector, if the complementary connector component 1800 is misaligned upon initial insertion into the bore 2703 of the connector 2700.

The alignment member 2720 comprises a substantial chevron shape.

The internal alignment feature is configured to provide haptic feedback during connection of the end cap 2000 to the complementary connector component 1800, by providing a resistance that is experienced by a user making the connection, owing to the arrangement of the alignment features.

The connector 2700 may comprise two diametrically opposed alignment members 2720. Each alignment member 2720 may have substantially the same shape and functionality.

In the configuration shown, the body 2701 of the connector 2700 comprises one or more external alignment feature(s) 2761 that is/are configured to interact with complementary external alignment feature(s) 1861 on the complementary connector component 1800.

The external alignment feature(s) 2761 may comprise one or more protrusions or recesses and the complementary external alignment feature(s) 1861 may comprise one or more complementary recesses or protrusions. The protrusion(s) will be arranged to be received in the recess(es).

The protrusion(s) and recess(es) may be axially oriented.

In the configuration shown, the external alignment feature(s) 2761 comprise one or more axially oriented recesses located in the body 2701 of the connector 2700 at the terminal end 2704 of the bore 2703.

The complementary external alignment feature(s) 1861 comprise one or more axially oriented protrusions protruding from a portion of the body 1801 in the region between the proximal portion 1800a and the distal portion 1800b.

The external alignment feature(s) 2761 and complementary external alignment feature(s) 1861 may have a tapered configuration in the axial direction to assist with guiding the feature(s) 2761, 1861 into contact with one another.

The external alignment feature(s) 2761, 1861 act to physically align the connector 2700 and complementary connector component 1800 as they are connected with each other. Additionally, they can provide an externally visible aid for alignment of the connector 2700 and complementary connector component 1800.

The connection assembly may comprise one, two, three, four, or more external alignment features 2761 and complementary external alignment features 1861.

The one or more retaining features comprises one or more retaining protrusions 2710 that extend into the bore 2703 from the inner wall 2702 of the body 2701.

In the configuration shown, the retaining features comprise diametrically opposed retaining protrusions 2710. In alternative configurations, the connector 2700 may comprise one, two, three, four, or more retaining protrusions 2710.

Referring to Figure 35(b), the retaining protrusions 2710 each have an angled or chamfered entry wall 2711 to assist with guiding/deflecting locking fingers 1820 radially inward and over the retaining protrusions 2710 during insertion of the complementary connector component into the bore 2703, a radially inward surface 2713, and/or a retaining face 2715 to assist with preventing unintended disengagement of the complementary connector component 1800 from the bore 2703. The radially inward end of the retaining face 2715 terminates at the radially inward surface 2713.

The retaining protrusion(s) 2710 may provide an audible click when engaged with the engagement features 1810.

The end cap 2000 comprises at least one vent 2003 for a flow of gases from the cavity 2703b to an external surface of the end cap 2000.

The at least one vent 2003 provides a bypass flow feature for the flow of gases from the respiratory conduit 401.

The at least one vent 2003 comprises a channel or aperture in the body 2701 of the end cap 2000.

The at least one vent 2003 can have any suitable configuration. In the configuration shown the at least one vent 2003 comprises a channel having a generally U-shaped configuration. In an alternative configuration, the generally U-shaped configuration could be provided by a plurality of apertures instead of a channel. The generally U-shaped configuration of the vent provides a resiliently flexible tongue 2004 in the body 2001 that has greater flexibility than adjacent portions of the body 2001.

The retaining protrusion 2710 is provided adjacent the vent 2003. In the configuration shown, the retaining protrusion 2710 is provided on the tongue 2004. By providing the retaining protrusion 2710 adjacent the vent 2003, the connection/disconnection forces can be configured to a desired level.

The connection/disconnection forces may be configured to enable the end cap 2000 to support the weight of the respiratory conduit 401 and resist flow through the conduit 401 and the at least one vent 2003, but to enable a user to disconnect the end cap 2000 from the respiratory conduit 401.

The connection forces can be altered by changing the stiffness/rigidity of the resiliently flexible tongue 2004. The stiffness/rigidity of the resiliently flexible tongue 2004 may be impacted by the area of the vent 2003. The connection forces could be reduced by increasing the length of the at least one vent 2003 in an axial direction of the end cap 2000 or could be increased by reducing the length of the at least one vent 2003 in the axial direction of the end cap 2000.

In some configurations, the one or more retaining features is/are configured with respect to the size of the at least one vent 2003 to enable a flow of gases through the conduit 401 and the vent 2003 of the end cap 2000 of up to about 60 litres per minute, and optionally of up to about 90 litres per minute, without the end cap disconnecting from the complementary connector component 1800. The end cap can be configured to enable a flow of gases of more than 90 litres per minute without the end cap disconnecting from the complementary connector component.

The body 2701 has a sealing region 2705 in the bore 2703 for engaging with a seal 1841 on the complementary connector component 1800. This encourages the gases from the respiratory conduit 401 to pass through the at least one vent 2003.

The end cap 2000 could have one, two, three, four, or more vents 2003. In the configuration shown, the end cap 2000 comprises two diametrically opposed vents 2003, with a retraining protrusion 2710 provided on each tongue 2004 adjacent a respective vent 2003.

The support feature 2001 is configured so that the respiratory conduit end cap 2000 and a connected respiratory conduit 401 can be hung from another item by the support feature. Said another item could be any suitable item such as a medical stand or pole, or a wall hook or support for example. Figure 32(a) schematically shows the end cap 2000 and connected respiratory conduit hanging from an arm of a medical stand or pole.

In the configuration shown, the support feature 2001 comprises a loop 2001 positioned at an opposite end of the body 2701 from the terminal end 2704 of the bore. The loop may be circular or non-circular. The support feature 2001 could have a different configuration. For example, the support feature 2001 could comprise a hook or a latch to connect to said another item. Additionally, or alternatively, the support feature 2001 could be provided elsewhere on the end cap 2000; for example, on the side of the end cap.

The support feature 2001 may be sized to accommodate a user's finger and/or thumb. The support feature 2001 may be used, in conjunction with the grip surface 1886 of the complementary connector component 1800, to disconnect the end cap 2000 from the complementary connector component 1800 and thereby the end cap 2000 from the respiratory conduit 401.

The support feature 2001 is oriented so that its long dimension is aligned with the grip surfaces 1886 of the complementary connector component 1800. The long dimension of the support feature 2001 is transverse to the grip surfaces 1886.

The end cap 2000 may comprise a grip surface 2886. The grip surface 2886 may comprise a surface texture to provide a grasping surface for a user. In the configuration shown, the grip surface 2886 comprises one or a plurality of protrusions 2886a that project(s) outwardly from a wall of the body 2701. Additionally, or alternatively, the grip surface 2886 may comprise one or a plurality of recesses that extend(s) inwardly into a wall of body 2701.

The protrusion(s) and/or recess(es) may partially extend in transverse direction across the periphery of the wall of the body 2701, to enable an axial force to be applied to the end cap 2000. For example, the protrusion(s) and/or recess(es) may comprise a spline or waveform or curved configuration that has a portion that extends in the transverse direction. In another configuration, the protrusion(s) and/or recess(es) may extend substantially in the transverse direction. In that configuration, the protrusion(s) and/or recess(es) may be substantially straight.

Additionally, or alternatively, the grip surface 2886 may comprises a rough or high friction surface finish.

The grip surface 2886 may be provided on opposed outer sides of the body 2701.

The grip surfaces 2886 may be aligned with the ends of the support feature 2001 where they contact the body 2701 adjacent its end wall 2701a. The grip surfaces 2886 may be aligned with the grip surfaces 1886 to provide a visual indicator of when the end cap 2000 is correctly aligned with the complementary connector component 1800, and to encourage a user to use the grip surfaces 1886, 2886 to connect/disconnect the end cap 2000 and complementary connector component 1800. The grip surfaces 1886, 2886 may reduce twisting of the respiratory conduit 401 when the grip surfaces are used to disconnect the end cap 2000 from the complementary connector component 1800. The external alignment features 1861, 2761 may further assist with that.

The end cap 2000 or at least its body may be formed of any suitable materials. Example materials include rigid polymeric materials that may be biocompatible. Example materials include Polycarbonate (PC), Polyethylene (PE), Acrylonitrile Butadiene Styrene (ABS) or polypropylene (PP).

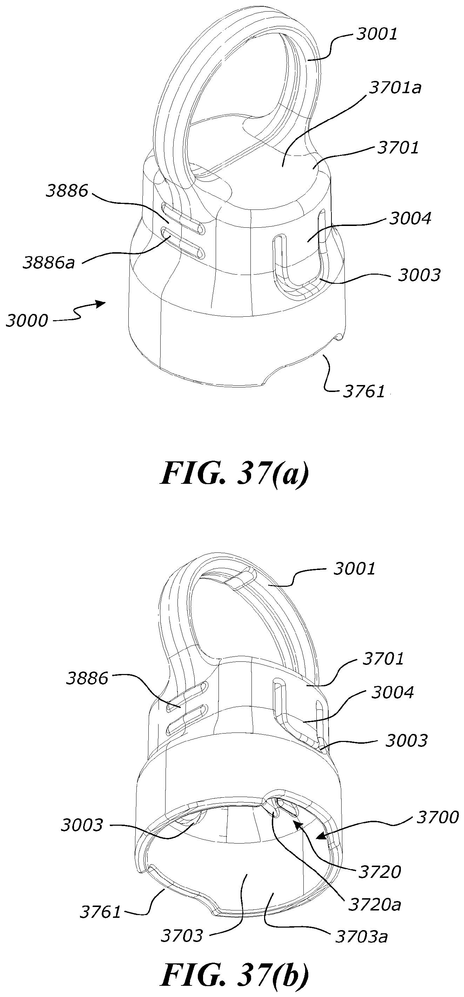

Figures 37(a) to 39(b) show an alternative configuration respiratory conduit end cap 3000. Unless described as otherwise below, the features, functionality, and options are the same as for end cap 2000, and like reference numbers indicate like parts with the addition of 1000.

In this configuration, the at least one alignment member 3720 comprises or is formed by a plurality of ribs 3720a that extend in an axial direction of the bore 3703. Using a plurality of ribs 3720a may provide manufacturability benefits.

In the configuration shown, each alignment member 3720 comprises five ribs, but in alternative configurations, each alignment member could have a greater or smaller number of ribs, such as three, four, five, six, or more ribs for example.

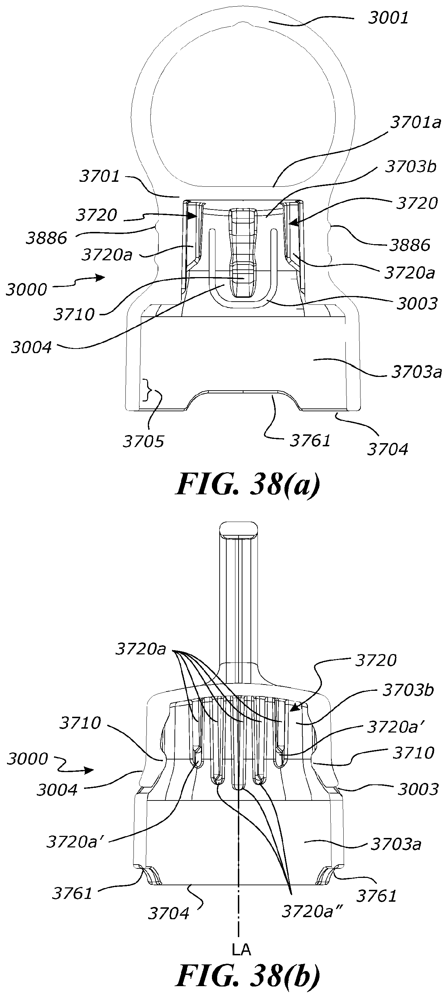

The ribs 3720a are configured to approximate the shape of the alignment member 2720 of the previous configuration. In particular, and with reference to Figure 38(b), the outer ribs are shorter than their adjacent ribs, which in turn are shorter than the central rib(s).

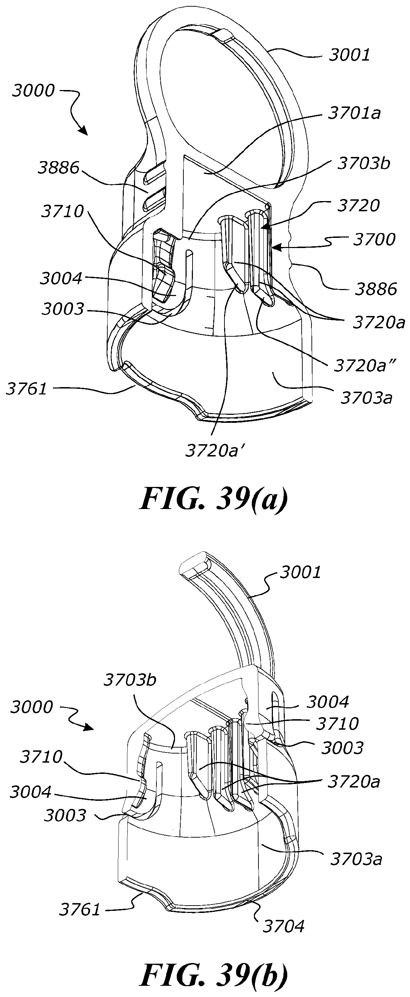

With reference to Figures 38(b) and 39(a), the two outer ribs have tapered leading edges 3720a', and the other, more central ribs, have leading edges 3720a" which are substantially normal to the inner wall 3702 of the body 3701. This enables the ribs 3720a to approximate the twist of the alignment member 2720 of the previous configuration.

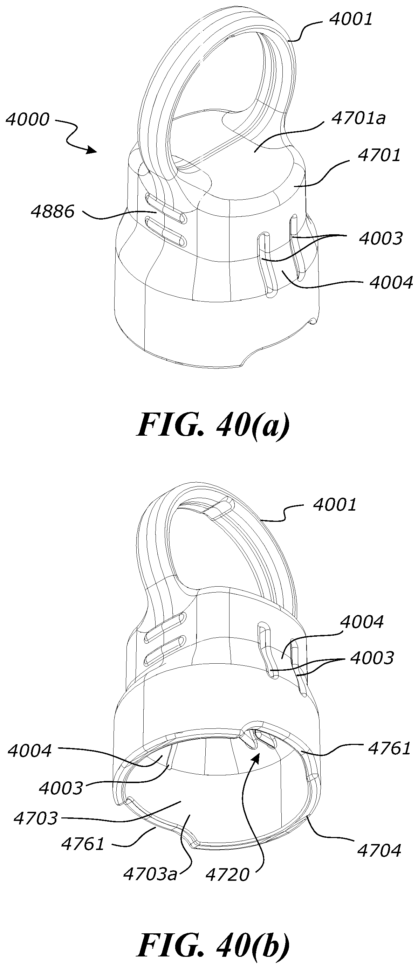

Figures 40(a) to 42(b) show an alternative configuration respiratory conduit end cap 4000. Unless described as otherwise below, the features, functionality, and options are the same as for end cap 3000, and like reference numbers indicate like parts with the addition of 1000.

In this configuration, the at least one vent 4003 for a flow of gases from the cavity 4703b to the external surface of the end cap 4000 is provided by two spaced apart channels.

The channels are provided in the body 4001 of the end cap 4000.

The channels are shown as being substantially parallel to each other and extending in an axial direction of the bore 4703. In alternative configurations, the channels could be non-parallel and/or could extend in a non-axial direction of the bore 4703.

Rather than having one free end and one connected end, the resiliently deformable tongue 4004 provided between the channels has two fixed ends.

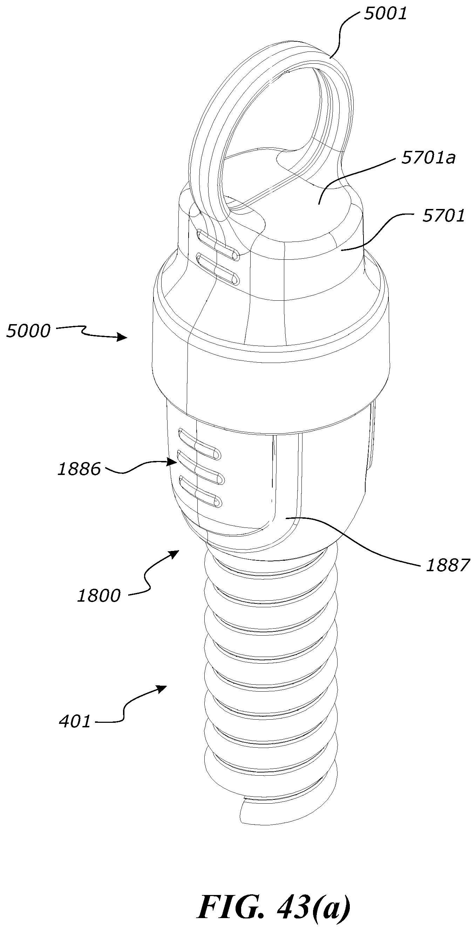

Figures 43(a) to 47(b) show an alternative configuration respiratory conduit end cap 5000. Unless described as otherwise below, the features, functionality, and options are the same as for end cap 3000, and like reference numbers indicate like parts with the addition of 2000.

In this configuration, the at least one vent 5003 for a flow of gases from the cavity 5703b to an external surface of the respiratory conduit end cap 5000 comprises a gap between the body 5701 and a portion of the complementary connector component 1800 when the respiratory conduit end cap 5000 is connected to the complementary connector component 1800 of the respiratory conduit 401.

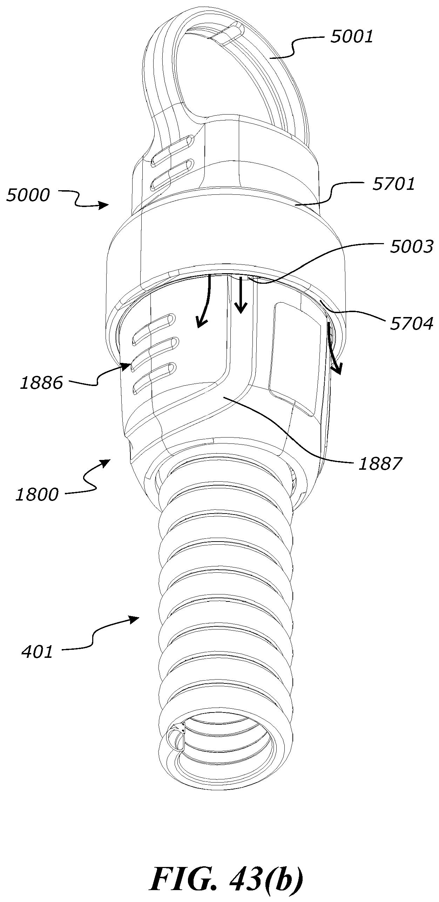

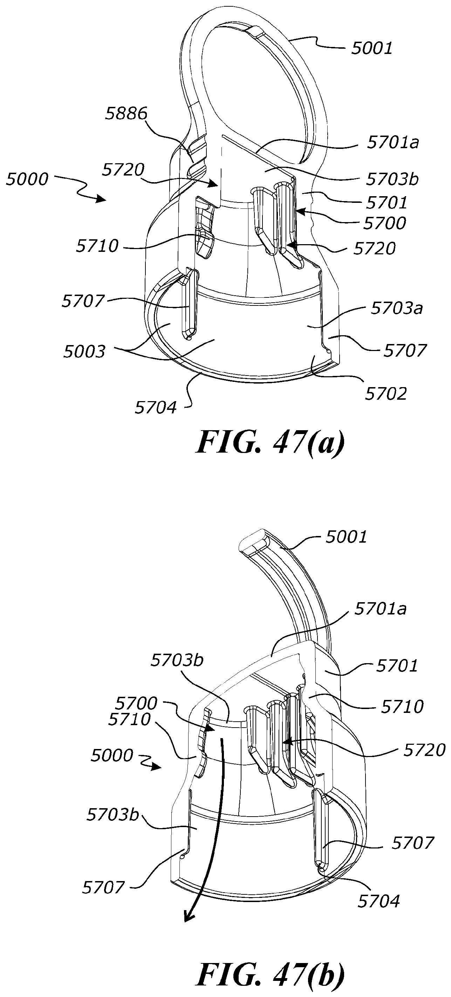

This configuration end cap 5000 does not have a sealing region for sealing with the seal 1841 on the complementary connector component. Instead, the gases may vent out between the end cap 5000 and the complementary connector component 1800 in the direction indicated by the arrows in Figure 43(b) and 47(b). In some configurations, the end cap 5000 cooperates with the complementary connector component 1800 to allow gases to vent out via surface features 1887 of grip surface 1886.

One or more ribs 5707 are provided in the interior of the cavity 5703. The ribs 5757 may extend inwardly into the proximal portion 5703a of the cavity from the wall 5702 of the body 5701.

The rib(s) 5707 are configured to provide anti-rocking functionality to inhibit rocking of the complementary connector component 1800 relative to the end cap 5000. The rib(s) 5707 may engage with the seal 1841, with gaps provided between the ribs 5707 to provide the vents 5003.

When a plurality of ribs 5707 are provided, they may each have the same length or at least some of the ribs may have differing lengths.

The longer ribs 5707 may be configured to be received in the channels 1887 adjacent the grip surface 1886 on the complementary connector component 1800 to provide alignment functionality.

The ribs 5707 extend in an axial direction of the end cap 5000 but may alternatively be oriented in a different direction.

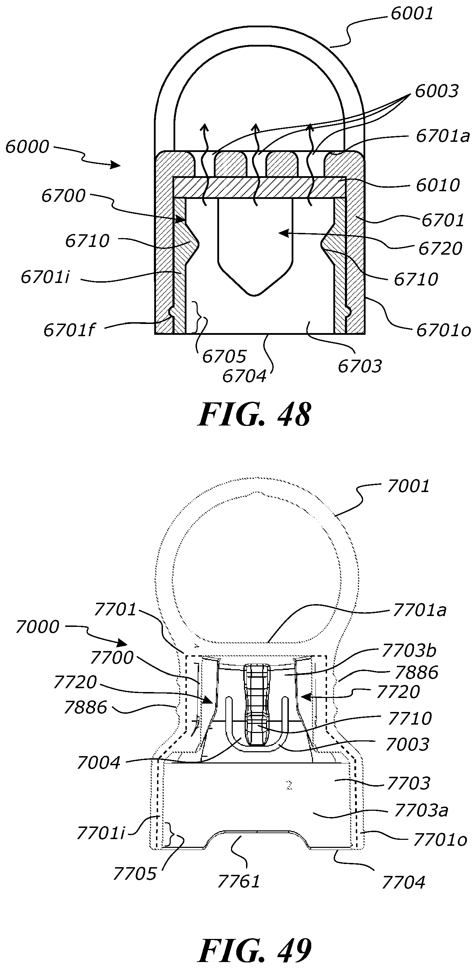

Figure 48 shows an alternative respiratory conduit end cap 6000. Unless described as otherwise below, the features, functionality, and options are the same as for end cap 2000, and like reference numbers indicate like parts with the addition of 4000.

In this configuration, the at least one vent 6003 is provided by one or more apertures or channels in the end wall 6701a of the body 6701 of the end cap 6000. A filtration material 6010 is provided adjacent the aperture(s) or channel(s) to filter the gases that are exiting through the at least one vent 6003 of the end cap.

The body 6701 may be of unitary construction or may be made of a plurality of parts. In the form shown, the body 6701 comprises an outer moulding 6701o and an inner moulding 6701i.

The outer moulding 6701o and inner moulding 6701i may comprise complementary engagement features 6701f to retain the mouldings in engagement with each other. The complementary engagement features 6701f may be a protrusion engageable with a recess. The protrusion may be located on inner moulding 6701i and the recess may be located on the outer moulding 6701o as illustrated in figure 48. In some configurations, the protrusion may be located on outer moulding 6701o and the recess may be located on the inner moulding 6701i. There may be one or more complementary engagement features 6701f. The complementary engagement features 6701f may be discrete and/or continuous features.

The inner and outer mouldings may comprise the same materials. In another configuration, the inner moulding 6701i may comprise a softer or more flexible material than the outer moulding 6701o, to provide a desired level of resilience or flexibility for the at least one retaining protrusion 6710.

The other respiratory conduit end caps described herein may have unitary bodies or may have bodies that are made of a plurality of parts. For example, Figure 49 shows an alternative respiratory conduit end cap 7000 based on the end cap 3000. Unless described as otherwise below, the features, functionality, and options are the same as for end cap 3000, and like reference numbers indicate like parts with the addition of 4000.

The body 7701 of the end cap 7700 comprises an outer moulding 7701o and in inner moulding 7701i. The mouldings may have any one or more of the features described for the end cap 6000.

Any of the respiratory conduit end caps described herein may have any one or more of the features and functionality described in relation to any of the other respiratory conduit end caps.

The respiratory conduit end caps may each comprise a flexible tether to tether the end cap to the respiratory conduit 401.

By providing a combined end cap and support feature, the end caps 2000, 3000, 4000, 5000, 6000, 7000 can be used to store a heated respiratory conduit 401 between uses by retaining the support feature 2001, 3001, 4001, 5001, 6001, 7001 on another item.

The at least one vent 2003, 3003, 4003, 5003, 6003, 7003 enables a flow generator and/or humidifier to continue running while the respiratory conduit 401 is hanging in a 'resting' state, to maintain an appropriate temperature and/or humidity for therapy and reduce the time needed to start/resume therapy.

A positive flow of gases through the respiratory conduit 401 in the 'resting' state minimises the likelihood of pathogens/contaminants entering the respiratory conduit.

When the disclosed connection assemblies and/or components thereof are used between a filter 501 and an inspiratory conduit 401, the filter 501 can ensure that the components upstream of the filter 501 are not contaminated and the system components are correctly connected to provide the requisite therapy. This enables the system components upstream of the filter 501 to be reused for subsequent patients, which may be particularly beneficial in a multiple-patient and/or short time period use environment such as an ambulance or emergency department. With the disclosed connection assembly and components thereof, the connection assembly can be used between the filter 501 and the inspiratory conduit 401 and is configured such that the inspiratory conduit 401 only connects to the filter 501 and the patient interface conduit 603 cannot directly connect to the inspiratory conduit 401. After use by one patient, the filter 501 and downstream patient interface components can be disconnected from the inspiratory conduit 401 and replaced with new components for the next patient.

The connection assemblies and/or components thereof can be used with any suitable type of breathing assistance apparatus 1000.

The breathing assistance apparatus 1000 can be used in a variety of applications. For instance, the apparatus 1000 can be any of the following breathing assistance apparatuses or respiratory apparatuses: a continuous positive air pressure (CPAP) device, a ventilator, a humidifier, a high flow therapy device, a surgical humidifier (for example, an insufflator), an anaesthetic machine, combinations of the same, or the like.

CPAP treatment of obstructive sleep apnea involves the delivery of pressurized, breathable gas, usually air, to a user's airways using an inspiratory conduit and a patient interface, such as a mask. The gas pressures employed for CPAP typically range from about 4 cm H2O to about 28 cm H2O at flow rates of up to about 180 L/min (measured at the patient interface), depend upon the requirements of the user. The pressurized gas acts as a pneumatic splint for the airway of the user. As such, the pressurized gas reduces the likelihood of collapsing of the airway.

The breathing assistance apparatus 1000 may be a high flow therapy apparatus.

The respiratory system may be a high flow therapy apparatus or system. High flow therapy as discussed herein is intended to be given its typical ordinary meaning as understood by a person of skill in the art, which generally refers to a respiratory support system delivering a targeted flow of humidified respiratory gases via an intentionally unsealed patient interface with flow rates generally intended to meet or exceed inspiratory flow of a patient. Typical patient interfaces include, but are not limited to, a nasal or oral patient interface or a tracheostomy interface. Typical flow rates for adults often range from, but are not limited to, about fifteen litres per minute (LPM) to about seventy LPM or greater. Typical flow rates for paediatric patients (such as neonates, infants and children) often range from, but are not limited to, about one litre per minute per kilogram of patient weight to about three litres per minute per kilogram of patient weight or greater. High flow therapy can also optionally include gas mixture compositions including supplemental oxygen and/or administration of therapeutic medicaments. High flow therapy is often referred to as nasal high flow (NHF), humidified high flow nasal cannula (HHFNC), high flow nasal oxygen (HFNO), high flow therapy (HFT), among other common names. The flow rates used to achieve 'high flow" may be any of the flow rates listed below.

For example, in some configurations, for an adult patient "high flow therapy" may refer to the delivery of gases to a patient at a flow rate of greater than or equal to about 10 LPM, such as between about 10 LPM and about 120 LPM, or between about 10 LPM and about 100 LPM, or between about 15 LPM and about 95 LPM, or between about 20 LPM and about 90 LPM, or between 25 LPM and 75 LPM, or between about 25 LPM and about 85 LPM, or between about 30 LPM and about 80 LPM, or between about 35 LPM and about 75 LPM, or between about 40 LPM and about 70 LPM, or between about 45 LPM and about 65 LPM, or between about 50 LPM and about 60 LPM. In some configurations, for a neonatal, infant, or child patient 'high flow therapy' may refer to the delivery of gases to a patient at a flow rate of greater than 1 LPM, such as between about 1 LPM and about 25 LPM, or between about 2 LPM and about 25 LPM, or between about 2 LPM and about 5 LPM, or between about 5 LPM and about 25 LPM, or between about 5 LPM and about 10 LPM, or between about 10 LPM and about 25 LPM, or between about 10 LPM and about 20 LPM, or between about 10 LPM and 15 LPM, or between about 20 LPM and 25 LPM. A high flow therapy apparatus with an adult patient, a neonatal, infant, or child patient, may deliver gases to the patient at a flow rate of between about 1 LPM and about 100 LPM, or at a flow rate in any of the sub-ranges outlined above. The flow therapy apparatus 10 can deliver any concentration of oxygen (e.g., fraction of delivered oxygen, FdO2), up to 100%, at any flowrate between about 1 LPM and about 100 LPM. In some configurations, any of the flowrates can be in combination with oxygen concentrations (FdO2) of about 20%-30%, 21%-30%, 21%-40%, 30%-40%, 40%-50%, 50%-60%, 60%-70%, 70%-80%, 80%-90%, and 90%-100%. In some combinations, the flow rate can be between about 25 LPM and 75 LPM in combination with an oxygen concentration (FdO2) of about 20%-30%, 21%-30%, 21%-40%, 30%-40%, 40%-50%, 50%-60%, 60%-70%, 70%-80%, 80%-90%, and 90%-100%. In some configurations, the flow therapy apparatus 10 may include safety thresholds when operating in manual mode that prevent a user from delivering too much oxygen to the patient.

High flow therapy may be administered to the nares of a user and/or orally and/or via a user's trachea. High flow therapy may deliver gases to a user at a flow rate at or exceeding the intended user's peak inspiratory flow requirements. The high flow therapy may generate a flushing effect in the nasopharynx such that the anatomical dead space of the upper airways is flushed by the high incoming gases flow. This can create a reservoir of fresh gas available for each breath, while minimizing re-breathing of nitrogen and carbon dioxide. Meeting inspiratory demand and flushing the airways is additionally important when trying to control the patient's fraction of inhaled oxygen, FiO2. High flow therapy can be delivered with a non-sealing patient interface such as, for example, a nasal cannula. The nasal cannula may be configured to deliver breathing gases to the nares of a user at a flow rate exceeding the intended user's peak inspiratory flow requirements.

Nasal high flow can provide a level of pressure support to a patient reverse synchrony to the breathing of the patient. For example, nasal high flow being provided to a patient can increase pressure during the expiratory phase of a patient. This can reduce the respiratory rate of the patient and reduce respiratory effort of the patient. Reduced respiratory effort and respiratory rate are helpful to a patient with respiratory conditions e.g. COPD.

The term "non-sealing patient interface" as used herein can refer to an interface providing a pneumatic link between an airway of a patient and a gases flow source (such as from flow generator 15 that does not completely occlude the airway of the patient. Non-sealed pneumatic link can comprise an occlusion of less than about 95% of the airway of the patient. The non-sealed pneumatic link can comprise an occlusion of less than about 90% of the airway of the patient. The non-sealed pneumatic link can comprise an occlusion of between about 40% and about 80% of the airway of the patient. The airway can include one or more of a nare or mouth of the patient. For a nasal cannula the airway is through the nares.

The breathing assistance apparatus can deliver heated and humidified gases to a patient or user. The apparatus may be suitable for treating chronic obstructive pulmonary disease (COPD). The apparatus may be configured to deliver gases to a patient interface at a high flow rate (high flow therapy), particularly nasal high flow therapy.

The breathing assistance apparatus could, for example, be of the type described in WO 2016/207838 or US 2018/0185606.

Alternatively, the features described herein may be used with an apparatus for a different purpose. The apparatus may be a high flow therapy apparatus, or may be a low flow therapy apparatus. For example, the features may be provided in an apparatus for providing continuous positive airway pressure (CPAP), which may deliver gases (humidified or otherwise) at lower flow rates, or may be provided in a medical insufflation apparatus.

The features could be used with a stand-alone humidifier. The standalone humidifier may have a housing, a recess for receipt of the humidification chamber, and a heater plate, but may not have a motor unit. The standalone humidifier may receive gases from an external source.

Accordingly, an alternative form breathing assistance apparatus 1000 may be a standalone humidifier apparatus comprising a base unit defining a main housing and a humidifier.

The standalone humidifier apparatus can deliver heated and humidified gases for various medical procedures, including respiratory therapy, laparoscopy, and the like. These apparatuses can be configured to control temperature and/or humidity. The apparatuses can also include medical circuits comprising various components that can be used to transport heated and/or humidified gases to and/or from patients. For example, in some breathing circuits, gases inhaled by a patient are delivered from a heater-humidifier through an inspiratory tube or conduit. As another example, tubes can deliver humidified gas (commonly CO2) into the abdominal cavity in insufflation circuits. This can help prevent desiccation or 'drying out' of the patient's internal organs, and can decrease the amount of time needed for recovery from surgery. Heater wires may extend inside of at least a portion of the tubing forming the circuit to prevent or at least reduce the likelihood of the formation of significant condensation.

A standalone humidifier apparatus would typically include a base unit and a humidification chamber. The base unit can comprise a heater plate. The humidification chamber can be configured to hold a volume of a liquid, such as water. The heater plate can be configured to heat the volume of liquid held within the humidification chamber to produce vapour.

The humidification chamber is removable from the base unit to allow the humidification chamber to be more readily sterilized or disposed, or to re-fill the chamber with liquid. The body of the humidification chamber can be formed from a non-conductive glass or plastics material but the humidification chamber can also include conductive components. For instance, the humidification chamber can include a highly heat-conductive base (for example, an aluminum base) contacting or associated with the heater plate on the heater base.

The base unit can also include electronic controls such as a master controller. In response to user-set humidity or temperature values input via a user interface and other inputs, the master controller determines when (or to what level) to energize the heater plate to heat the liquid within the humidification chamber.

The standalone humidifier apparatus can include a flow generator to deliver gases to the humidification chamber. In some configurations, the flow generator can comprise a ventilator, blower, or any other suitable source of pressurized gases suitable for breathing or use in medical procedures. The flow generator may be positioned in the base unit.

Alternatively, the standalone humidifier apparatus may comprise just the base unit and the humidification chamber, and may be used with a separate or remote flow generator. The base unit may be configured to fluidly connect to the separate or remote flow generator.

Therefore, the flow generator that is used with a standalone humidifier apparatus may be a wall gases source, ventilator, blower, or gas tank for example.

A standalone humidifier apparatus can be used with breathing therapies, positive pressure apparatus, non-invasive ventilation, surgical procedures including but not limited to laparoscopy, and the like. Desirably, the humidifier apparatus can be adapted to supply humidity or vapour to a supply of gases. The humidifier apparatus can be used with continuous, variable, or bi-level PAP systems or other form of respiratory therapy. In some configurations, the humidifier apparatus can be integrated into a system that delivers any such types of therapy.

An exemplary standalone humidifier apparatus is described in WO 2015/038013 or US patent number 10,245,407.

The standalone humidifier apparatus may have any one or more of the features described or shown herein.

Connectors are described herein for connecting a first respiratory system component to a second respiratory system component. For example, the connectors may connect a filter or a respiratory conduit end cap to a complementary connector component. The connectors described herein could be coupled to or formed with the first respiratory system components. When a connector is formed with the first respiratory system component, the connector is an integral part of the first respiratory system component and therefore could be considered to be the first respiratory system component. The connectors could be used as, or in, an adapter.

In some configurations, the connector 700, 1700 could be provided in a patient interface assembly comprising: a patient interface; a conduit 603; and the connector 700, 1700 at a terminal end of the conduit 603. The connector is for engagement with a complementary connector component 800, 1800. The connector could be either of the connectors 700, 1700 described herein and/or could have any one or more of the features or functionality described in relation to those connectors. The connector 700, 1700 comprises: a body 701, 1701 defining a bore 703, 1703 for receipt of the complementary connector component 800, 1800, the bore 703, 1703 having a terminal end 704, 1704 that provides an entry into the bore for the complementary connector component 800, 1800 and a sealing region 705, 1705 for engaging with a seal 841, 1841 on the complementary connector component; and one or more retaining features to assist with retaining the complementary connector component 800, 1800 in engagement with the connector 700, 1700.

In some configurations, the connector 700, 1700 is coupled to or is formed with the conduit 603.

In some configurations, the terminal end of the conduit comprises a filter 501, 1501, and the connector 700, 1700 is coupled to or is formed with the filter 501, 1501.

In some configurations, the one or more retaining features is/are in the bore 703, 1703.

In some configurations, the one or more retaining features comprise one or more retaining protrusions 710, 1710 that extend into the bore 703, 1703 from an inner wall 702, 1702 of the body 701, 1701.

In some configurations, the one or more retaining protrusions comprise diametrically opposed retaining protrusions 710, 1710.

In some configurations, the sealing region 705, 1705 is closer to the terminal end 704, 1704 relative to the retaining protrusion(s) 710, 1710.

In some configurations, the connector 700, 1700 comprises a plurality of the retaining protrusions 710, 1710.

In some configurations, the retaining protrusions 710, 1710 are configured for engaging with engagement features 810, 1810 on male engagement fingers 820, 1820 of the complementary connector component 800, 1800.

In some configurations, the connector 700, 1700 comprises at least one internal alignment feature 720, 1720 and/or at least one external alignment feature 761, 1761.

In some configurations, the patient interface comprises a nasal cannula. In some configurations, the patient interface comprises a non-sealing patient interface. In alternative configurations, the patient interface comprises one of the alternative arrangements described herein for delivering gases to a patient.

In alternative configurations, the patient interface assembly may comprise one of the other types of connectors 8700, 9700, 10700, 11700 disclosed herein.

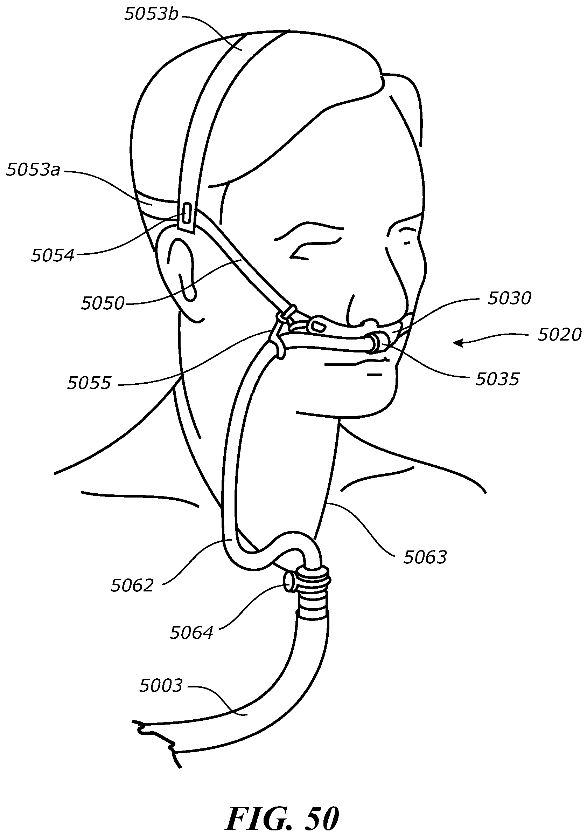

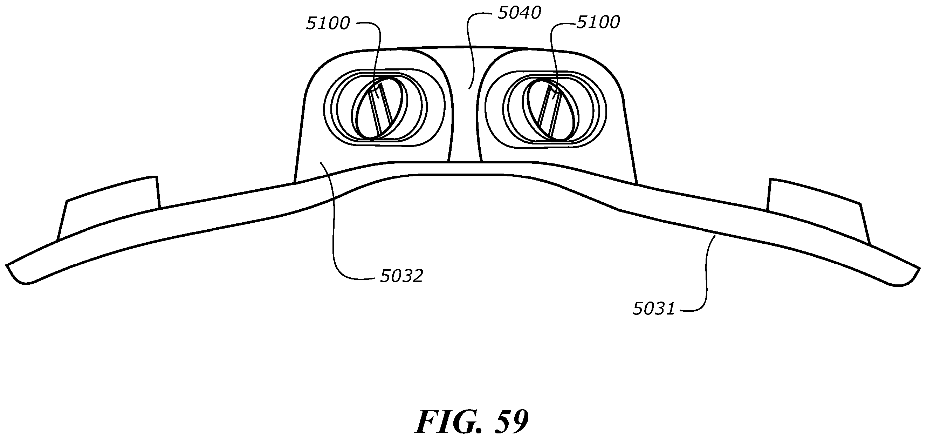

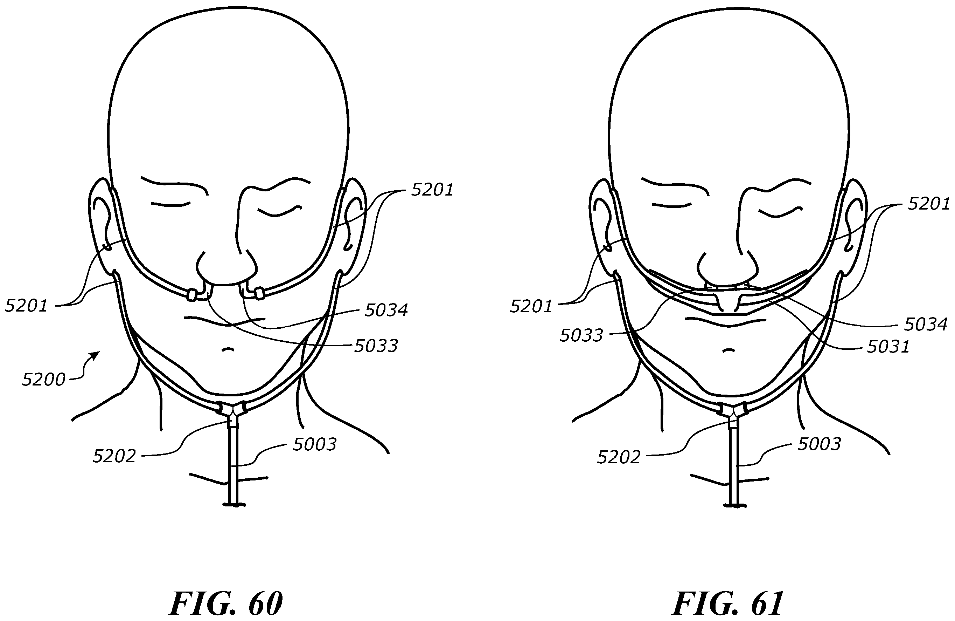

Figures 50 to 61 show an exemplary patient interface 5020 comprising a nasal cannula that can be used in the respiratory therapy systems described herein and that can be provided in a respiratory therapy kit with at least a filter as described herein.

Figure 50 shows the first form of the patient interface 5020. The patient interface 5020 broadly consists of a head securement mechanism and a nasal cannula arrangement 5030, and also includes a gases inlet conduit or secondary supply conduit 5062. The head securement mechanism enables a user to place and maintain the nasal cannula arrangement 5030 in the correct operational position. The gases inlet conduit or secondary supply conduit 5062 forms a fluid or gases connection between the outlet end of the main delivery conduit 5003 and the nasal cannula arrangement 5030 to allow fluids or gases to flow between the main delivery conduit and nasal cannula arrangement. The secondary supply conduit 5062 and detail of the main portion of the nasal cannula arrangement 5030 will be described in detail below.

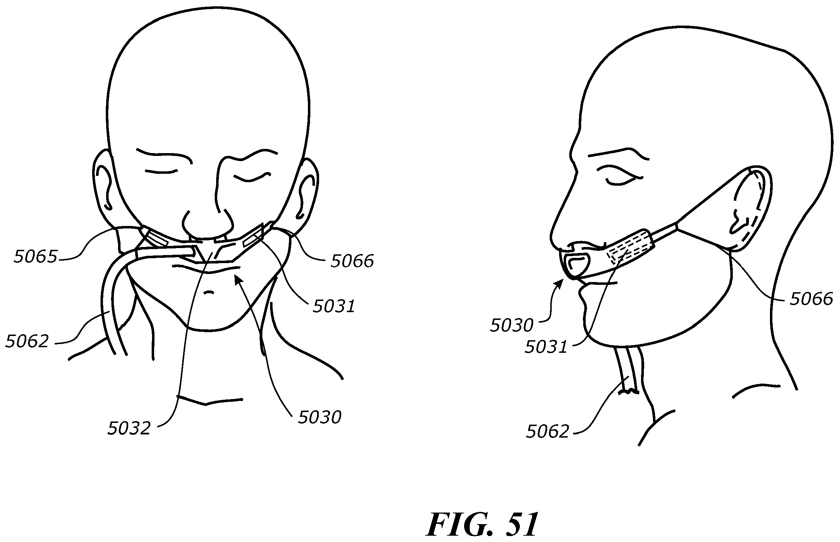

Alternative forms of head securement mechanism, which form part of the patient interface 5020, will be described with particular reference to Figures 50 and 51. Figure 50 shows one form of the head securement mechanism, while Figure 51 shows an alternate form of the head securement mechanism.

The patient interface 5020 is secured to the patient's head or face by a front strap 5050 and rear and top straps 5053a and 5053b, as shown in Figure 50. The front strap 5050 in use connects with the nasal cannula arrangement 5030 and the rear strap 5053a and the top strap 5053b connect with the front strap 5050, the rear strap 5053a wrapping around the top and back of the patient's head in use. The head strap device may be adjustable to allow patients of different sizes and head shapes to use the nasal cannula arrangement 5030. For example, an adjustment buckle 5054 could be included which allows a patient to loosen or tighten the head strap 5053.

Alternatively the patient interface is secured to the patient's head and face by a front strap 5050 and a single rear strap 5053a attached to the front strap 5050. The rear strap may be attached to the front strap 5050 via a buckle 5054 or alternatively the rear strap 5053a may be integral with the front strap 5050. The buckle 5054 allows a patient to loosen or tighten the front and/or rear straps in order to achieve a comfortable fit. Alternatively the integral front and rear straps are elastic and can be stretched over a patients head. The elasticity of the straps exerts a force upon the head to hold the nasal cannula in the optimal position when in use. Elastic straps 5050, 5053a can be used with the adjustment buckle 5054 or the elastic straps 5050, 5053a may be used on their own without the buckle 5054.

The head strap arrangement may also include a loop 5055 which holds and supports the interface tube or secondary supply conduit/gases inlet conduit 5062 at or close to the inlet end (described in detail below).

A neck tie or lanyard 5063 may also be provided with the patient interface 5020. Figure 50 shows an example of a neck tie or lanyard 5063. In one form the neck tie or lanyard 5063 is connected to the gases inlet conduit 5062 or at a location at or close to the connection between the delivery conduit 5003 and the gases inlet conduit 5062, supporting the weight of the delivery conduit 5003 and the gases inlet conduit 5062 in use.

A toggle 5064 is provided with the neck tie 5063 to allow adjustment of the neck tie's length. The toggle 5064 makes the neck tie 5063 suitable for any sized patient to use the patient interface 5020. The neck tie 5063 supports the weight of delivery conduit 5003 in use, such that the weight does not act on the user or the nasal cannula arrangement 5030. The use of the neck tie 5063 prevents the combined weight of the delivery conduit 5003 and the gases inlet conduit 5062 from pulling on the nasal cannula arrangement 5030, helping to prevent the nasal prongs 5033, 5034 from interfering with the sensitive lining of the nasal passages, or becoming displaced or disoriented in use. One configuration of neck tie or lanyard 5063 is loose fitting around the neck so as to prevent strangulation of the user. The lanyard 5063 also provides a convenient way of supporting the delivery conduit 5003 and the gases inlet conduit 5062. This allows the patient to turn in bed without tugging or pulling on the conduit 5003 and helps avoid having the gases inlet conduit 5062 from overheating under the blankets.

In one form the neck tie or lanyard 5063 has a clip that allows the lanyard to be opened and closed by a user in order place and secure the neck tie 5063 around a user's neck. The clip comprises a male and female connector that snap fit together. The clip is removed by pulling one end of the neck tie 5063. The clip is easily removable and "breaks away" undoing the clip when the user pulls on one side of the neck tie. This allows the neck tie 5063 to be removed quickly in an emergency situation.

An alternate configuration of the head securement mechanism is shown in Figure 51. The nasal cannula arrangement 5030 is secured to a patient's head with the aid of over the ear loops 5066. The loops 5066 are configured to hang over a patient's ears to support the weight of the cannula arrangement 5030. The loops are attached to the nasal cannula arrangement by the side straps 5031 (described below) of the nasal cannula arrangement. The ear loops are horizontally slideable relative to the straps 5031. The horizontal movement capability allows a user to adjust the tightness of the ear loops to ensure the nasal cannula arrangement fits comfortably and correctly upon a user's face. The loops 5066 bear the weight of the nasal cannula arrangement 5030, such that the user's nasal passages are not put under undue stress because of the weight of the nasal cannula arrangement 5030. The loops make the cannula arrangement 5030 more comfortable to wear.

The interface tube or secondary supply conduit 5062 will now be described in detail. The secondary supply conduit 5062 is a short length of conduit or tubing which runs between the outlet of the main delivery conduit 5003 and the nasal cannula arrangement 5030. In use, gases exit the main delivery conduit 5003 and enter the secondary supply conduit 5062, travelling along the secondary supply conduit 5062 to the patient. One reason that secondary conduits such as the secondary supply conduit 5062 are used is as follows: the main delivery conduit 5003 is relatively heavy and cumbersome as it is used to transport gases over a reasonably long distance (from the humidifier unit to a point close to the patient). The main delivery conduit 5003 is therefore required to have a wall that is strong enough to support its own weight without collapsing. As the main delivery conduit 5003 is therefore relatively long (e.g. 8 to 10 feet), this additional length and the thicker wall structure adds to the weight of the main delivery conduit 5003. If the outlet of the main delivery conduit 5003 is connected directly to the patient interface in such a manner that the user 5002 is required to support this weight, this can cause discomfort to the user due to the weight of main delivery conduit acting on the user. A lighter, shorter secondary conduit (e.g. secondary supply conduit 5062) running between the outlet of the main delivery conduit 5003 and the patient interface 5020 is used. Secondary supply conduit 5062 is lighter and shorter than the main delivery conduit 5003, and as outlined above, is generally used with e.g. a neck tie or lanyard 5063 connected to the secondary supply conduit 5062 or to the connection between the main delivery conduit 5003 and the secondary supply conduit 5062, to support the weight of the main delivery conduit 3 and the secondary supply conduit 5062 in use.

The connection between the outlet of the main delivery conduit 5003 and the inlet of the secondary supply conduit 5063 is placed near to the patient to reduce torsion or pulling on the nasal cannula arrangement 5030 and reduce possible heat problems or over heating close to the patient due to the heating element provided in the main delivery conduit 5003. In order to reduce condensate forming in the unheated secondary supply conduit 5062, a conduit that has vapour transmission properties can be provided.

The secondary supply conduit 5062 can be integrally formed with the main delivery conduit 5003 or may be attached by some connection mechanism, allowing for detachment of the secondary supply conduit 5062 from the main delivery conduit 5003. The connection mechanism can be a threaded screw type connector or a friction locking mechanism.

Although not shown in these figures, the filter 501, 1501 described herein may be provided between the main delivery conduit 5003 and the secondary supply conduit 5062. The end of the main delivery conduit 5003 may comprise the complementary connector component 800, 1800 for connecting to the connector 700, 1700 of the filter 501, 1501.

By providing the filter 501, 1501 in that position, a small form factor filter can be used.

The secondary supply conduit may be made from a breathable material that allows water vapour to pass through the supply conduit and into ambient air while substantially preventing liquid water or breathing gases to pass out of the supply conduit. The supply conduit may have regions of breathable material along its length or alternatively the entire conduit wall may be breathable. Materials may be breathable due to their composition, physical structure or a combination thereof. The mechanisms of water vapour transmission through these breathable materials are numerous and known in the art. The purpose of the breathable region or regions of the supply conduit wall is to allow passage of water vapour from the gases path independent of specific drain locations. This reduces the build-up of condensation within the breathing tube by drying the humidified breathing gases (by transmitting water vapour to the surrounding ambient air) during their flow through the breathing tube. An example of such a material is SYMPATEXTM or GORETEX^{™} or NAFION^{™} and so on.

The result of providing a short secondary supply conduit 5062 is that a majority of humidity in the gases is transported to the patient, and there is an insignificant and negligible loss of humidity through the breathable wall of the short secondary supply conduit, while condensate forming is reduced.

The nasal cannula and its various features will now be described in more detail.

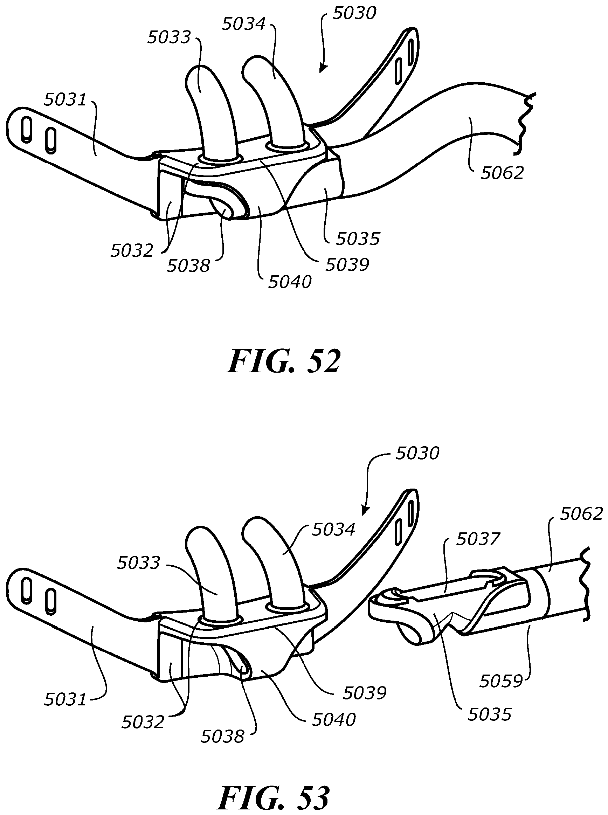

One form of the nasal cannula 5030 which forms part of the patient interface 5020 shall now be described in more detail with particular reference to Figures 52, 53, 54, 55, 56, 57, and 58.

The nasal cannula 5030 of the preferred form comprises two main parts: a manifold portion 5035 and a face mount part 5032. When assembled, the manifold portion 5035 and face mount part 5032 form a manifold of the nasal cannula 5030. Some configurations of these two parts will now be described with particular reference to Figures 52 and 53.

In one form, the manifold portion 5035 is in use connected to and in fluid communication with the secondary supply conduit 5062 as has been described above. However, it could be connected directly to the main delivery conduit 5003 in alternative configurations. Where the phrase "gases inlet manifold part" is used in this specification, this should be taken to mean the manifold portion 5035 in combination with the secondary supply conduit 5062, or just the manifold portion 5035, as appropriate.

It should also be noted that the form as shown in Figures 52 and 53 shows the manifold portion 5035 as being detachable from the remainder of the nasal cannula 5030. However, the manifold portion 5035 could also be formed as an integral part of the nasal cannula 5030 if preferred or required - that is, so that the manifold portion 5035 and the face mount part 5032 (described below) are one item.

One form of manifold part 5035 is generally tubular in shape having a substantially circular inlet 5059 on one side that curves to an elongated oval outlet 5037, the outlet 5037 being formed on one side of the manifold portion 5035 so that it is perpendicular to the inlet 5059. The circular inlet 5059 in the preferred form receives the patient end of the secondary supply conduit 5062, such that the gases from the secondary supply conduit 5062 can flow through the manifold portion 5035 (inlet 5059 could alternatively be oval, or any other suitable shape - it does not have to be circular). In the form shown the manifold portion 5035 is integrated with the secondary supply conduit 5062 (i.e. it is not intended to be removed and replaced repeatedly in use, although it can be removed if required), but alternatively the manifold portion 5035 could be removably attached to the secondary supply conduit 5062. The manifold portion 5035 engages with the face mount part 5032 so that gases can pass through the outlet 5037 and transfer from the secondary supply conduit 5062 to the patient 5002 through the nasal prongs 5033, 5034 (described in detail below).

In one form the manifold portion 5035 is manufactured from a hard plastic material that only deforms under relatively high loading conditions (that is, it cannot easily be crushed in the hand of a user). The manifold portion 5035 may be moulded, injection moulded, machined or cast.

The manifold portion 5035 in use is connected to the face mount part 5032, so that gases exiting the manifold portion 5035 enter the face mount part 5032. The term "connected" in the context of this specification should be taken to mean either "detachable" or "integral with", as appropriate. The face mount part will now be described in detail.

The face mount part 5032 includes the nasal prongs 5033, 5034, so gases passing through the face mount part 5032 can enter the nasal prongs 5033, 5034 and be delivered to the patient. In one form the nasal prongs 5033, 5034 extend parallel to each other, curving upwards and inwards from the face mount portion 5032. In one form, each nasal prong is equidistant from the centre of the face mount part. The structure of the prongs 5033, 5034 will be described in detail below.

The face mount part 5032 includes side straps 5031 and an open tubular recess 5038, integrally moulded together as shown in Figures 52 and 53. The open tubular recess 5038 extends below the face mount part 5032 and is adapted to receive the manifold portion 5035 (for one configuration where the face mount part 5032 and the manifold portion 5035 are separable items). The face mount part 5032 has a lip 5039 that extends around the upper edge of the open tubular recess 5038. The manifold portion 5035 is connected to the face mount part 5032 by a friction fit and the lip 5039 on the face mount part 5032 helps to grip the manifold portion 5035 and form a strong sealed connection between the manifold portion 5035 and the face mount part 5032. The open tubular recess 5038 is divided by a rib 5040 which extends below the face mount part 5032. The rib 5040 helps to cradle and hold the manifold portion 5035 in the correct position as it engages with the face mount part 5032, the rib 5038 extending around the outside of the manifold portion 5035. Outlet 5037 on the manifold portion 5035 aligns in use with the underside of the face mount 5032 portion when the manifold portion 5035 is connected to the face mount part 5032. This alignment minimises and reduces the amount of gases which leak out of the nasal cannula arrangement 5030, allowing effective treatment of the user by delivering maximum amount of humidified gases.

The side straps 5031 are used to attach the headgear strap 5050 or the ear loops to the face mount part 5032. The side straps 5031 comprise a pair of straps (shown as straps 5031 on the figures) which extend from either side of the face mount part 5032, and which in one form are formed as an integral part of the face mount part 5032. The headgear strap 5050 is in use attached to the side straps 5031 so that the patient interface can be worn by a user in use. In some configurations, the ends of the headgear strap 5050 are looped through a pair of slits on the side straps 5031, with the ends including Velcro or similar to hold the ends in place when the y are looped back on themselves. Alternatively the headgear strap 5050 or loops 5066 may be clipped onto the side straps 5031, for example by way of co-operating male-female clips, or adhesively attached to the side straps 5031.

In some configurations, the face mount part, nasal prongs, side straps and the open tubular recess are all manufactured as one continuous item.

In some configurations, the face mount part 5032, nasal prongs 5033, 5034, side straps 5031 and/or the open tubular recess 38 may be manufactured out of flexible polymer material or silicone, preferably a soft thermoplastic elastomer (TPE).

The nasal prongs will now be described in more detail.

The following is a description of the nasal prongs. In the following description the term "rear", or "back" or any such synonym refers to that part of the structure that faces towards and is closest to the patients face when the nasal cannula is in use. The term "front" or "forward" or any such synonym refers to the side, face or part which faces away from and is furthest away from the face of a user of patient in use. The term "top" or "upper" refers to the side, face or part that is pointing away from the floor, when a user or patient wearing the interface is standing or sitting upright and looking forward. The term "bottom" or "lower" refers to the side, face or part that is directed or pointing toward the ground, again when a user or patient wearing the interface is standing or sitting upright and looking forward.

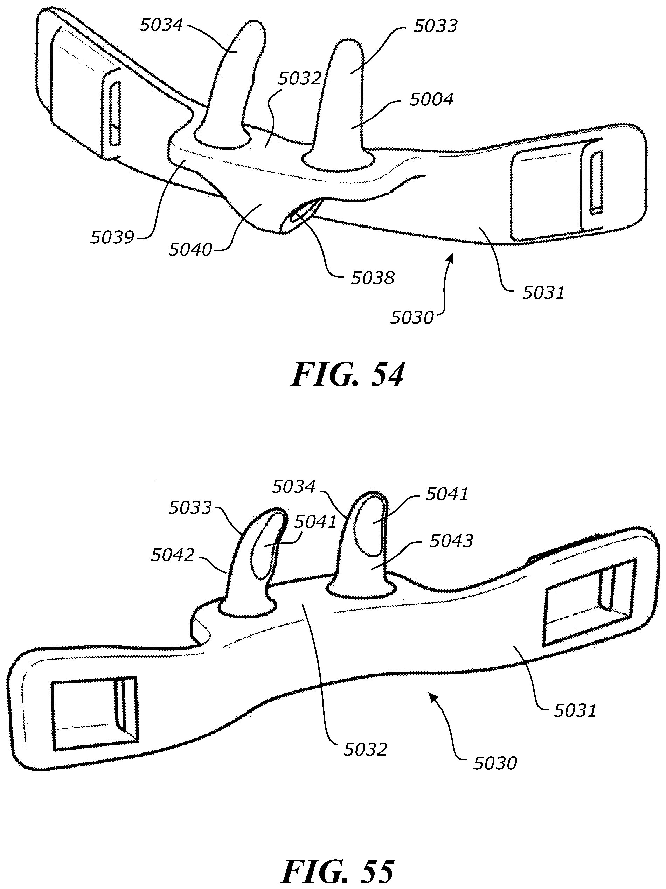

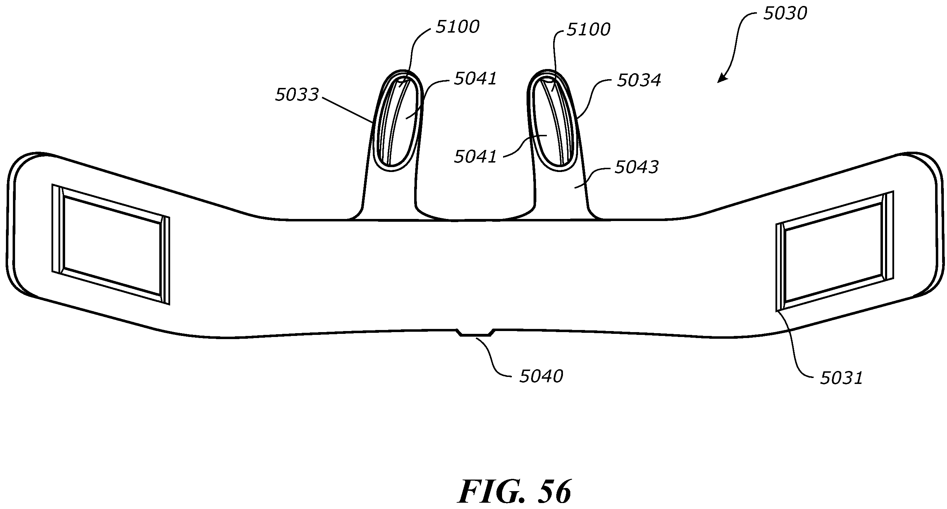

In some configurations the face mount part 5032 includes two nasal prongs 5033, 5034 extending upwards and curving inwards from the upper surface of the face mount part 5032 as shown in Figures 52-55. Referring to Figures 55 and 56, the nasal prongs 5033, 5034 extend from the upper surface of the face mount part 5032 and each prong is placed in each nostril of the patient when the nasal cannula arrangement is in use. The prongs 5033, 5034 are configured to deliver gases to a patient. The prongs 5033, 5034 receive humidified gases from the delivery conduit 5003 via the secondary supply conduit 5062, the manifold portion 5035 and the face mount part 5032. It should be noted that in some configurations, the gases inlet manifold portion 5035 receives the gases from the secondary supply conduit 5062, with the gases passing through the gases inlet manifold part to the face mount part 5032 and then into the nasal prongs 5033, 5034. The nasal prongs 5033, 5034 are therefore in fluid connection with the gases inlet manifold portion 5035 and receive the gases from the secondary supply conduit 5062.

As has already been outlined above, the gases inlet manifold portion 5035 and the face mount part 5032 could be formed as one item - that is, as a combined manifold and face mount part, and this item could if required be formed to act as a manifold, with the prongs integrally formed with the manifold, the manifold attaching to one or more gas hoses or tubes, in a similar manner to typical nasal cannulae which are known in the prior art. Where the phrase "gases inlet manifold part" is used in this specification, it should be taken as being broad enough to encompass this arrangement. The phrase should also be taken as being broad enough to be inclusive of dual hoses of the type known in the prior art that connect one to each side or end of the manifold tubing and which loop over the ears of a user before attaching to a main delivery conduit or a secondary supply conduit. It should also be noted that where the phrase "a gases inlet manifold part adapted to form a fluid connection with a delivery conduit" is used, this should be taken to mean that the gases inlet manifold part may be directly connected, or indirectly connected with intervening items included such as a secondary supply conduit, or dual hoses of the type known in the prior art (or both).

In some configurations the nasal prongs 5033, 5034 are generally tubular in shape, with an upwards and rearwards curve. The nasal prongs curve upwards and towards the back of the patient's head when in use. Preferably the prongs are curved toward the back of the patient's nasal passages, such that the stream of gases delivered by the prongs is directed toward the back of the patient's nasal passages. The curvature of the nasal prongs 5033, 5034 ensures the prongs follow the natural curve of a human's nasal passage. The prongs may follow a curve of radius 10.5mm but any radius between 5 and 20mm is suitable, and larger or smaller sizes are also possible. The curvature of the prongs 5033, 5034 ensures gases are delivered into the nasal cavity of the patient and this helps to reduce leakage of gases from the nasal cavity. The curvature of the prongs 5033, 5034 provides the advantages of added comfort and effective delivery of respiratory gases into a patient's nasal cavity.

In some configurations the nasal prongs 5033, 5034 fit into the patient's nasal passage. Preferably each of the nasal prongs are generally circular in cross section. Alternatively the nasal prongs may be triangular or oval in cross section. A circular cross-section is most advantageous for use since this shape conforms most closely to the shape of a human's nasal passage, thus providing a comfortable fit for the patient and ensuring the correct delivery of the therapy. However, the nostrils and nasal cavities of users are not perfectly circular or geometrically standard, and other cross-sections (such as the triangular or oval cross sections mentioned above) may be preferable.

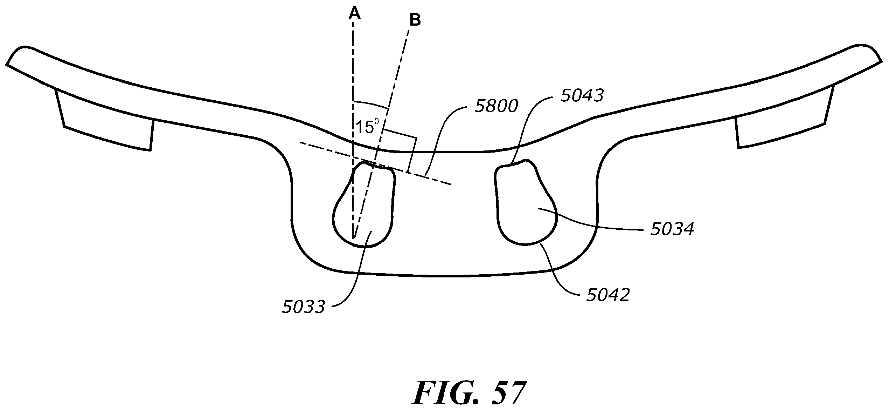

In one form the nasal prongs are arranged equidistant from the centre of the face mount part. The nasal prongs may be angled to face slightly inward towards one another as best shown by Figure 57. When viewed from the top, the centre of each of the nasal prongs is preferably angled 15 degrees inward from the vertical line A as seen on Figure 57. That is, the angle X between line A and line B, as shown on Figure 57, is 15 degrees. This applies to both of the pair. The line A defines a vertical plane, which is substantially parallel to the vertical plane of symmetry which bisects the face mount part 5032 of the nasal cannula 5030 - that is, a line or plane which would bisect the human nose when the nasal cannula is positioned on the face of a user. The prongs 5033, 5034 are angled inward towards one another at 15 degrees to provide the most comfortable fit when in use. It has been found that having the nasal prongs angled inwards at 15 degrees provides the most comfortable fit or position for a user and an optimal position for delivering therapeutic gases to a patient. The nasal prongs may be placed at any other angle larger or smaller than 15 degrees. The range of angles between line A and line B could for example be between 0 degrees to 60 degrees of inward angle. Alternatively the nasal prongs could be angled outward from the vertical line A. Angling the nasal prongs outward is not preferred because angling the prongs outwards means the prongs may not follow the natural shape of the nasal passage potentially making the prongs uncomfortable to use for most users. However, this may be suitable in some situations, or for some users in certain circumstances.

Each of the nasal prongs includes a gases exit cut-out section 5041 on the rear side 5043 of the nasal prong, as shown in figures 55, 56, and 58. The gases exit cut-out or cut-out section gives each of the prongs the appearance of a scoop. The front side 5042 of the nasal prong (the side further away from the patient) extends further upwards and inwards from the face mount part 5032, and forms a guide wall that guides humidified gases into the patient's nasal passage when the nasal prongs 5033, 5034 are in use. The gases exit cut-out in the prong has a sectional area greater than the cross-sectional area of the prong at or close to the point of entry of gases into the prong from the manifold section - that is, the cross-sectional area of the prong is greater at the point where the gases exit the prong (and enter the users nare), in comparison to a point at or close to where the gases enter the prong from the manifold section.

The cut-out section 5041 can be formed in various shapes. In some configurations, the cut-out section 5041 is oval in shape when viewed from the rear, as best seen in Figure 56. That is, when viewed from the rear, the perimeter of the cut-out section 5041 describes an oval shape, the top of the oval angled slightly inwards towards the other nasal prong. The cut-out section could also be triangular (with one point of the triangle oriented towards the base of the prong, and the other two corners at the topmost inner edges of the cut-out section 5041). The cut-out could also be rectangular in shape.

The cut-out can extend from various positions along the nasal prong. Preferably the cut-out section 5041 extends from between halfway and two thirds of the way along the nasal prong, when measured from the top tip of the nasal prong. Alternatively the cut-out section 5041 may extend from less than halfway along the nasal prong, when measured from the top tip of the nasal prong. As a further alternative the cut-out may extend the entire length of the prong. In some configurations,, the cut-out section 5041 extends from between halfway and two-thirds of the way along the nasal prong to provide the best advantages. It has been found that having the cut-out extending between halfway and two thirds ensures the optimal size of the opening. Placing the cut-out at this position ensures the most optimal size of cut-out to provide the advantages described later in this specification. For nasal prongs according to some configurations, this corresponds to a cut-out having a height of 3mm to 15mm. However, the size of the cut-out could fall outside this range if required for alternative forms.

The cut out 41 may be formed during the moulding process. In some configurations, the prongs are moulded by injection moulding, casting or vacuum forming. The mould used to produce the desired prong shape has the cut-out feature built into it.

In alternative configurations the cut-out section 5041 is created by cutting across the rear 5043 of each of the prongs 5033, 5034 after these have been formed in an initial forming operation - e.g. after the face mount part 5032 has been moulded in an initial forming operation, the cut-out is formed by removing material either by machining or by hand.

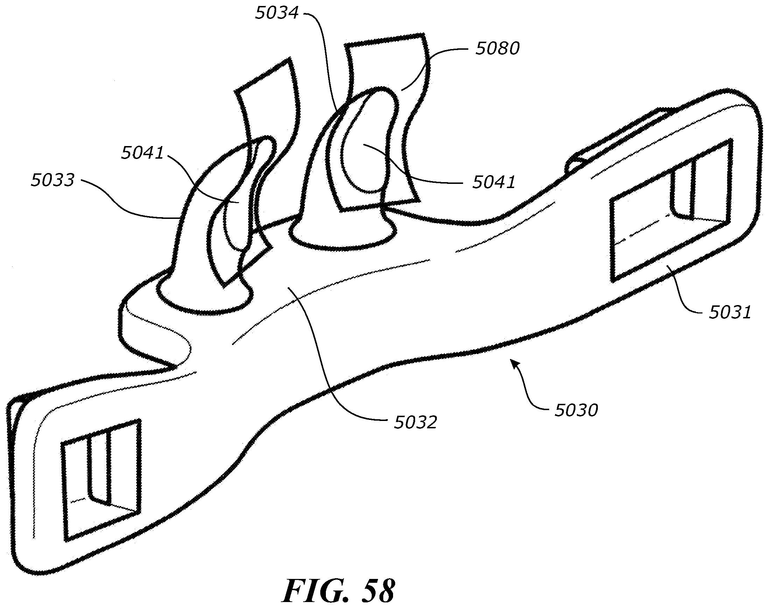

In some configurations, a reverse S-shaped surface 5080 may define the cut-out section, as shown in Figure 58. The bottom edge of the surface 5080 is shown in Figure 57 as line 5800. As can be seen, for each prong, line 5800 is perpendicular to a line through the centre of the nasal prong, and is aligned with the rear edge of the prong. This is best shown in Figure 57. The surface 5080 may be shaped as a reverse S shape as shown in Figure 58. The surface 5080 extends a certain distance inward to form the preferred 'scoop' shape of the gases exit cut-out. The reverse S shape is aligned substantially vertically. The reverse S shaped surface produces the most preferred size and shaped cut-out. After forming, the edges or perimeter of the cut-out section 5041 conforms to the surface of the reverse S-shaped surface, as shown in Figure 58. Such a surface results in the optimal cut-out shape that provides the advantages described below.

In some configurations the rear wall 5043 of the nasal prongs 5033, 5034 may also include a reinforcing feature (not shown in the figures) that extends upward along the length of the rear wall of the nasal prong. The reinforcing feature helps to maintains the rear wall 5043 of the nasal prong in an upright position. The reinforcing feature may be formed as a ridge running at least part of the way along the rear face 5043 of the nasal prongs 5033, 5034. This ridge can be on either the inside or the outside of the rear wall 5043, as the rear wall does not in use generally contact the upper lip or nares of a user, and the reinforcing feature will therefore tend not to interfere with the face of the user and make them uncomfortable. Alternative forms of reinforcing feature may be provided will be described in greater detail below.

Due to the curvature and shape of the nasal prongs 5033, 5034, the stream of air will tend to flow along the front wall of the prongs 5033, 5034, rather than the rear wall 5043 - the air stream flows along the outside of the bend rather than the inside.

Advantages of the described nasal interface 5020 comprising nasal cannula 5030.

In at least some configurations of the cut-out sections, the cut-out sections within each nasal prong provide a number of advantages. The main advantages are as follows:
1) Each of the prongs can deform or misshape more easily, as they have less structural rigidity (a piece of their support structure is missing, so they can deform more easily), and are therefore more comfortable in a patient's nasal passage,
2) The gases do not exit from the prong as a jet, through a small aperture. The cut-out provides a larger area of exit aperture at the exit of the prongs, so that the velocity or air speed of the gases is reduced at the point where they exit the prong(s). That is, the size of the exit aperture (defined by the edge or perimeter of the cut-out section) is greater than the size or cross-sectional area of the inlet aperture, which is defined by the base of the prong where it is connected to the face mount part 5032. The air speed of the gases reduces as the area increases. That is, each prong is shaped so that the velocity of gases exiting said prong is reduced in comparison to the velocity of gases at or close to the gases point of entry to the prong. This allows a proportionally greater volume of gases to be delivered to a patient without causing discomfort (in comparison to a cannula prong which does not include a cut-out). With the cut-out cannula, air jetting effects are reduced. The jetting of the airflow is reduced based on the continuity equation for energy or mass conservation, which states that increasing the cross sectional area equates to a reduction in the velocity of the airflow. A jet of gas delivered into a user's nasal passage can irritate or potentially damage the tissue within the nasal passage. A reduction in the velocity of the flow of gases as delivered by the nasal prongs reduces irritation in the user's nostrils and thus the jetting effects. It also follows from the continuity equation that the larger the aperture a gas is flowing through, the larger the amount of diffusion.
3) The stream of gases is directed in a generally rearwards direction (relative to the head of a user) relative to the nasal passage of a user.

These advantages are discussed in more detail below.

The nasal cannula arrangement 5030 as shown in Figures 55 and 56 is suitable for the delivery of high airflow, high humidity gas flow to the patient's nasal cavity. In some configurations, the cut-out extends between from the top of the prong to between half and two thirds of the distance to provide the largest cut-out. Further, the shape of the cut-out (the optional reverse S-shape surface as described above) contributes to ensuring maximum diffusion and reduction of air jetting effects.

In prior art cannulas, the cannula prongs will generally have an exit aperture which is substantially the same size as their inlet aperture (e.g. where the base of the prong is connected to a manifold). In the cannula described above, the size and shape of the cut-out helps to reduce the air speed at the point of exit from the prong, and to direct the gases in a generally rearwards direction. It has been found that this helps to increase user comfort and compliance with a therapy regime to a surprising degree. Furthermore, the decreased velocity flow of respiratory gases from the cut-out 5041 of the nasal prongs 5033, 5034 helps to ensure that the user will breathe as normally as possible.

The reduction in air velocity due to the cut-outs in the prongs 5033, 5034 allows the use of a higher flow rate than is generally the case in the prior art. In therapy, high flow rates are preferred in order to meet the patients requirements. Using high flow rate ensures that where possible, the entire volume of an inhaled breath comprises respiratory gases. However, due to increased patient discomfort and potentially dangerous side-effects with higher flow rates, a trade-off is normally made between patient comfort/safety, and flow rate. Lower flow rates than may be optimal are used to ensure the patient is comfortable enough to conform with a therapy regime. Using these lower flow rate means at least part of, and generally a majority of, the user's breath is composed of ambient air which can be detrimental to the therapy provided by medical gases. Using relatively higher flow rates and having nasal prongs that allow humidified medical gases to be delivered at high flow rates is advantageous. This helps to ensure that the most efficient and effective therapy provided to a patient. Surprisingly, it has been found that by using the prongs as described above - i.e. prongs that include a cut-out section - flow rates between (but not limited to) just above 0L/min to 80L/min can be delivered to a user and initial user feedback suggests that there is decreased discomfort and a greater tendency towards regime compliance. The prongs can be re-sized - e.g. for use in neonatal applications - with the flow rates or flow range being considerably lower in neonatal applications. It is anticipated that flow rates of up to 120L/min could be used in certain circumstances. However, it is anticipated that the preferred range will be in the order of 20-90L/min for adults, 5-30L/min for Paediatric patients, and just over 0L/min to 8L/min for Neonatal patients. The cut-out design is effective at low flow rates when used on neonatal patients (as small as 400gms), where flow rates of 1-8L/min would otherwise create very high velocities due to the small size of the cannula and patient.

The cut-out sections 5041 in the nasal prongs 5033, 5034 causes the nasal prongs 5033, 5034 to be more deformable than prior art nasal prongs which do not include cut-outs. Surprisingly, it has been found that the addition of cut-outs does not significantly negatively impact on the gases delivery efficiency, and as well as the advantages outlined above, allows the nasal prongs 5033, 5034 to be bent and flexed to a greater extent than prior art cannula prongs, to fit comfortably into a patient's nasal passage. A range of sizes of cannulae will normally be used, to ensure a fitment range for all users. However, within each 'bracket' or range, the greater bending or flexibility helps improve user comfort. The cut-out 5041 causes the nasal prongs 5033, 5034 to be more flexible than completely "tubular" or round shaped nasal prongs. Generally in use the nasal prongs rest against the nasal mucosa. In other nasal cannula arrangements the nasal prongs exert a force on the nasal mucosa and this pressure can irritate the user, making wearing nasal prongs uncomfortable. This may even result in damage to the delicate nasal tissue. The gases exit cut-outs 5041 within the nasal prongs allow the prongs 5033, 5034 a greater degree of flexibility within the nasal passage, as the prong pushes against the nasal mucosa tissue. The flexing of the prongs reduces the pressure exerted on the nasal mucosa making it more comfortable and potentially safer for the user to wear.

The cut out sections 5041 within the cannula are also advantageous because they have made manufacturing of the cannulas quicker. The cut out sections 5041 allow the cannula to be easily lifted off the forming tool by a robot or human operator. The cut-out sections 5041 have halved the cycle time.

In some configurations, the nasal prongs may include a reinforcing feature 5100 running along the inner surface of the front wall of the nasal prong helps the nasal prong to return to its original shape after bending and flexing. This is shown in Figure 56. The feature may provide strengthening for the nasal prong against a compressive or tensile force or both acting on the nasal prong. The feature effectively forms a reinforcing spine along the inside surface of the front wall 5042 of the prong, with the feature extending upward from the base of the prong and following the contour of the prong. The reinforcing feature 5100 acts to allow lateral and rotational movement of the prong and allows the nasal prong to elastically deform in compressive and tensile force directions and exerts a restorative force to ensure the prong returns to its original shape. In one form, the reinforcing feature 5100 is an upward extending bead running from the base of the prong to the top of the prong. In one form, the rib is located along the inner surface of the front wall 5042. The bead extends upward from the base of the prong to the top of the prong. The bead may extend the entire length of the prong and follow the contour of the prong. Alternatively the bead may only extend a partial height of the prong. Alternatively the bead may be located on the outer surface of the front wall. In a further alternative form the bead may be located along the back wall, either on the inner or outer surface of the back wall. The bead is preferably over moulded onto the prong. The reinforcing feature (in this form the bead) may be formed from a more rigid material than the prongs. The bead is applied to the prong by a co-injection process. The co-injection process involves injection moulding the prong from one material, transferring the prong and/or manifold and face mount part to another tool where the bead material is injection moulded over the prong. The bead acts like a spine to support the prong.

In another form the reinforcing feature 5100 may be a rib that extends upward from the base of the prong, along the height of the prong and follows the contour of the prong. The rib is preferably located on the inside surface of the front wall 5042 but may be located on the outer surface of the front wall 5042. Alternatively the rib may be located on the back wall 5043. The rib may be located either on the inner surface or outer surface of the back wall 5043. The prong preferably includes a plurality of ribs formed along the inner surface of the front wall. The ribs preferably extend the entire distance of the prong, but may alternatively only extend a partial distance. The ribs may be identical to each other in dimensions. The ribs may be equally spaced apart along the prong. The ribs form a skeletal structure that supports the prong and reduces deformation of the prongs. The ribs may be formed from a co-injection moulding process as described for the bead. The ribs may be made of a material that is more rigid than the material used to make the prongs.

The prong may also include a series of ribs (not shown) running generally horizontally across the prong. The ribs maybe used in combination with the reinforcing feature (e.g. the bead) to strengthen the nasal prongs in

compressive and tensile directions, while allowing lateral and rotational movement. The reinforcing feature (with or without ribs) may be present on the front wall 5042 of nasal prong. This is advantageous since this provides the greatest strengthening and also because the material used for the prongs responds best in compression. The reinforcing feature may be formed integrally with the nasal prongs during the forming process. Alternatively, a reinforcing feature can be attached to the nasal prongs after forming - e.g. by gluing or ultrasonic welding. The feature may be made from the same material as the nasal prong. Alternatively the feature or the ribs could be made from a stiff material such as another polymer material.

The reinforcing feature could alternatively be created by having the front wall 5042 thicker than the backwall 5043, when viewed from above. The increased thickness of the front wall 5042 effectively provides lateral and rotational movement of the prong while providing improved strength characteristics under compressive and tensile loads. The thicker front wall 5042 ensures that the nasal prongs 33, 34 do not collapse or tear when subjected to compressive or tensile forces.

A potential problem with "tubular" or "round" nasal prongs of the prior art type is the possibility of creating a seal in the patient's nasal passage. Although a seal is desirable in certain circumstances if using e.g. nasal pillows of the type described in WO 2008/014543, in other circumstances, a seal within the patient's respiratory system can lead to an overpressure being created within the patient's nares. This overpressure can lead to barrotrauma resulting in severe injury and possible patient death. It can also interfere with the patients natural breathing or self-breathing. The additional flexibility and greater aperture size provided to the nasal prongs 5033, 5034 by the cut-outs 5041 aids in minimizing the risk of the cannula creating a seal in the patient's nares. However, it should be noted that a seal is sometimes desirable, and although a nasal cannula arrangement which is not intended to seal has been described, the nasal prongs 5033, 5034, or the face mount part 5032, or both, could be adapted to seal against the nostrils of a user.

The flexibility of the side straps 5031 allows for easy securement of the nasal cannula arrangement 5030 on the user's face since the straps can easily be bent and flexed to fit around a user's face. The flexibility of the open tubular recess 38 enables the open tubular recess 5038 to fit around the manifold portion 5035 and create a secure friction fit or snap on fit. The face mount part 5032 is moulded as a single piece of flexible plastic, silicone or rubber material for reliability and ease of use.

The nasal cannula arrangement and the nasal prongs in particular, as shown in Figures 50 to 58 are predominantly useful for delivering gases with high humidity and high flow rate which is advantageous to the patient.

An alternative configuration of the patient interface can alternatively be provided.

The alternative configuration of the patient interface broadly consists of a head securement mechanism substantially similar to that described above, and a nasal cannula arrangement. The head securement mechanism is used to attach the patient interface to a patient's face and maintain the position of the patient interface in the correct position when in use. The head securement mechanism as described in relation to Figure 50 and 51 can also be used with the alternative configuration of the nasal cannula. Alternatively no separate head securement mechanism needs to be used with the nasal interface. This alternative form of head securement will be described later. The humidification or respiratory therapy system with which the alternative nasal cannula arrangement is used can include a secondary supply conduit 5062 similar to that described above, which allows gaseous or fluidic communication between the outlet end of the main delivery conduit 5003 and the main part of the nasal cannula arrangement. However, in this alternative form, the secondary supply conduit 5062 and the main delivery conduit 5003 can be thought of as a single 'delivery conduit' in this context.

The nasal cannula of the alternative configuration will now be described in more detail. The nasal cannula of the alternative configuration comprises three main parts: a pair of carrier tubes, a manifold section, and a pair of nasal prongs, one each of the pair of nasal prongs attached to each of the carrier tubes, the carrier tubes connected to the manifold section, which is connected to the delivery conduit as outlined above so that a stream of gases is delivered to the manifold section. The carrier tubes are used instead of the secondary conduit. The manifold section is formed as a Y-piece connector or a T-piece connector. The carrier tubes are connected to the branches of the Y- piece or T-piece manifold section, preferably with a friction fit. Alternatively the carrier tubes may be connected to the Y- or T-piece by threading or gluing. An even further alternative is the carrier tubes are formed integral to the Y- or T-piece. The Y- piece connector directs flow of gases from the secondary supply conduit to each of the carrier tubes. Preferably the Y- or T-piece is made of a rigid polymer material, the material rigid enough that it does not readily deform under common operational loads.

The carrier tubes can be attached to a head securement, or they can themselves be adapted to be used as a head securement mechanism. The carrier tubes are wrapped around behind the ears. The carrier tubes allow flexibility for head securement. The carrier tubes are light enough to wrap around a patient's ears and be comfortable for the patient to use. The use of carrier tubes makes the entire nasal cannula light in weight. This can help to increase the comfort level for a patient while using the nasal cannula. The carrier tubes also let people of various sizes to use the nasal cannula arrangement as long as the carrier tubes are long enough to be placed over their ears. The carrier tubes connect to the manifold and form a fluid connection with the manifold. The carrier tubes supply breathing gases to the manifold. The manifold has at least one prong extending from it, the prong delivering breathing gases from the manifold to the patient's nasal passage.

In an alternate form a nasal prong is attached to each of the carrier tubes at the patient end. The nasal prongs can be detachable from the carrier tubes. Preferably the nasal prongs are attached to the carrier tubes by a friction fit. Alternatively the nasal prongs are threaded into the carrier tubes. Another alternative is the nasal prongs are glued or attached to the carrier tubes by an industrial adhesive. As a further alternative the nasal prongs may be integrally formed with the carrier tubes.

In this alternative form, the prongs are substantially the same as prongs 5033, 5034 described above. Each prong may include a cut-out on the rear side (that part closest to the face of a user in use), which in one form is cut out of the rear of each of the prongs so that the edges of the cut-out conform to the surface of a reverse S-shaped surface.

The nasal cannula can be used in high flow, high pressure therapy. A stream of gases enters the manifold substantially horizontally because the cannula has a side entry manifold. The stream of gases flows from the manifold into the prongs, out of the top of the prongs and into a user's nostrils. The inlet stream of gases enters the manifold in a substantially horizontal direction that is approximately orthogonal to the prongs. The inlet stream of gases turns through approximately ninety degrees as the gases flow into and up the prongs such that the stream of gases flows substantially aligned with the prongs axis of extension relative to the manifold. In prior art nasal cannula a substantial amount of the gases generally changes direction or turns at the entry of the prongs, which is a small area at the base of the prongs. The turning of the stream of gases within the entry to the prong causes the velocity of the gases stream to reduce. The reduction in flow velocity causes a pressure drop across the entry of the prongs since the pressure of the gases stream is proportionally related to the velocity of the gases stream. In prior art cannula approximately 65% of the gases stream is turned within the entry of the prongs. The pressure drop is proportional to the radius of the prongs to the power of four. The pressure and velocity drop is undesirable because it reduces the effectiveness of the therapy being delivered to the patient. The reduced pressure and velocity may also be dangerous for the patient as the patient may not be getting enough breathing gases. In CPAP type treatment the airways of the patient need to be consistently pressurised in order to allow the patient to breathe properly. A reduction in gases stream pressure due to the pressure drop across the entry to the prongs can cause the airways of the patient to collapse due to lack of pressure being supplied to the patient. The reduction in pressure can also cause the blower speed and power to increase in order to compensate for the pressure drop. This can be dangerous because the blower may be operating at high speeds. The pressure and velocity drop can also be adverse to patients receiving ventilator therapy because these patients will not receive adequate breathing pressure and the ventilator can begin to operate outside normal operating levels to try and compensate for the pressure and velocity drop. The prior art cannula may suffer from a pressure drop of approximately 25cm H2O.

The prongs and manifold are shaped and adapted to turn at least part of the stream of gases inside the manifold rather than inside the prongs.

Although the present disclosure has been described in terms of certain embodiments, other embodiments apparent to those of ordinary skill in the art also are within the scope of this disclosure. For instance, various components may be repositioned as desired. Features from any of the described embodiments may be combined with each other and/or an apparatus may comprise one, more, or all of the features of the above described embodiments. Moreover, not all of the features, aspects and advantages are necessarily required to practice the present disclosure. Accordingly, the scope of the present disclosure is intended to be defined only by the claims that follow.

## Claims

1. A connector (700) for use in a respiratory system, the connector comprising:
a body (701) defining a bore (703) for receipt of a complementary connector component, the bore having a terminal end (704) that provides an entry into the bore for the complementary connector component, a sealing region (705) for engaging with a seal on the complementary connector component, and a sealing region dimension between opposed surfaces of the sealing region, the body comprising diametrically opposed retaining protrusions (710) that extend into the bore from an inner wall of the body, the diametrically opposed retaining protrusions having a distance between radially inward surfaces thereof, **characterised in that** the sealing region dimension is between 25 mm and 27 mm and wherein the distance between the radially inward surfaces is between 21 mm and 23 mm;
wherein the connector comprises one or more axially oriented recesses (761) for interacting with complementary external alignment feature(s) on the complementary connector component, the one or more axially oriented recesses located in the body of the connector at the terminal end of the bore and extending from a radially inner surface of the body to a radially outer surface of the body.

2. The connector of claim 1, wherein the sealing region dimension is between 25.5 mm and 26.5 mm, is 25 mm, is 25.1 mm, is 25.2 mm, is 25.3 mm, is 25.4 mm, is 25.5 mm, is 25.6 mm, is 25.7 mm, is 25.8 mm, is 25.9 mm, is 26 mm, is 26.1 mm, is 26.2 mm, is 26.3 mm, is 26.4 mm, is 26.5 mm, is 26.6 mm, is 26.7 mm, is 26.8 mm, is 26.9 mm, is 27 mm, or is any value between any two of those values.

3. The connector of claim 1 or 2, wherein the distance between the radially inward surfaces is between 21.5 mm and 22.5 mm, or is 21 mm, is 21.1 mm, is 21.2 mm, is 21.3 mm, is 21.4 mm, is 21.5 mm, is 21.6 mm, is 21.7 mm, is 21.8 mm, is 21.9 mm, is 22 mm, is 22.1 mm, is 22.2 mm, is 22.3 mm, is 22.4 mm, is 22.5 mm, is 22.6 mm, is 22.7 mm, is 22.8 mm, is 22.9 mm, is 23 mm, or is any value between any two of those values.

4. The connector of any one of claims 1 to 3, wherein each of the diametrically opposed retaining protrusions has a retaining face, wherein the angle of the retaining face is between 90 degrees and 125 degrees relative to a longitudinal axis of the bore.

5. The connector of claim 4, wherein the angle of the retaining face is between 95 degrees and 120 degrees, is between 100 degrees and 115 degrees, is between 105 degrees and 115 degrees, is 90 degrees, is 91 degrees, is 92 degrees, is 93 degrees, is 94 degrees, is 95 degrees, is 96 degrees, is 97 degrees, is 98 degrees, is 99 degrees, is 100 degrees, is 101 degrees, is 102 degrees, is 103 degrees, is 104 degrees, is 105 degrees, is 106 degrees, is 107 degrees, is 108 degrees, is 109 degrees, is 110 degrees, is 111 degrees, is 112 degrees, is 113 degrees, is 114 degrees, is 115 degrees, is 116 degrees, is 117 degrees, is 118 degrees, is 119 degrees, is 120 degrees, is 121 degrees, is 122 degrees, is 123 degrees, is 124 degrees, or is 125 degrees relative to a longitudinal axis of the bore, or is any angle between any two of those values.

6. The connector of any one of claims 1 to 5, wherein the diametrically opposed retaining protrusions are configured to provide a retention force in an axial direction of the connector of between 10 N and 100 N, optionally between 10 N and 75 N, optionally between 10 N and 50 N, the retention force being a pull out force required to remove the complementary connector component from the bore of the connector when in use.

7. The connector of any one of claims 1 to 6, wherein the diametrically opposed retaining protrusions comprise a first pair of adjacent retaining protrusions on one side of the bore and a second pair of adjacent retaining protrusions on an opposite side of the bore.

8. The connector of any one of claims 1 to 7, wherein the sealing region comprises an effective sealing location for contact by a surface of a seal on the complementary connector component, and wherein the sealing region dimension is a dimension of the effective sealing location.

9. The connector of claim 8, wherein the effective sealing location is substantially at a centre of the sealing region, for contact by the surface of the seal which is at a centre of the seal.

10. The connector of any one of claims 1 to 9, wherein the sealing region is closer to the terminal end relative to the diametrically opposed retaining protrusions.

11. The connector of any one of claims 1 to 10, wherein the connector comprises at least one internal alignment feature.

12. The connector of claim 11, wherein the at least one internal alignment feature comprises at least one alignment member that extends into the bore from an inner wall of the body, wherein the at least one internal alignment member is configured to be received in a recess in the complementary connector component.

13. The connector of claim 12, wherein the at least one alignment member is at a 90 degree offset around the bore from retaining faces of the retaining protrusions.

14. A filter for use in a respiratory therapy system, the filter comprising:
an inlet port for the flow of gases;
an outlet port for the flow of gases;
a cavity containing a filtration material in a flow path through the filter; and
the connector of any one of claims 1 to 13 at the inlet port.

## Patentansprüche

1. Verbinder (700) zur Verwendung in einem Atemsystem, wobei der Verbinder Folgendes umfasst:
einen Körper (701), der eine Bohrung (703) zur Aufnahme einer komplementären Verbinderkomponente definiert, wobei die Bohrung ein Anschlussende (704), das einen Eintritt in die Bohrung für die komplementäre Verbinderkomponente bereitstellt, einen Dichtungsbereich (705) zum Eingreifen mit einer Dichtung an der komplementären Verbinderkomponente und eine Dichtungsbereichsabmessung zwischen gegenüberliegenden Oberflächen des Dichtungsbereiches aufweist, wobei der Körper diametral gegenüberliegende Haltevorsprünge (710) umfasst, die sich von einer Innenwand des Körpers in die Bohrung erstrecken, wobei die diametral gegenüberliegenden Haltevorsprünge einen Abstand zwischen radial inneren Oberflächen davon aufweisen,
**dadurch gekennzeichnet, dass** die Dichtungsbereichsabmessung zwischen 25 mm und 27 mm beträgt, und wobei der Abstand zwischen den radial inneren Oberflächen zwischen 21 mm und 23 mm beträgt;
wobei der Verbinder eine oder mehrere axial ausgerichtete Aussparungen (761) zum Zusammenwirken mit (einem) komplementären externen Ausrichtungsmerkmal(en) an der komplementären Verbinderkomponente umfasst, wobei sich die eine oder die mehreren axial ausgerichteten Aussparungen in dem Körper des Verbinders an dem Anschlussende der Bohrung befinden und sich von einer radial inneren Oberfläche des Körpers zu einer radial äußeren Oberfläche des Körpers erstrecken.

2. Verbinder nach Anspruch 1, wobei die Dichtungsbereichsabmessung zwischen 25,5 mm und 26,5 mm beträgt, 25 mm beträgt, 25,1 mm beträgt, 25,2 mm beträgt, 25,3 mm beträgt, 25,4 mm beträgt, 25,5 mm beträgt, 25,6 mm beträgt, 25,7 mm beträgt, 25,8 mm beträgt, 25,9 mm beträgt, 26 mm beträgt, 26,1 mm beträgt, 26,2 mm beträgt, 26,3 mm beträgt, 26,4 mm beträgt, 26,5 mm beträgt, 26,6 mm beträgt, 26,7 mm beträgt, 26,8 mm beträgt, 26,9 mm beträgt, 27 mm beträgt oder einen beliebigen Wert zwischen zwei beliebigen dieser Werte beträgt.

3. Verbinder nach Anspruch 1 oder 2, wobei der Abstand zwischen den radial inneren Oberflächen zwischen 21,5 mm und 22,5 mm beträgt oder 21 mm beträgt, 21,1 mm beträgt, 21,2 mm beträgt, 21,3 mm beträgt, 21,4 mm beträgt, 21,5 mm beträgt, 21,6 mm beträgt, 21,7 mm beträgt, 21,8 mm beträgt, 21,9 mm beträgt, 22 mm beträgt, 22,1 mm beträgt, 22,2 mm beträgt, 22,3 mm beträgt, 22,4 mm beträgt, 22,5 mm beträgt, 22,6 mm beträgt, 22,7 mm beträgt, 22,8 mm beträgt, 22,9 mm beträgt, 23 mm beträgt oder einen beliebigen Wert zwischen zwei beliebigen dieser Werte beträgt.

4. Verbinder nach einem der Ansprüche 1 bis 3, wobei jeder der diametral gegenüberliegenden Haltevorsprünge eine Haltefläche aufweist, wobei der Winkel der Haltefläche relativ zu einer Längsachse der Bohrung zwischen 90 Grad und 125 Grad beträgt.

5. Verbinder nach Anspruch 4, wobei der Winkel der Haltefläche zwischen 95 Grad und 120 Grad beträgt, zwischen 100 Grad und 115 Grad beträgt, zwischen 105 Grad und 115 Grad beträgt, 90 Grad beträgt, 91 Grad beträgt, 92 Grad beträgt, 93 Grad beträgt, 94 Grad beträgt, 95 Grad beträgt, 96 Grad beträgt, 97 Grad beträgt, 98 Grad beträgt, 99 Grad beträgt, 100 Grad beträgt, 101 Grad beträgt, 102 Grad beträgt, 103 Grad beträgt, 104 Grad beträgt, 105 Grad beträgt, 106 Grad beträgt, 107 Grad beträgt, 108 Grad beträgt, 109 Grad beträgt, 110 Grad beträgt, 111 Grad beträgt, 112 Grad beträgt, 113 Grad beträgt, 114 Grad beträgt, 115 Grad beträgt, 116 Grad beträgt, 117 Grad beträgt, 118 Grad beträgt, 119 Grad beträgt, 120 Grad beträgt, 121 Grad beträgt, 122 Grad beträgt, 123 Grad beträgt, 124 Grad beträgt oder 125 Grad relativ zu einer Längsachse der Bohrung beträgt oder einen beliebigen Winkel zwischen zwei beliebigen dieser Werte beträgt.

6. Verbinder nach einem der Ansprüche 1 bis 5, wobei die diametral gegenüberliegenden Haltevorsprünge dazu konfiguriert sind, eine Haltekraft in einer axialen Richtung des Verbinders zwischen 10 N und 100 N, optional zwischen 10 N und 75 N, optional zwischen 10 N und 50 N bereitzustellen, wobei die Haltekraft eine Auszugskraft ist, die erforderlich ist, um die komplementäre Verbinderkomponente bei Verwendung aus der Bohrung des Verbinders zu entfernen.

7. Verbinder nach einem der Ansprüche 1 bis 6, wobei die diametral gegenüberliegenden Haltevorsprünge ein erstes Paar von benachbarten Haltevorsprüngen auf einer Seite der Bohrung und ein zweites Paar von benachbarten Haltevorsprüngen auf einer gegenüberliegenden Seite der Bohrung umfassen.

8. Verbinder nach einem der Ansprüche 1 bis 7, wobei der Dichtungsbereich eine wirksame Dichtstelle zur Berührung durch eine Oberfläche einer Dichtung an der komplementären Verbinderkomponente umfasst und wobei die Dichtungsbereichsabmessung eine Abmessung der wirksamen Dichtstelle ist.

9. Verbinder nach Anspruch 8, wobei sich die wirksame Dichtungsstelle im Wesentlichen in einer Mitte des Dichtungsbereichs zum Kontakt durch die Oberfläche der Dichtung befindet, die sich in einer Mitte der Dichtung befindet.

10. Verbinder nach einem der Ansprüche 1 bis 9, wobei sich der Dichtungsbereich relativ zu den diametral gegenüberliegenden Haltevorsprüngen näher an dem Anschlussende befindet.

11. Verbinder nach einem der Ansprüche 1 bis 10, wobei der Verbinder mindestens ein internes Ausrichtungsmerkmal umfasst.

12. Verbinder nach Anspruch 11, wobei das mindestens eine interne Ausrichtungsmerkmal mindestens ein Ausrichtungselement umfasst, das sich von einer Innenwand des Körpers in die Bohrung erstreckt, wobei das mindestens eine interne Ausrichtungselement dazu konfiguriert ist, in einer Aussparung in der komplementären Verbinderkomponente aufgenommen zu werden.

13. Verbinder nach Anspruch 12, wobei das mindestens eine Ausrichtungselement um 90 Grad um die Bohrung von Halteflächen der Haltevorsprünge versetzt ist.

14. Filter zur Verwendung in einem Atemtherapiesystem, wobei der Filter Folgendes umfasst:
eine Einlassöffnung für den Gasstrom;
eine Auslassöffnung für den Gasstrom;
einen Hohlraum, der ein Filtrationsmaterial in einem Strömungsweg durch den Filter enthält; und
den Verbinder nach einem der Ansprüche 1 bis 13 an der Einlassöffnung.

## Revendications

1. Raccord (700) destiné à être utilisé dans un système respiratoire, le raccord comprenant :
un corps (701) définissant un alésage (703) destiné à recevoir un composant de raccord complémentaire, l'alésage possédant une extrémité terminale (704) qui fournit une entrée dans l'alésage pour le composant de raccord complémentaire, une zone d'étanchéité (705) destinée à venir en prise avec un joint d'étanchéité sur le composant de raccord complémentaire, et la dimension de la zone d'étanchéité entre les surfaces opposées de la zone d'étanchéité, le corps comprenant des saillies de retenue diamétralement opposées (710) qui s'étendent dans l'alésage à partir d'une paroi interne du corps, les saillies de retenue diamétralement opposées présentant une distance entre leurs surfaces radialement vers l'intérieur,
**caractérisé en ce que** la dimension de la zone d'étanchéité est comprise entre 25 mm et 27 mm et ladite distance entre les surfaces radialement vers l'intérieur est comprise entre 21 mm et 23 mm ;
ledit raccord comprenant un ou plusieurs évidements orientés axialement (761) destinés à interagir avec une ou plusieurs caractéristiques d'alignement externes complémentaires sur le composant de raccord complémentaire, lesdits un ou plusieurs évidements orientés axialement étant situés dans le corps du raccord au niveau de l'extrémité terminale de l'alésage et s'étendant d'une surface radialement interne du corps à une surface radialement externe du corps.

2. Raccord selon la revendication 1, ladite dimension de la zone d'étanchéité étant comprise entre 25,5 mm et 26,5 mm, étant de 25 mm, étant de 25,1 mm, étant de 25,2 mm, étant de 25,3 mm, étant de 25,4 mm, étant de 25,5 mm, étant de 25,6 mm, étant de 25,7 mm, étant de 25,8 mm, étant de 25,9 mm, étant de 26 mm, étant de 26,1 mm, étant de 26,2 mm, étant de 26,3 mm, étant de 26,4 mm, étant de 26,5 mm, étant de 26,6 mm, étant de 26,7 mm, étant de 26,8 mm, étant de 26,9 mm, étant de 27 mm, ou étant une valeur quelconque entre deux de ces valeurs.

3. Raccord selon la revendication 1 ou 2, ladite distance entre les surfaces radialement vers l'intérieur étant comprise entre 21,5 mm et 22,5 mm, ou étant de 21 mm, étant de 21,1 mm, étant de 21,2 mm, étant de 21,3 mm, étant de 21,4 mm, étant de 21,5 mm, étant de 21,6 mm, étant de 21,7 mm, étant de 21,8 mm, étant de 21,9 mm, étant de 22 mm, étant de 22,1 mm, étant de 22,2 mm, étant de 22,3 mm, étant de 22,4 mm, étant de 22,5 mm, étant de 22,6 mm, étant de 22,7 mm, étant de 22,8 mm, étant de 22,9 mm, étant de 23 mm, ou étant une valeur quelconque entre deux de ces valeurs.

4. Raccord selon l'une quelconque des revendications 1 à 3, chacune des saillies de retenue diamétralement opposées comportant une face de retenue, ledit angle de la face de retenue étant compris entre 90 degrés et 125 degrés par rapport à l'axe longitudinal de l'alésage.

5. Raccord selon la revendication 4, ledit angle de la face de retenue étant compris entre 95 degrés et 120 degrés, étant compris entre 100 degrés et 115 degrés, étant compris entre 105 degrés et 115 degrés, étant de 90 degrés, étant de 91 degrés, étant de 92 degrés, étant de 93 degrés, étant de 94 degrés, étant de 95 degrés, étant de 96 degrés, étant de 97 degrés, étant de 98 degrés, étant de 99 degrés, étant de 100 degrés, étant de 101 degrés, étant de 102 degrés, étant de 103 degrés, étant de 104 degrés, étant de 105 degrés, étant de 106 degrés, étant de 107 degrés, étant de 108 degrés, étant de 109 degrés, étant de 110 degrés, étant de 111 degrés, étant de 112 degrés, étant de 113 degrés, étant de 114 degrés, étant de 115 degrés, étant de 116 degrés, étant de 117 degrés, étant de 118 degrés, étant de 119 degrés, étant de 120 degrés, étant de 121 degrés, étant de 122 degrés, étant de 123 degrés, étant de 124 degrés, ou étant de 125 degrés par rapport à l'axe longitudinal de l'alésage, ou étant un angle quelconque entre deux de ces valeurs.

6. Raccord selon l'une quelconque des revendications 1 à 5, lesdites saillies de retenue diamétralement opposées étant conçues pour fournir une force de retenue suivant la direction axiale du raccord comprise entre 10 N et 100 N, éventuellement entre 10 N et 75 N, éventuellement entre 10 N et 50 N, la force de retenue étant la force de traction nécessaire pour retirer de l'alésage du raccord le composant de raccord complémentaire lors de l'utilisation.

7. Raccord selon l'une quelconque des revendications 1 à 6, lesdites saillies de retenue diamétralement opposées comprenant une première paire de saillies de retenue adjacentes sur un côté de l'alésage et une seconde paire de saillies de retenue adjacentes sur un côté opposé de l'alésage.

8. Raccord selon l'une quelconque des revendications 1 à 7, ladite zone d'étanchéité comprenant un emplacement d'étanchéité effectif pour le contact par une surface d'un joint d'étanchéité sur le composant de raccord complémentaire, et ladite dimension de la zone d'étanchéité étant la dimension de l'emplacement d'étanchéité effectif.

9. Raccord selon la revendication 8, ledit emplacement d'étanchéité effectif se trouvant sensiblement au centre de la zone d'étanchéité, pour un contact par la surface du joint d'étanchéité qui se trouve au centre du joint d'étanchéité.

10. Raccord selon l'une quelconque des revendications 1 à 9, ladite zone d'étanchéité étant plus proche de l'extrémité terminale par rapport aux saillies de retenue diamétralement opposées.

11. Raccord selon l'une quelconque des revendications 1 à 10, ledit raccord comprenant au moins une caractéristique d'alignement interne.

12. Raccord selon la revendication 11, ledit au moins un élément d'alignement interne comprenant au moins un élément d'alignement qui s'étend dans l'alésage à partir d'une paroi interne du corps, ledit au moins un élément d'alignement interne étant conçu pour être reçu dans un évidement dans le composant de raccord complémentaire.

13. Raccord selon la revendication 12, ledit au moins un élément d'alignement se trouvant à un décalage de 90 degrés autour de l'alésage par rapport aux faces de retenue des saillies de retenue.

14. Filtre destiné à être utilisé dans un système de thérapie respiratoire, le filtre comprenant :
un orifice d'entrée pour l'écoulement des gaz ;
un orifice de sortie pour l'écoulement des gaz ;
une cavité contenant un matériau de filtration dans un trajet d'écoulement à travers le filtre ; et
le raccord selon l'une quelconque des revendications 1 à 13 au niveau de l'orifice d'entrée.
